# EUROPEAN PATENT APPLICATION

(11) **EP 3 906 921 A1**
(43) Date of publication of application: **10.11.2021**
(21) Application number: 21162169.3
(22) Date of filing: 27.04.2015
(51) Int. Cl.: A61K 31/337, A61K 31/47, A61K 31/517, A61K 31/7068, A61P 35/00

(54) **METHOD OF TREATING LUNG ADENOCARCINOMA**

(30) Priority: 25.04.2014 US 201461984599 P
(62) Divisional of application: 15721100.4
(71) Applicant: Exelixis, Inc., Alameda, CA 94502 (US)
(72) Inventor: AFTAB, Dana T, San Rafael, CA California 94903 (US); YU, Peiwen, Cupertino, CA California 95014 (US)
(74) Representative: Arch, Peter Jonathan Sanders

(57) **Abstract**

This invention is directed to the treatment of cancer in a patient, particularly a patient with lung adenocarcinoma, and more particularly a patient with SLC34A2-ROS1, CD74-ROS1, or FIG-ROS1 fusion-positive non-small cell lung cancer, with an inhibitor of MET, VEGFR2, and ROS1 which is a compound of Formula I: 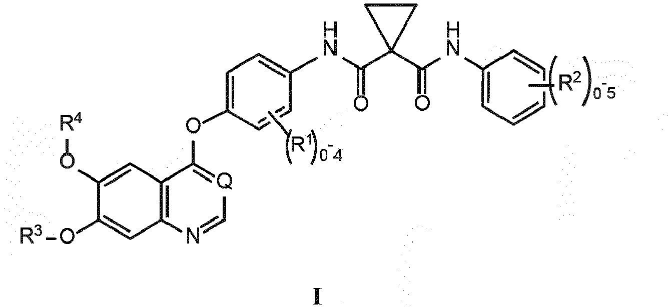 or a pharmaceutically acceptable salt thereof.

## Description

### Cross Reference to Related Applications

This PCT application claims the benefit of U.S. Provisional Application Serial No. 61/984,599, filed April 25, 2014, the disclosure of which is hereby incorporated by reference in its entirety.

### Sequence Listing

This application incorporates by reference in its entirety the Sequence Listing entitled "EX14-003C-PC_2015_04_20_SEQUENCE_LISTING_ST25.txt," created on April 27, 2015, at 12:54 pm, that is 26 KB, and filed electronically herewith.

### Field of the Invention

This invention is directed to the detection, diagnosis and treatment of cancer, particularly lung adenocarcinoma, using an inhibitor of receptor tyrosine kinases.

### Background of the Invention

Lung cancer is the leading cause of cancer-related mortality worldwide. Recent developments in targeted therapies have led to a treatment paradigm shift in non-small-cell lung cancer (NSCLC). Mok TS, Wu YL, Thongprasert S, Yang CH, Chu DT, Saijo N, Sunpaweravong P, Han B, Margono B, Ichinose Y, Nishiwaki Y, Ohe Y, Yang JJ, Chewaskulyong B, Jiang H, Duffield EL, Watkins CL, Armour AA, Fukuoka M. Gefitinib or carboplatin-paclitaxel in pulmonary adenocarcinoma. N Engl J Med. 2009 Sep 3;361(10):947-57.Maemondo M, Inoue A, Kobayashi K, Sugawara S, Oizumi S, Isobe H, Gemma A, Harada M, Yoshizawa H, Kinoshita I, Fujita Y, Okinaga S, Hirano H, Yoshimori K, Harada T, Ogura T, Ando M, Miyazawa H, Tanaka T, Saijo Y, Hagiwara K, Morita S, Nukiwa T; North-East Japan Study Group. Gefitinib or chemotherapy for non-small-cell lung cancer with mutated EGFR. N Engl J Med. 2010 Jun 24;362(25):2380-8. Epidermal growth factor receptor (EGFR) tyrosine kinase inhibitors (TKIs), gefitinib and erlotinib, and the anaplastic lymphoma kinase (ALK) TKI, crizotinib, have shown clinical activity in NSCLC patients with *EGFR* mutations or *ALK* gene rearrangements. Kwak EL, Bang YJ, Camidge DR, Shaw AT, Solomon B, Maki RG, Ou SH, Dezube BJ, Jänne PA, Costa DB, Varella-Garcia M, Kim WH, Lynch TJ, Fidias P, Stubbs H, Engelman JA, Sequist LV, Tan W, Gandhi L, Mino-Kenudson M, Wei GC, Shreeve SM, Ratain MJ, Settleman J, Christensen JG, Haber DA, Wilner K, Salgia R, Shapiro GI, Clark JW, Iafrate AJ. Anaplastic lymphoma kinase inhibition in non-small-cell lung cancer. N Engl J Med. 2010 Oct 28;363(18):1693-703. Shaw AT, Camidge, Engelman JA, Solomon BJ, Kwak EL, Clark JW, Salgia R, Shapiro, Bang YJ, Tan W, Tye L, Wilner KD, Stephenson P, Varella-Garcia M, Bergethon K, Iafrate AJ, Ou SH. Clinical activity of crizotinib in advanced non-small cell lung cancer (NSCLC) harboring ROS1 gene rearrangement J Clin Oncol. 2012 30 (suppl; abstr 7508). Fusion of the *KIF5B* (the kinesin family 5B) gene and the *RET* oncogene has been recently reported as a driver mutation in 1-2% of NSCLC patients and are a focus as a therapeutic target. Kohno T, Ichikawa H, Totoki Y, Yasuda K, Hiramoto M, Nammo T, Sakamoto H, Tsuta K, Furuta K, Shimada Y, Iwakawa R, Ogiwara H, Oike T, Enari M, Schetter AJ, Okayama H, Haugen A, Skaug V, Chiku S, Yamanaka I, Arai Y, Watanabe S, Sekine I, Ogawa S, Harris CC, Tsuda H, Yoshida T, Yokota J, Shibata T. KIF5B-RET fusions in lung adenocarcinoma. Nat Med. 2012 Feb 12;18(3):375-7. Takeuchi K, Soda M, Togashi Y, Suzuki R, Sakata S, Hatano S, Asaka R, Hamanaka W, Ninomiya H, Uehara H, Lim Choi Y, Satoh Y, Okumura S, Nakagawa K, Mano H, Ishikawa Y. RET, ROS1 and ALK fusions in lung cancer. Nat Med. 2012 Feb 12;18(3):378-81. Lipson D, Capelletti M, Yelensky R, Otto G, Parker A, Jarosz M, Curran JA, Balasubramanian S, Bloom T, Brennan KW, Donahue A, Downing SR, Frampton GM, Garcia L, Juhn F, Mitchell KC, White E, White J, Zwirko Z, Peretz T, Nechushtan H, Soussan-Gutman L, Kim J, Sasaki H, Kim HR, Park SI, Ercan D, Sheehan CE, Ross JS, Cronin MT, Jänne PA, Stephens PJ. Identification of new ALK and RET gene fusions from colorectal and lung cancer biopsies. Nat Med. 2012 Feb 12;18(3):382-4. Thus, it is becoming more important to identify key driver genes in NSCLC and to develop therapies for each genomic subset of patients.

### Summary of the Invention

These and other needs are met by the present invention which is directed to a method for treating lung adenocarcinoma using an inhibitor of tyrosine kinase activity, specifically, ROS 1 kinase activity. The method comprises administering a therapeutically effective amount of a compound or a pharmaceutically acceptable salt thereof that modulates ROS1 kinase and/or a ROS1 fusion protein to a patient in need of such treatment. In one embodiment, the lung adenocarcinoma is non-small cell lung cancer (NSCLC). More particularly, the lung adenocarcinoma is most frequently SLC34A2-ROS1 fusion-positive NSCLC, CD74-ROS1 fusion positive NSCLC, FIG-ROS1 fusion positive NSCLC, (e.g. CD74-ROS1, FIG-ROS1, FIG-ROS1(L), FIG-ROS1(S), and FIG-ROS1(VL)) and NSCLC harboring other known ROS1 fusions proteins. ROS1 kinase is encoded by the ROS1 gene on chromosome 6 in humans (6q22).

In one aspect, the present invention is directed to a method for treating NSCLC in a patient in need of such treatment, comprising administering a therapeutically effective amount of a compound or a pharmaceutically acceptable salt thereof that simultaneously modulates ROS 1 and/or a ROS1 fusion protein, to the patient

In one embodiment of this and other aspects, the ROS1 kinase inhibitor or ROS1 fusion kinase inhibitor is a compound of Formula I: 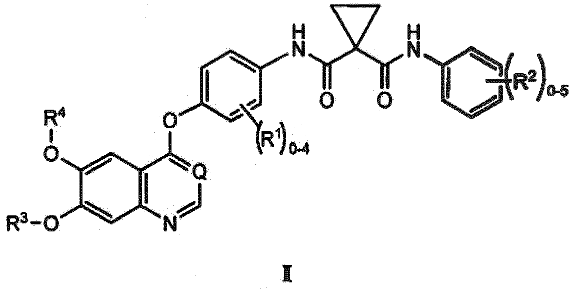 or a pharmaceutically acceptable salt thereof, wherein:
R¹ is halo;
R² is halo;
R³ is (C₁-C₆)alkyl;
R⁴ is (C₁-C₆)alkyl; and
Q is CH or N.

In another embodiment, the compound of Formula I is a compound of Formula Ia 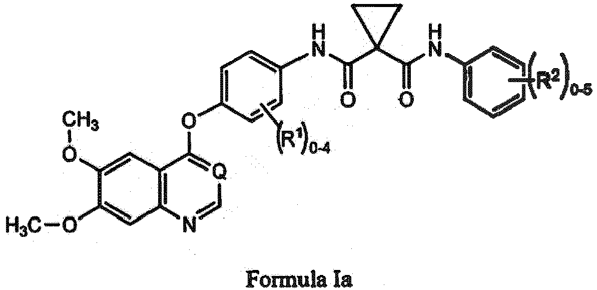 or a pharmaceutically acceptable salt thereof, wherein:
R¹ is halo;
R² is halo; and
Q is CH or N.

In another embodiment, the compound of Formula I is compound 1: 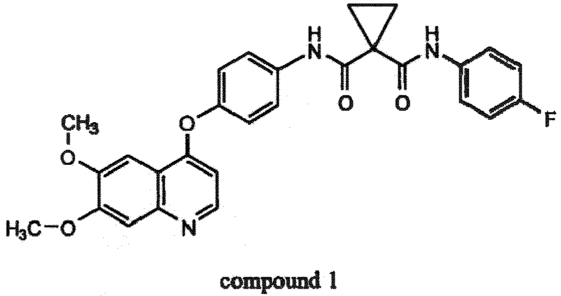 or a pharmaceutically acceptable salt thereof. compound 1 is known as N-(4-{[6,7-bis(methyloxy)quinolin-4-yl]oxy}phenyl)-N'-(4-fluorophenyl)cyclopropane-1,1-dicarboxamide and by the name Cabozantinib. On November 29, 2012, the S-malate salt (i.e., L-malate salt) of N-(4-[(6,7-dimethoxyquinolin-4-yl)oxylphenyl}-N'-(4-fluorophenyl) cyclopropane-1,1-dicarboxamide (also known as cabozantinib or COMETRIQ®) was approved by the United States Food and Drug Administration for the treatment of progressive, metastatic medullary thyroid cancer (MTC). In December 2013, the European Committee for Medicinal Products for Human Use (CHMP), issued a positive opinion on the Marketing Authorization Application (MAA), submitted to the European Medicines Agency, or EMA, for COMETRIQ for the proposed indication of progressive, unresectable, locally advanced, or metastatic MTC. Cabozantinib is being evaluated in a broad development program, including ongoing phase 3 pivotal trials in metastatic renal cell cancer (RCC), and advanced hepatocellular cancer (HCC).

Compound 1 is a potent inhibitor of c-MET, RET, and VEGFR2. Yakes FM, Chen J, Tan J, Yamaguchi K, Shi Y, Yu P, Qian F, Chu F, Bentzien F, Cancilla B, Orf J, You A, Laird AD, Engst S, Lee L, Lesch J, Chou YC, Joly AH. Cabozantinib (XL184), a novel MET and VEGFR2 inhibitor, simultaneously suppresses metastasis, angiogenesis, and tumor growth. Mol Cancer Ther. 2011 Dec;10(12):2298-308; Bentzien, F., Zuzow, M., Heald, N., Gibson, A., Shi, Y., Goon, L., Yu, P., Engst, S., Zhang, W., Huang, S., Zhao, L., Vysotskaia, V., Chu, F., Bautista, R., Cancilla, B., Lamb, P., Joly, A. and Yakes, M. In vitro and in vivo activity of cabozantinib (XL184), an inhibitor of RET, MET, and VEGFR2, in a model of medullary thyroid cancer. Thyroid, 2013 (23) 1569-1577. In preclinical studies, compound 1-mediated inhibition of kinase activity produced rapid and robust regression of tumor vasculature, reduced, tumor invasiveness and metastasis, and prolonged survival. Sennino B., Ishiguro-Oonuma T, Wei Y, Naylor RM, Williamson CW, Bhagwandin V, Tabruyn SP, You WK, Chapman HA, Christensen JG, Aftab DT, McDonald DM. (2012), "Suppression of tumor invasion and metastasis by concurrent inhibition of c-Met and VEGF signaling in pancreatic neuroendocrine tumors.", Cancer Discovery, 2(3):270-87 (Epublished, 02/24/2012).

In another embodiment, the compound of Formula I, Ia, compound 1, or a pharmaceutically acceptable salt thereof, is administered as a pharmaceutical composition comprising a pharmaceutically acceptable additive, diluent, or excipient

In one embodiment, the compound of Formula I, Ia, or compound 1, or a pharmaceutically acceptable salt thereof, is administered as a pharmaceutical composition comprising a pharmaceutically acceptable additive, diluent, or excipient is administered to inhibit oncogenic fusion kinases involving the orphan receptor tyrosine kinase ROS1 resulting from chromosomal rearrangements that lead to constitutive activation of the ROS1 kinase activity. These ROS1 fusion kinases are targeted for inhibition using the compound of Formula I, Ia, or compound 1 or a pharmaceutically acceptable salt thereof. These compounds can be administered as a pharmaceutical composition comprising a pharmaceutically acceptable additive, diluent, or excipient to treat lung adenocarcinoma, for example, non-small cell lung carcinoma harboring one or more oncogenic ROS1 fusion kinases.

In another aspect, the invention provides a method for inhibiting a ROS1 kinase and/or ROS1 fusion kinase activity in one or more non-small cell lung cancers or NSCLC cell lines, comprising administering a therapeutically effective amount of a pharmaceutical composition comprising a compound of Formula I or the malate salt of a compound of Formula I or another pharmaceutically acceptable salt of a compound of Formula I to said non-small cell lung cancers or NSCLC cell lines harboring a ROS1 kinase and/or a ROS1 fusion polypeptide.

In another aspect, the invention provides a method for detecting, diagnosing and treating a ROS1 fusion positive NSCLC, for example, a SLC34A2-ROS1 fusion-positive NSCLC, and other known ROS1 fusion positive NSCLC, including ROS1 fusions CD74-ROS1, FIG-ROS1, FIG-ROS1(L), FIG-ROS1(S), and FIG-ROS1(VL), and other ROS1 fusion proteins encoded by the ROS1 gene on chromosome 6 in humans (See: Bergethon K, Shaw AT, Ou SH, Katayama R, Lovly CM, McDonald NT, Massion PP, Siwak-Tapp C, Gonzalez A, Fang R, Mark EJ, Batten JM, Chen H, Wilner KD, Kwak EL, Clark JW, Carbone DP, Ji H, Engelman JA, Mino-Kenudson M, Pao W, Iafrate AJ. ROS1 rearrangements define a unique molecular class of lung cancers. J Clin Oncol. 2012 Mar 10;30(8):863-70) comprising administering a therapeutically effective amount of a pharmaceutical composition comprising a compound of Formula I or the malate salt of a compound of Formula I or another pharmaceutically acceptable salt of a compound of Formula I, to a patient in need of such treatment. In a specific embodiment, the compound of Formula I is compound 1 or the malate salt of compound 1, particularly the S-malate salt of compound 1.

In another aspect, the invention provides a method for treating a lung adenocarcinoma which is SLC34A2-ROS1 fusion positive non-small cell lung cancer in a patient in need of such treatment, comprising administering to the patient an effective amount of compound 1: 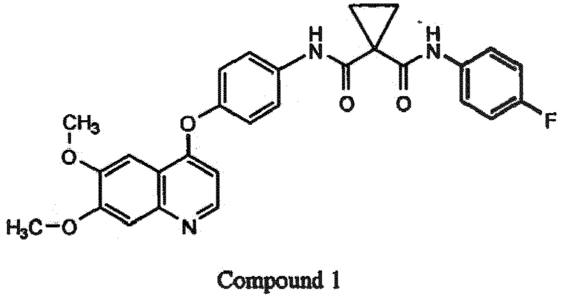 or a pharmaceutically acceptable salt thereof.

In another aspect, the invention provides a method for treating a lung adenocarcinoma which is CD74-ROS1 fusion positive non-small cell lung cancer in a patient in need of such treatment, comprising administering to the patient an effective amount of compound 1: 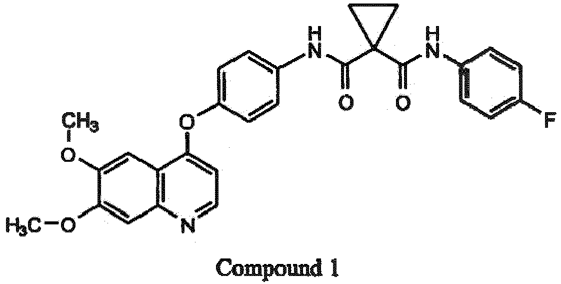 or a pharmaceutically acceptable salt thereof.

In another aspect, the invention provides a method for treating a lung adenocarcinoma which is FIG-ROS1 fusion positive non-small cell lung cancer in a patient in need of such treatment, comprising administering to the patient an effective amount of compound 1: 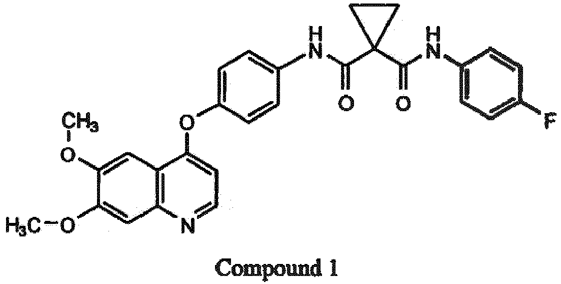 or a pharmaceutically acceptable salt thereof.

In another aspect, the invention provides a method for inhibiting ROS1 fusion kinase activity in a NSCLC cell, the method comprising contacting said cell with an effective amount of a compound of Formula I: 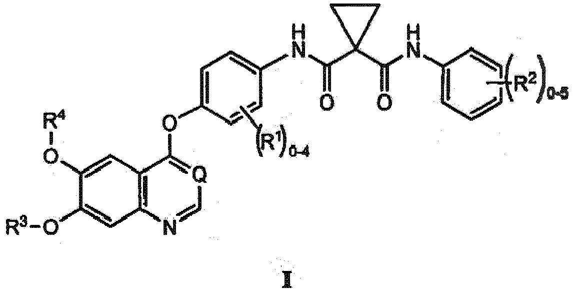 or a pharmaceutically acceptable salt thereof, wherein:
R¹ is halo;
R² is halo;
R³ is (C₁-C₆)alkyl;
R⁴ is (C₁-C₆)alkyl; and
Q is CH or N.

### Brief Description of the Figures

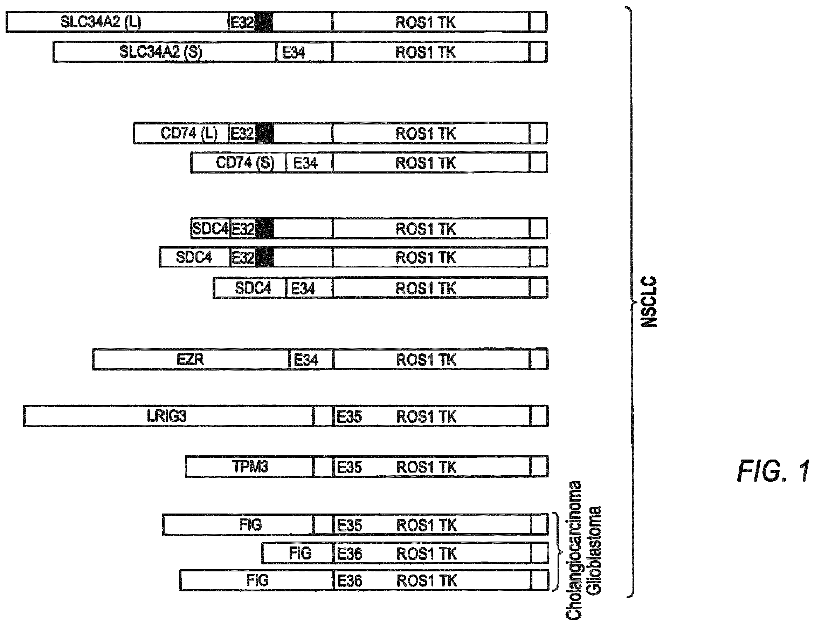

Figure 1 depicts a graphical representation of ROS1 fusion proteins known to exist in Non-Small Cell Lung Carcinoma (NSCLC) tumor samples. ROS1 rearrangements in NSCLC, glioblastoma, and cholangiocarcinoma. CD74(L)/(S), cluster of differentiation 74, long/short isoforms; EZR, ezrin; FIG, fused in glioblastoma; LRIG3, leucine-rich repeats and immunoglobulin-like domains 3; ROS1 TK, ROS1 tyrosine kinase domain with fusion partners; SDC4, syndecan-4; SLC34A2(L)/(S), solute carrier family 34 (sodium phosphate), member 2, long/short isoforms; SDC4, syndecan-4; TPM3, tropomyosin 3. Exon numbers E32, E34, E35, or E36 indicate break points of ROS1. Red box denotes the transmembrane domain retained in ROS1.

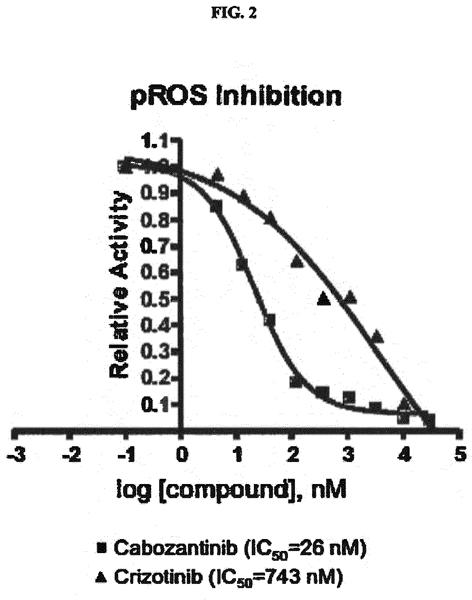

Figure 2 depicts inhibition of phosphorylation of SLC34A2-ROS1 fusion in vitro in HCC-78 NSCLC cells that were administered single escalating doses of compound 1, or 3-[(1*R*)-1-(2,6-dichloro-3-fluorophenyl)ethoxy]-5-(1-piperidin-4-ylpyrazol-4-yl)pyridin-2-amine (Crizotinib; XALKORI®).

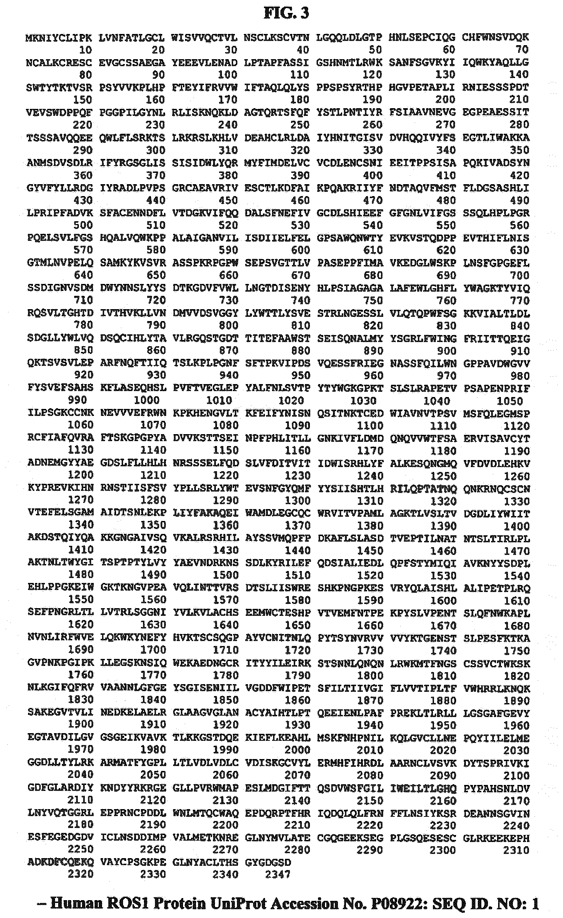

Figure 3 is the amino acid sequence of an exemplary human ROS1 protein denoted herein as SEQ ID NO:1.

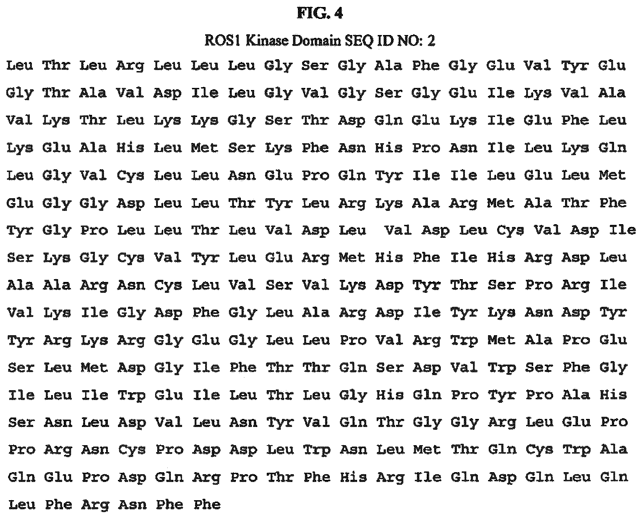

Figure 4 is an exemplary amino acid sequence of human ROS1 kinase domain denoted herein as SEQ ID NO: 2.

### Detailed Description of the Invention

### Abbreviations and Definitions

The following abbreviations and terms have the indicated meanings throughout this application.

| **Abbreviation** | **Meaning** |
|---|---|
| Ac | Acetyl |
| Br | Broad |
| °C | Degrees Celsius |
| c- | Cyclo |
| CBZ | CarboBenZoxy = benzyloxycarbonyl |
| d | Doublet |
| dd | Doublet of doublet |
| dt | Doublet of triplet |
| DCM | Dichloromethane |
| DME | 1,2-dimethoxyethane |
| DMF | *N,N*-Dimethylformamide |
| DMSO | dimethyl sulfoxide |
| EI | Electron Impact ionization |
| G | Gram(s) |
| h or hr | Hour(s) |
| HPLC | High pressure liquid chromatography |
| L | Liter(s) |
| M | Molar or molarity |
| m | Multiplet |
| Mg | Milligram(s) |
| MHz | Megahertz (frequency) |
| Min | Minute(s) |
| mL | Milliliter(s) |
| µL | Microliter(s) |
| µM | Micromole(s) or micromolar |
| mM | Millimolar |
| Mmol | Millimole(s) |
| Mol | Mole(s) |
| MS | Mass spectral analysis |
| N | Normal or normality |
| nM | Nanomolar |
| NMR | Nuclear magnetic resonance spectroscopy |
| q | Quartet |
| RT | Room temperature |
| s | Singlet |
| t or tr | Triplet |
| TFA | Trifluoroacetic acid |
| THF | Tetrahydrofuran |
| TLC | Thin layer chromatography |

The symbol "-" means a single bond, "=" means a double bond.

When chemical structures are depicted or described, unless explicitly stated otherwise, all carbons are assumed to have hydrogen substitution to conform to a valence of four. For example, in the structure on the left-hand side of the schematic below there are nine hydrogens implied. The nine hydrogens are depicted in the right-hand structure. Sometimes a particular atom in a structure is described in textual formula as having a hydrogen or hydrogens as substitution (expressly defined hydrogen), for example, -CH₂CH₂-. It is understood by one of ordinary skill in the art that the aforementioned descriptive techniques are common in the chemical arts to provide brevity and simplicity to description of otherwise complex structures.

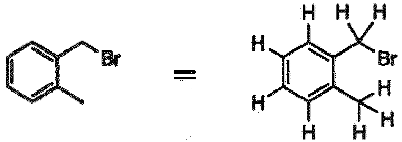

If a group "R" is depicted as "floating" on a ring system, as for example in the formula: 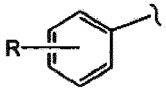 then, unless otherwise defined, a substituent "R" may reside on any atom of the ring system, assuming replacement of a depicted, implied, or expressly defined hydrogen from one of the ring atoms, so long as a stable structure is formed.

If a group "R" is depicted as floating on a fused ring system, as for example in the formulae: 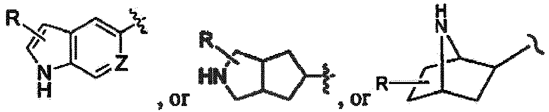 then, unless otherwise defined, a substituent "R" may reside on any atom of the fused ring system, assuming replacement of a depicted hydrogen (for example the -NH- in the formula above), implied hydrogen (for example as in the formula above, where the hydrogens are not shown but understood to be present), or expressly defined hydrogen (for example where in the formula above, "Z" equals =CH-) from one of the ring atoms, so long as a stable structure is formed. In the example depicted, the "R" group may reside on either the 5-membered or the 6-membered ring of the fused ring system. When a group "R" is depicted as existing on a ring system containing saturated carbons, as for example in the formula: 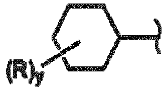 where, in this example, "y" can be more than one, assuming each replaces a currently depicted, implied, or expressly defined hydrogen on the ring; then, unless otherwise defined, where the resulting structure is stable, two "R's" may reside on the same carbon. A simple example is when R is a methyl group; there can exist a geminal dimethyl on a carbon of the depicted ring (an "annular" carbon). In another example, two R's on the same carbon, including that carbon, may form a ring, thus creating a spirocyclic ring (a "spirocyclyl" group) structure with the depicted ring as for example in the formula:

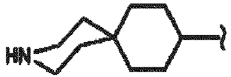

"Halogen" or "halo" refers to fluorine, chlorine, bromine or iodine.

As used herein, "ROS1" protein or polypeptide includes mammalian ROS1, for example, human ROS1 kinase protein (encoded by the ROS1 gene) which is a 2347 amino acid long receptor tyrosine kinase that is prone to aberrant expression leading to cancer. A description of full length human ROS1 kinase (with the amino acid sequence of the human ROS1 protein) can be found at UniProt Accession No. P08922. Additionally, there are multiple known naturally-occurring variants of ROS1 (see, e.g., Greenman et al., Nature 446: 153-158, 2007). The nucleotide and amino acid sequences of murine full-length ROS1 are known (see, e.g., UniProt Accession No. Q78DX7). Using routine experimentation, the ordinarily skilled biologist would be readily able to determine corresponding sequences in non-human mammalian ROS 1 homologues.

By "wild-type" ROS1 is meant the full length ROS1 kinase (i.e., for human ROS1, the 2347 amino acid long polypeptide (SEQ ID NO:1) or 2320 amino acid long polypeptide (mature sequence) following removal of the signal peptide sequence) in healthy (or normal) tissue (e.g., non-cancerous tissue) of a normal individual (e.g., a normal individual who is not suffering from cancer). ROS1 kinase (full length or truncated) does not appear to be expressed in normal lung tissue in humans. However, using the methods described below, the inventors have made the surprising discovery of specific inhibition of ROS1 kinase in lung cancer cells using the compounds described herein. Such expression in an atypical cell (in this case a cancerous cell) where no expression is seen in a typical cell (e.g., a non-cancerous lung cell) is aberrant.

Aberrantly expressed ROS1 kinase, in the form of a fusion with another protein, for example, SLC34A2, CD74 or FIG, has been reported in glioblastoma (see Charest et al., Charest et al., Genes Chromosomes Cancer 37: 58-71, 2003; Charest et al., Proc. Natl. Acad. Sci. USA 100: 916-921,2003) in liver cancer (see, e.g., PCT Publication No. WO2010/093928) in bile duct cancer (see Gu et al., PLOS one 2011 Jan 6;6(1):e15640. doi:
10.1371/journal.pone.0015640) and in NSCLC adenocarcinoma (See Kurtis D. Davies et al. Identifying and Targeting ROS1 Gene Fusions in Non-Small Cell Lung Cancer, Clin Cancer Res. 2012 September 1; 18(17): 4570-4579).

As used herein, the term "ROS1 fusion" refers to a portion of the ROS1 polypeptide comprising the kinase domain of the ROS1 protein (or polynucleotide encoding the same) fused to all or a portion of another polypeptide (or polynucleotide encoding the same), where the name of that second polypeptide or polynucleotide is named in the fusion. (The term "fusion" simply means all or a portion of a polypeptide or polynucleotide from first gene fused to all or a portion of a polypeptide or a polynucleotide from a second gene). For example, an SLC34A2-ROS1 fusion is a fusion between a portion of the SLC34A2 polypeptide (or polynucleotide encoding the same) and a portion of the ROS1 polypeptide (or polynucleotide encoding the same) comprising the kinase domain ROS1. An ROS1 fusion often results from a chromosomal translocation or inversion. There are numerous known ROS1 fusions, all of which are ROS 1 fusions of the invention and include, without limitation, the SLC34A2-ROS1 fusion proteins whose members include SLC34A2-ROS1(VS), SLC34A2-ROS1(S), SLC34A2-ROS1(L) (see U.S. Patent Publication No. 20100143918), CD74-ROS1 (see U.S. Patent Publication No. 20100221737) and the FIG-ROS1 fusion proteins whose members include FIG-ROS1(S), FIG-ROS1(L), and FIG-ROS1(XL) (see PCT Publication No. WO2010/093928), the disclosures of which are each incorporated herein in their entireties. The designation of (L), (S), (VS), (VL) (XS) or (XL) following a ROS1 polypeptide or ROS1 fusion, generally refers to long, short, very short, very long, extra short, and extra-long ROS1polypeptide or polynucleotide respectively.

All of the known ROS1 fusion proteins comprise the full kinase domain of full length ROS1. Thus, as used herein, by a "polypeptide with ROS1 kinase activity" (or "polypeptide having ROS1 kinase activity") is meant a protein (or polypeptide) that includes the full kinase domain of full length ROS1 protein and, thus, retains ROS1 kinase activity. Non-limiting examples of proteins with ROS1 kinase activity include, without limitation, full length ROS 1 protein, the SLC34A2-ROS1 fusion proteins, whose members include SLC34A2-ROS1(VS), SLC34A2-ROS1(S), SLC34A2-ROS1(L) (see U.S. Patent Publication No. 20100143918), CD74-ROS1 (see U.S. Patent Publication No. 20100221737) and the FIG-ROS1 fusion proteins whose members include FIG-ROS1(S), FIG-ROS1(L), and FIG-ROS1(XL) (see PCT Publication No. WO2010/093928), and any truncated or mutated form of ROS1 kinase that retains the kinase domain of full-length ROS1 kinase protein. An exemplary kinase domain of ROS1 is set forth in SEQ ID NO: 2, a "polypeptide with ROS1 kinase activity" is one whose amino acid sequence comprises SEQ ID NO: 2, or a kinase active portion thereof.

As used herein, "polypeptide" (or "amino acid sequence" or "protein") refers to a polymer formed from the linking, in a defined order, of preferably, a-amino acids, D-, L-amino acids, and combinations thereof. The link between one amino acid residue and the next is referred to as an amide bond or a peptide bond. Non-limiting examples of polypeptides include refers to an oligopeptide, peptide, polypeptide, or protein sequence, and fragments or portions thereof, and to naturally occurring or synthetic molecules. Polypeptides also include derivatized molecules such as glycoproteins and lipoproteins as well as lower molecular weight polypeptides. "Amino acid sequence" and like terms, such as "polypeptide" or "protein", are not meant to limit the indicated amino acid sequence to the complete, native amino acid sequence associated with the recited protein molecule.

It will be recognized in the art that some amino acid sequences of a polypeptide of the invention can be varied without significant effect of the structure or function of the mutant protein. If such differences in sequence are contemplated, it should be remembered that there will be critical areas on the protein which determine activity (e.g. the kinase domain of ROS1). In general, it is possible to replace residues that form the tertiary structure, provided that residues performing a similar function are used. In other instances, the type of residue may be completely unimportant if the alteration occurs at a non-critical region of the protein.

Thus, a polypeptide with ROS1 activity of the invention further includes variants of the full length ROS1 protein or the various ROS1 fusion polypeptides described herein that shows substantial ROS1 kinase activity. Some non-limiting conservative substitutions include the exchange, one for another, among the aliphatic amino acids Ala, Val, Leu and Ile; exchange of the hydroxyl residues Ser and Thr; exchange of the acidic residues Asp and Glu; exchange of the amide residues Asn and Gln; exchange of the basic residues Lys and Arg; and exchange of the aromatic residues Phe and Tyr. Further examples of conservative amino acid substitutions known to those skilled in the art are: Aromatic: phenylalanine tryptophan tyrosine (e.g., a tryptophan residue is replaced with a phenylalanine); Hydrophobic: leucine isoleucine valine; Polar: glutamine asparagines; Basic: arginine lysine histidine; Acidic: aspartic acid glutamic acid; Small: alanine serine threonine methionine glycine. As indicated in detail above, further guidance concerning which amino acid changes are likely to be phenotypically silent (i.e., are not likely to have a significant deleterious effect on a function) can be found in Bowie et al., Science 247, supra.

"SLC34A2" may refer to the sodium-dependent phosphate transport protein 2B encoded by the human SLC34A2 gene protein, or the SLC34A2 gene. The human SLC34A2 protein (isoform A, which has an NCBI Accession number NP_001171469) is a 689 amino acid protein. SLC34A2 is further described herein.

CD74 is also referred to as the HLA class II histocompatibility antigen gamma chain also known as HLA-DR antigens-associated invariant chain or CD74 (Cluster of Differentiation 74), is a protein that in humans is encoded by the CD74 gene. An illustrative human CD74 protein has an NCBI accession number of NP_001020329 and an mRNA accession number of NM_001025158. CD74 has 160 amino acids and is encoded by the CD74 gene located on chromosome 5 (5q32). CD74 has 9 exons. CD74 is further described herein.

FIG (Fused in Glioblastoma) is also referred to as "Golgi-associated PDZ and coiled-coil motif containing" or "GOPC" protein, is encoded by a gene located on chromosome 6 at 6q21. This protein in human has 462 amino acids. FIG has 3 isoforms produced by alternative splicing. An exemplary human FIG protein referred to in the present invention has a UniProtKB identifier of Q9HD26-1.

"SLC34A2-ROS1 fusion protein or gene" refers to somatic gene fusions of the partners SLC34A2 and ROS1. In some embodiments, the SLC34A2-ROS1 fusion protein including N-terminal domain of a fusion partner, such as SLC34A2 and the C-terminal kinase domain of ROS1 protein. The N-terminal domain of a fusion partner may be positioned at N-terminus of the fusion protein, and the C-terminal kinase domain of ROS1 protein may be positioned at the C-terminus of the fusion protein. The fusion partner may be a N-terminal domain of SLC34A2 protein, which is positioned at N-terminus of the fusion protein. In this case, the fusion protein may be represented as SLC34A2-ROS1 protein which includes N-terminal domain of SLC34A2 protein at N-terminus and C-terminal kinase domain of ROS1 protein at C-terminus. Another embodiment provides a fusion gene encoding the fusion protein, where a gene encoding the N-terminal domain of the fusion partner positions at 5' end and a gene encoding the C-terminal kinase domain of the ROS1 protein positions at 3' end. In some embodiments, portions of the SLC34A2 protein is fused in frame to portions of the ROS1 protein comprising a functional kinase domain as a result of an aberrant chromosomal translocation yielding a ROS1 chromosomal rearrangement with SLC34A2. In some embodiments, SLC34A2-ROS1 fusion protein can include the fusion SLC34A2 exon 4-ROS1 exon 32. In some embodiments, SLC34A2-ROS1 fusion protein can include the fusion SLC34A2 exon 4-ROS1 exon 34 SLC34A2-ROS1 fusion protein or gene having a functional ROS1 kinase domain as used in the present invention may contain other breakpoints involving the two translocation partners SLC34A2 and ROS1. Exemplary SLC34A2-ROS1 fusion proteins can be found in COSMIC-Catalogue of somatic mutations in cancer database v.68 at cancer.sanger.ac.uk/cancergenome/projects/cosmic/ the contents of which are incorporated herein by reference in its entirety.

"CD74-ROS1 fusion protein or gene" refers to somatic gene fusions of the partners CD74 and ROS1. Exemplary CD74-ROS1 fusion proteins can be found in COSMIC-Catalogue of somatic mutations in cancer database v.68 at cancer.sanger.ac.uk/cancergenome/projects/cosmic/ the contents of which are incorporated herein by reference in its entirety.

"FIG-ROS1 fusion protein or gene" refers to somatic gene fusions of the partners FIG and ROS1. In some embodiments, an infra-chromosomal homozygous deletion of 240 kilobases on human chromosome 6, at position 6q21 is responsible for the formation of the FIG-ROS 1 locus. In some embodiments, the FIG-ROS 1 transcript is encoded by 7 FIG exons and 9 ROS1-derived exons. In some embodiments, the FIG-ROS1 gene encodes for an in-frame fusion protein with a constitutively active and functional ROS1 kinase domain. Exemplary FIG-ROS1 fusion proteins can be found in COSMIC-Catalogue of somatic mutations in cancer database v.68 at cancer.sanger.ac.uk/cancergenome/projects/cosmic/ the contents of which are incorporated herein by reference in its entirety.

"Patient" for the purposes of the present invention includes humans and other animals, particularly mammals, and other organisms. Thus the methods are applicable to both human therapy and veterinary applications. In another embodiment the patient is a mammal, and in another embodiment the patient is human.

"Pharmaceutically acceptable salt" of a compound means a salt that is pharmaceutically acceptable and that possesses the desired pharmacological activity of the parent compound. It is understood that the phannaceutically acceptable salts are non-toxic. Additional information on suitable pharmaceutically acceptable salts can be found in Remington's Pharmaceutical Sciences, 17th ed., Mack Publishing Company, Easton, PA, 1985, which is incorporated herein by reference or S. M. Berge, et al., "Pharmaceutical Salts," J. Pharm. Sci., 1977;66:1-19 both of which are incorporated herein by reference.

Examples of pharmaceutically acceptable acid addition salts include those formed with inorganic acids such as hydrochloric acid, hydrobromic acid, sulfuric acid, nitric acid, phosphoric acid, and the like; as well as organic acids such as acetic acid, trifluoroacetic acid, propionic acid, hexanoic acid, cyclopentanepropionic acid, glycolic acid, pyruvic acid, lactic acid, oxalic acid, maleic acid, malonic acid, succinic acid, fumaric acid, tartaric acid, malic acid, citric acid, benzoic acid, cinnamic acid, 3-(4-hydroxybenzoyl)benzoic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, p-toluenesulfonic acid, and salicylic acid and the like.

"Prodrug" refers to compounds that are transformed (typically rapidly) *in vivo* to yield the parent compound of the above formulae, for example, by hydrolysis in blood. Common examples include, but are not limited to, ester and amide forms of a compound having an active form bearing a carboxylic acid moiety. Examples of pharmaceutically acceptable esters of the compounds of this invention include, but are not limited to, alkyl esters (for example with between about one and about six carbons) the alkyl group is a straight or branched chain. Acceptable esters also include cycloalkyl esters and arylalkyl esters such as, but not limited to benzyl. Examples of pharmaceutically acceptable amides of the compounds of this invention include, but are not limited to, primary amides, and secondary and tertiary alkyl amides (for example with between about one and about six carbons). Amides and esters of the compounds of the present invention may be prepared according to conventional methods. A thorough discussion of prodrugs is provided in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987, both of which are incorporated herein by reference for all purposes.

"ROS1" or the "ROS1 protein" is a transmembrane receptor tyrosine kinase, and is further described herein.

"Therapeutically effective amount" is an amount of a compound of the invention, that when administered to a patient, ameliorates a symptom of the disease. A therapeutically effective amount is intended to include an amount of a compound alone or in combination with other active ingredients effective to modulate c-Met, and/or VEGFR2, or effective to treat or prevent cancer. The amount of a compound of the invention which constitutes a "therapeutically effective amount" will vary depending on the compound, the disease state and its severity, the age of the patient to be treated, and the like. The therapeutically effective amount can be determined by one of ordinary skill in the art having regard to their knowledge and to this disclosure.

"Treating" or "treatment" of a disease, disorder, or syndrome, as used herein, includes (i) preventing the disease, disorder, or syndrome from occurring in a human, i.e. causing the clinical symptoms of the disease, disorder, or syndrome not to develop in an animal that may be exposed to or predisposed to the disease, disorder, or syndrome but does not yet experience or display symptoms of the disease, disorder, or syndrome; (ii) reversing or inhibiting the disease, disorder, or syndrome, i.e., arresting its development; and (iii) relieving the disease, disorder, or syndrome, i.e., causing regression of the disease, disorder, or syndrome. As is known in the art, adjustments for systemic versus localized delivery, age, body weight, general health, sex, diet, time of administration, drug interaction and the severity of the condition may be necessary, and will be ascertainable with routine experience.

"Yield" for each of the reactions described herein is expressed as a percentage of the theoretical yield.

### Embodiments

In one embodiment the compound of Formula I is the compound of Formula Ia: 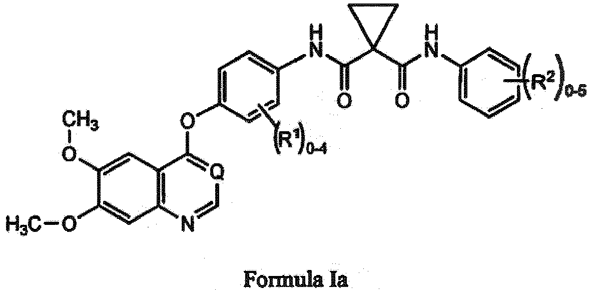 or a pharmaceutically acceptable salt thereof, wherein:
R¹ is halo;
R² is halo; and
Q isCHorN.

In another embodiment, the compound of Formula I is compound 1: 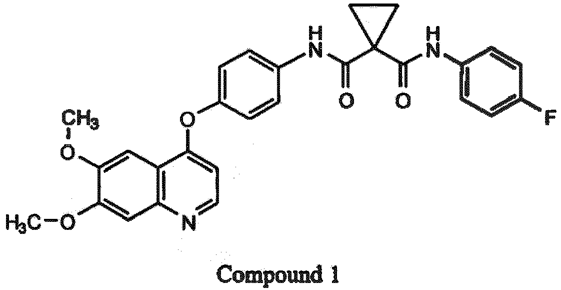 or a pharmaceutically acceptable salt thereof. As indicated previously, compound 1 is referred to herein as N-(4-{[6,7-bis(methyloxy)quinolin-4-yl]oxy}phenyl)-N'-(4-fluorophenyl)cyclopropane-1,1-dicarboxamide. WO 2005/030140, which is incorporated herein by reference in its entirety, discloses compound 1 and describes how it is made (WO 2005/030140, Example 12,37,38, and 48) and also discloses the therapeutic activity of this compound to inhibit, regulate and/or modulate the signal transduction of kinases, (WO 2005/030140, Assays, Table 4, entry 289). Example 48 is on paragraph [0353] in WO 2005/030140.

In other embodiments, the compound of Formula I, Ia, or compound 1, or a pharmaceutically acceptable salt thereof, is administered as a pharmaceutical composition, wherein the pharmaceutical composition additionally comprises a pharmaceutically acceptable carrier, excipient, or diluent. In a specific embodiment, the compound of Formula I is compound 1.

The compound of Formula I, Formula Ia and compound I, as described herein, includes both the recited compounds as well as individual isomers and mixtures of isomers. In each instance, the compound of Formula I includes the pharmaceutically acceptable salts, hydrates, and/or solvates of the recited compounds and any individual isomers or mixture of isomers thereof.

In other embodiments, the compound of Formula I, Ia, or compound 1 can be the (L)-malate salt. The malate salt of the compound of Formula I and of compound 1 is disclosed in PCT/US2010/021194 and USSN 61/325095, both of which are incorporated herein by reference in their entireties.

In other embodiments, the compound of Formula I is the (D)-malate salt, which is also referred to as the R-malate salt

In other embodiments, the compound of Formula Ia is the malate salt

In other embodiments, the compound of Formula Ia is the (L)-malate salt, which is also referred to as the S-malate salt.

In other embodiments, compound 1 is the (D)-malate salt, which is also referred to as the S-malate salt

In other embodiments, compound 1 is the malate salt.

In other embodiments, compound 1 is the (L)-malate salt, which is also referred to as the S-malate salt.

In another embodiment, the malate salt is in the crystalline N-1 form or the N-2 form of the (L) malate salt and/or the (D) malate salt of compound 1 as disclosed in United States patent Application Ser. No. 61/325095. *Also see* WO 2008/083319, incorporated by reference in its entirety, for the properties of crystalline enantiomers, including the N-1 and/or the N-2 crystalline forms of the malate salt of compound 1. Methods of making and characterizing such forms are fully described in PCT/US10/021194, which is incorporated herein by reference in its entirety.

In another embodiment, the invention is directed to a method for reversing or inhibiting NSCLC, comprising administering to a patient in need of such treatment a therapeutically effective amount of a compound of Formula I or a pharmaceutically acceptable salt thereof, in any of the embodiments disclosed herein. In a specific embodiment, the compound of Formula I is compound 1, or a pharmaceutically acceptable salt thereof.

In another embodiment, the invention is directed to a method for reversing or inhibiting SLC34A2-ROS1 fusion-positive NSCLC, comprising administering to a patient in need of such treatment a therapeutically effective amount of a compound of Formula I or a pharmaceutically acceptable salt thereof, in any of the embodiments disclosed herein. In a specific embodiment, the compound of Formula I is compound 1 or a pharmaceutically acceptable salt thereof.

In another embodiment, the invention is directed to a method for reversing or inhibiting CD74-ROS1 fusion-positive NSCLC, comprising administering to a patient in need of such treatment a therapeutically effective amount of a compound of Formula I or a pharmaceutically acceptable salt thereof, in any of the embodiments disclosed herein. In a specific embodiment, the compound of Formula I is compound 1 or a pharmaceutically acceptable salt thereof

In another embodiment, the invention is directed to a method for reversing or inhibiting FIG-ROS1 fusion-positive NSCLC, comprising administering to a patient in need of such treatment a therapeutically effective amount of a compound of Formula I or a pharmaceutically acceptable salt thereof, in any of the embodiments disclosed herein. In a specific embodiment, the compound of Formula I is compound 1 or a pharmaceutically acceptable salt thereof.

In another embodiment, the compound of Formula I, or a pharmaceutically acceptable salt thereof, is administered before, concurrently, or subsequent to one or more other treatments. In another embodiment, the compound of Formula I, or a pharmaceutically acceptable salt thereof, is administered subsequent to one or more treatments. "Treatment" means any of the treatment options are available to the skilled artisan, including surgery, chemotherapeutic agents, hormone therapies, antibodies, immunotherapies, radioactive iodine therapy, and radiation. In particular, "treatment' means another chemotherapeutic agent or antibody.

Thus, in another embodiment, the compound of Formula I, or a pharmaceutically acceptable salt thereof, is administered post-cisplatin and/or gemcitabine treatment

In another embodiment, the compound of Formula I, or a pharmaceutically acceptable salt thereof, is administered post-docetaxel treatment.

In another embodiment, the compound of Formula I, or a pharmaceutically acceptable salt thereof, is administered post HER-2 antibody treatment In another embodiment, the HER-2 antibody is trastuzumab.

In another embodiment, the compound of Formula I, or a pharmaceutically acceptable salt thereof, is administered post-cisplatin and/or gemcitabine and/or docetaxel treatment.

In another embodiment, the compound of Formula I, Ia, or compound 1, or a pharmaceutically acceptable salt thereof, is administered orally once daily as a tablet or capsule. In these and other embodiments, the compound of Formula I, or a pharmaceutically acceptable salt thereof, is compound 1, or a pharmaceutically acceptable salt thereof.

In another embodiment, compound 1 is administered orally as its free base or the malate salt as a capsule or tablet.

In another embodiment, compound 1 is administered orally once daily as its free base or as the malate salt as a capsule or tablet containing up to 100 mg of compound 1.

In another embodiment, compound 1 is administered orally once daily as its free base or as the malate salt as a capsule or tablet containing 100 mg of compound 1.

In another embodiment, compound 1 is administered orally once daily as its free base or as the malate salt as a capsule or tablet containing 95 mg of compound 1.

In another embodiment, compound 1 is administered orally once daily as its free base or as the malate salt as a capsule or tablet containing 90 mg of compound 1.

In another embodiment, compound 1 is administered orally once daily as its free base or as the malate salt as a capsule or tablet containing 85 mg of compound 1.

In another embodiment, compound 1 is administered orally once daily as its free base or as the malate salt as a capsule or tablet containing 80 mg of compound 1.

In another embodiment, compound 1 is administered orally once daily as its free base or as the malate salt as a capsule or tablet containing 75 mg of compound 1.

In another embodiment, compound 1 is administered orally once daily as its free base or as the malate salt as a capsule or tablet containing 70 mg of compound 1.

In another embodiment, compound 1 is administered orally once daily as its free base or as the malate salt as a capsule or tablet containing 65 mg of compound 1.

In another embodiment, compound 1 is administered orally once daily as its free base or as the malate salt as a capsule or tablet containing 60 mg of compound 1.

In another embodiment, compound 1 is administered orally once daily as its free base or as the malate salt as a capsule or tablet containing 55 mg of compound 1.

In another embodiment, compound 1 is administered orally once daily as its free base or as the malate salt as a capsule or tablet containing 50 mg of compound 1.

In another embodiment, compound 1 is administered orally once daily as its free base or as the malate salt as a capsule or tablet containing 45 mg of compound 1.

In another embodiment, compound 1 is administered orally once daily as its free base or as the malate salt as a capsule or tablet containing 40 mg of compound 1.

In another embodiment, compound 1 is administered orally once daily as its free base or as the malate salt as a capsule or tablet containing 35 mg of compound 1.

In another embodiment, compound 1 is administered orally once daily as its free base or as the malate salt as a capsule or tablet containing 30 mg of compound 1.

In another embodiment, compound 1 is administered orally once daily as its free base or as the malate salt as a capsule or tablet containing 25 mg of compound 1.

In another embodiment, compound 1 is administered orally once daily as its free base or as the malate salt as a capsule or tablet containing 20 mg of compound 1.

In another embodiment, compound 1 is administered orally once daily as its free base or as the malate salt as a capsule or tablet containing 15 mg of compound 1.

In another embodiment, compound 1 is administered orally once daily as its free base or as the malate salt as a capsule or tablet containing 10 mg of compound 1.

In another embodiment, compound 1 is administered orally once daily as its free base or as the malate salt as a capsule or tablet containing 5 mg of compound 1.

In another embodiment, compound 1 is administered as its free base or malate salt orally once daily as a tablet as provided in the following table.

| **Ingredient** | **(% w/w)** |
|---|---|
| Compound 1 | 31.68 |
| Microcrystalline Cellulose | 38.85 |
| Lactose anhydrous | 19.42 |
| Hydroxypropyl Cellulose | 3.00 |
| Croscarmellose Sodium | 3.00 |
| **Total Intra-ranular** | **95.95** |
| Silicon dioxide, Colloidal | 0.30 |
| Croscarmellose Sodium | 3.00 |
| Magnesium Stearate | 0.75 |
| **Total** | **100.00** |

In another embodiment, compound 1 is administered orally as its free base or malate salt once daily as a tablet as provided in the following table.

| **Ingredient** | **(% w/w)** |
|---|---|
| Compound 1 | 25.0-33.3 |
| Microcrystalline Cellulose | q.s |
| Hydroxypropyl Cellulose | 3 |
| Poloxamer | 0-3 |
| Croscarmellose Sodium | 6.0 |
| Colloidal Silicon Dioxide | 0.5 |
| Magnesium Stearate | 0.5-1.0 |
| Total | 100 |

In another embodiment, compound 1 is administered orally as its free base or malate salt once daily as a tablet as provided in the following table.

| **Ingredient** | **Theoretical Quantity (mg/unit dose)** |
|---|---|
| Compound 1 | 100.0 |
| Microcrystalline Cellulose PH-102 | 155.4 |
| Lactose Anhydrous 60M | 77.7 |
| Hydroxypropyl Cellulose, EXF | 12.0 |
| Croscarmellose Sodium | 24 |
| Colloidal Silicon Dioxide | 1.2 |
| Magnesium Stearate (Non-Bovine) | 3.0 |
| Opadry Yellow | 16.0 |
| **Total** | **416** |

Any of the tablet formulations provided above can be adjusted according to the dose of compound 1, or a pharmaceutically acceptable salt thereof desired. Thus, the amount of each of the formulation ingredients can be proportionally adjusted to provide a table formulation containing various amounts of compound 1 as provided in the previous paragraphs. In another embodiment, the formulations can contain 20, 40, 60, or 80 mg of compound 1, or a pharmaceutically acceptable salt thereof

In some embodiments, the invention provides a method for inhibiting or reversing the progress of abnormal cell growth in a mammal, comprising administering a compound of Formula I, or a pharmaceutically acceptable salt thereof, wherein the abnormal cell growth is cancer mediated by a ROS1 fusion protein, for example, SLC34A2-ROS1 fusion protein, CD74-ROS1 fusion protein, FIG-ROS1 fusion protein, FIG-ROS1(L) fusion protein, FIG-ROS1(S) fusion protein, and FIG-ROS1(VL) fusion protein, and other ROS1 fusion proteins comprising a functional C-terminal ROS1 kinase domain as encoded by the ROS1 gene on chromosome 6 in humans (6q22). In one embodiment, the cancer is lung adenocarcinoma. In another, the lung adenocarcinoma is non-small cell lung cancer. In another embodiment, the lung adenocarcinoma is SLC34A2-ROS1 fusion-positive non-small cell lung cancer. In another embodiment, the lung adenocarcinoma is CD74-ROS1 fusion-positive non-small cell lung cancer. In another embodiment, the lung adenocarcinoma is FIG-ROS1 fusion-positive non-small cell lung cancer. In another embodiment, the lung adenocarcinoma is a ROS1 fusion-positive non-small cell lung cancer. In another embodiment, a compound of Formula I, or a pharmaceutically acceptable salt thereof is administered as a pharmaceutical composition comprising a compound of Formula I, or a pharmaceutically acceptable salt thereof and at least one pharmaceutically acceptable carrier. In another embodiment, the compound of Formula I is administered subsequent to another form of treatment In another embodiment, a compound of Formula I, or a pharmaceutically acceptable salt thereof, is administered post-cisplatin and/or gemcitabine treatment. In another embodiment, a compound of Formula I or a pharmaceutically acceptable salt thereof, is administered post-carboplatin and/or gemcitabine treatment. In another embodiment, a compound of Formula I is administered post-crizotinib treatment In another embodiment, a compound of Formula I, or a pharmaceutically acceptable salt thereof, is administered post-crizotinib and/or gemcitabine treatment. In another embodiment, a compound of Formula I or a pharmaceutically acceptable salt thereof, is administered post-docetaxel treatment In another embodiment, a compound of Formula I is administered post-carboplatin treatment In another embodiment, a compound of Formula I or a pharmaceutically acceptable salt thereof, is administered post-cisplatin and/or gemcitabine and/or docetaxel treatment. In another embodiment, a compound of Formula I or a pharmaceutically acceptable salt thereof, is administered post-carboplatin and/or gemcitabine and/or docetaxel treatment. In another embodiment, a compound of Formula I or a pharmaceutically acceptable salt thereof, is administered post-crizotinib and/or gemcitabine and/or docetaxel treatment.

In some embodiments, the invention provides a method for inhibiting or reversing the progress of abnormal cell growth in a mammal, comprising administering a compound of Formula Ia, or a pharmaceutically acceptable salt thereof, wherein the abnormal cell growth is cancer mediated by a ROS1 fusion protein, for example, SLC34A2-ROS1 fusion protein, CD74-ROS1 fusion protein, FIG-ROS1 fusion protein, FIG-ROS1(L) fusion protein, FIG-ROS1(S) fusion protein, and FIG-ROS1(VL) fusion protein, and other ROS1 fusion proteins comprising a functional C-terminal ROS1 kinase domain as encoded by the ROS1 gene on chromosome 6 in humans (6q22). In one embodiment, the cancer is lung adenocarcinoma. In another, the lung adenocarcinoma is non-small cell lung cancer. In another embodiment, the lung adenocarcinoma is SLC34A2-ROS 1 fusion-positive non-small cell lung cancer. In another embodiment, the lung adenocarcinoma is CD74-ROS1 fusion-positive non-small cell lung cancer. In another embodiment, the lung adenocarcinoma is FIG-ROS1 fusion-positive non-small cell lung cancer. In another embodiment, the lung adenocarcinoma is a ROS1 fusion-positive non-small cell lung cancer. In another embodiment, a compound of Formula Ia, or a pharmaceutically acceptable salt thereof is administered as a pharmaceutical composition comprising a compound of Formula Ia, or a pharmaceutically acceptable salt thereof and at least one pharmaceutically acceptable carrier. In another embodiment, the compound of Formula Ia is administered subsequent to another form of treatment. In another embodiment, a compound of Formula Ia, or a pharmaceutically acceptable salt thereof, is administered post-cisplatin and/or gemcitabine treatment. In another embodiment, a compound of Formula Ia, or a pharmaceutically acceptable salt thereof, is administered post-docetaxel treatment. In another embodiment, a compound of Formula Ia, or a pharmaceutically acceptable salt thereof, is administered post-cisplatin and/or gemcitabine and/or docetaxel treatment In another embodiment, a compound of Formula Ia or a pharmaceutically acceptable salt thereof is administered post-carboplatin and/or gemcitabine treatment. In another embodiment, a compound of Formula Ia, or a pharmaceutically acceptable salt thereof, is administered post-crizotinib treatment. In another embodiment, a compound of Formula Ia, or a pharmaceutically acceptable salt thereof, is administered post-crizotinib and/or gemcitabine treatment. In another embodiment, a compound of Formula Ia or a pharmaceutically acceptable salt thereof, is administered post-docetaxel treatment. In another embodiment, a compound of Formula Ia is administered post-carboplatin treatment In another embodiment, a compound of Formula Ia or a pharmaceutically acceptable salt thereof, is administered post-cisplatin and/or gemcitabine and/or docetaxel treatment. In another embodiment, a compound of Formula Ia or a pharmaceutically acceptable salt thereof, is administered post-carboplatin and/or gemcitabine and/or docetaxel treatment. In another embodiment, a compound of Formula Ia or a pharmaceutically acceptable salt thereof, is administered post-crizotinib and/or gemcitabine and/or docetaxel treatment.

In other embodiments, the invention provides a method for inhibiting or reversing the progress of abnormal cell growth in a mammal, comprising administering compound 1 or a pharmaceutically acceptable salt thereof, wherein the abnormal cell growth is cancer mediated by a ROS1 fusion protein, for example, SLC34A2-ROS1 fusion protein, CD74-ROS1 fusion protein, FIG-ROS1 fusion protein, FIG-ROS1(L) fusion protein, FIG-ROS1(S) fusion protein, and FIG-ROS1(VL) fusion protein, and other ROS1 fusion proteins comprising a functional C-terminal ROS1 kinase domain as encoded by the ROS1 gene on chromosome 6 in humans (6q22). In one embodiment, the cancer is lung adenocarcinoma. In another, the lung adenocarcinoma is non-small cell lung cancer. In another embodiment, the lung adenocarcinoma is SLC34A2-ROS1 fusion-positive non-small cell lung cancer. In another embodiment, the lung adenocarcinoma is CD74-ROS1 fusion-positive non-small cell lung cancer. In another embodiment, the lung adenocarcinoma is FIG-ROS1 fusion-positive non-small cell lung cancer. In another embodiment, the lung adenocarcinoma is a ROS1 fusion-positive non-small cell lung cancer. In another embodiment, compound 1 or a pharmaceutically acceptable salt thereof is administered as a pharmaceutical composition comprising compound 1 or a pharmaceutically acceptable salt thereof and at least one pharmaceutically acceptable carrier. In another embodiment, compound 1 is administered subsequent to another form of treatment In another embodiment, compound 1, or a pharmaceutically acceptable salt thereof, is administered post-cisplatin and/or gemcitabine treatment. In another embodiment, compound 1, or a pharmaceutically acceptable salt thereof, is administered post-docetaxel treatment In another embodiment, compound 1, or a pharmaceutically acceptable salt thereof, is administered post-cisplatin and/or gemcitabine and/or docetaxel treatment. In another embodiment, compound 1 or a pharmaceutically acceptable salt thereof, is administered post-carboplatin and/or gemcitabine treatment. In another embodiment, compound 1 or a pharmaceutically acceptable salt thereof is administered post-crizotinib treatment. In another embodiment, compound 1, or a pharmaceutically acceptable salt thereof, is administered post-crizotinib and/or gemcitabine treatment. In another embodiment, compound 1, or a pharmaceutically acceptable salt thereof, is administered post-docetaxel treatment In another embodiment, compound 1, or a pharmaceutically acceptable salt thereof, is administered post-carboplatin treatment. In another embodiment, compound 1, or a pharmaceutically acceptable salt thereof, is administered post-cisplatin and/or gemcitabine and/or docetaxel treatment. In another embodiment, compound 1, or a pharmaceutically acceptable salt thereof, is administered post-carboplatin and/or gemcitabine and/or docetaxel treatment. In another embodiment, compound 1, or a pharmaceutically acceptable salt thereof, is administered post-crizotinib and/or gemcitabine and/or docetaxel treatment In another embodiment, compound 1, or a pharmaceutically acceptable salt thereof, is administered post-crizotinib and/or carboplatin treatment.

In another embodiment, the invention provides a method for preventing, treating, inhibiting or reversing the progress of abnormal cell growth in a mammal, comprising administering compound 1, or a pharmaceutically acceptable salt thereof, wherein the abnormal cell growth is a cancer mediated by a ROS1 fusion protein, for example, SLC34A2-ROS 1 fusion protein, CD74-ROS1 fusion protein, FIG-ROS1 fusion protein, FIG-ROS1(L) fusion protein, FIG-ROS1(S) fusion protein, FIG-ROS1(VL) fusion protein, or other ROS1 fusion proteins, wherein the cancer has been previously treated with a therapeutic regimen of crizotinib, and/or carboplatin and is resistant to crizotinib, and/or carboplatin. In some embodiments, the cancer that is resistant to crizotinib, and/or carboplatin, harbors one or more ROS1 fusion proteins selected from: SLC34A2-ROS1 fusion protein, CD74-ROS1 fusion protein, FIG-ROS1 fusion protein, FIG-ROS1(L) fusion protein, FIG-ROS1(S) fusion protein, and FIG-ROS1(VL) fusion protein, and/or contains a mutation in the ROS1 kinase domain of the SLC34A2-ROS 1 fusion protein, CD74-ROS1 fusion protein, FIG-ROS1 fusion protein, FIG-ROS1(L) fusion protein, FIG-ROS1(S) fusion protein, or FIG-ROS1 (VL) fusion protein. In one embodiment, the crizotinib and/or carboplatin resistant cancer is a cancer having a mutation in the ROS1 kinase domain of a CD74-ROS1 fusion protein. In a related embodiment, the cancer is a crizotinib refractory CD74-ROS1 fusion positive lung adenocarcinoma, for example, a crizotinib refractory CD74-ROS1 fusion positive NSCLC, harboring a mutation in the ROS1 kinase domain. In another embodiment, the crizotinib and/or carboplatin resistant cancer is a cancer, for example, a crizotinib resistant lung adenocarcinoma, for example, a crizotinib resistant NSCLC, harboring a mutation selected from E1990G, G2032R, L2026M, L1951R, M2128V, K2003I, and combinations thereof in the ROS1 kinase domain of a CD74-ROS1fusion protein.

In another embodiment, the invention provides a method for preventing, treating, inhibiting or reversing the progress of abnormal cell growth in a mammal, for example, a cancer, the method comprising administering a compound of Formula I, or a pharmaceutically acceptable salt thereof, wherein the abnormal cell growth is a cancer mediated by a ROS1 fusion protein, for example, SLC34A2-ROS1 fusion protein, CD74-ROS1 fusion protein, FIG-ROS1 fusion protein, FIG-ROS1(L) fusion protein, FIG-ROS1(S) fusion protein, FIG-ROS1(VL) fusion protein, or other ROS1 fusion proteins, wherein the cancer has been previously treated with a therapeutic regimen of crizotinib, and/or carboplatin and is resistant to crizotinib, and/or carboplatin. In some embodiments, the cancer that is resistant to crizotinib, and/or carboplatin, harbors one or more ROS1 fusion proteins selected from: SLC34A2-ROS1 fusion protein, CD74-ROS1 fusion protein, FIG-ROS1 fusion protein, FIG-ROS1(L) fusion protein, FIG-ROS1(S) fusion protein, and FIG-ROS1(VL) fusion protein, and/or contains a mutation in the ROS1 kinase domain of the SLC34A2-ROS1 fusion protein, CD74-ROS1 fusion protein, FIG-ROS1 fusion protein, FIG-ROS1(L) fusion protein, FIG-ROS1(S) fusion protein, or FIG-ROS1(VL) fusion protein. In one embodiment, the crizotinib resistant cancer is a cancer having a mutation in the ROS1 kinase domain of a CD74-ROS1 fusion protein. In a related embodiment, the cancer is a crizotinib refractory CD74-ROS1 fusion positive lung adenocarcinoma, for example, a crizotinib refractory CD74-ROS1 fusion positive NSCLC, harboring a mutation in the ROS1 kinase domain. In another embodiment, the crizotinib resistant cancer is a cancer, for example, a crizotinib resistant lung adenocarcinoma, for example, a crizotinib resistant NSCLC, harboring a mutation selected from E1990G, G2032R, L2026M, L1951R, M2128V, K2003I, and combinations thereof in the ROS1 kinase domain of a CD74-ROS1 fusion protein. In various embodiments described above, a method for preventing, treating, inhibiting or reversing the progress of abnormal cell growth in a mammal, comprises administering a compound of Formula I, or a compound of Formula Ia, or compound 1, or a pharmaceutically acceptable salt thereof, in an exemplary therapeutically effective dose ranging from about 0.01mg/kg to about 100 mg/kg, or from about 0.1 mg/kg to about 75 mg/kg, or from about 0.1 mg/kg to about 50 mg/kg, or from about 0.1 mg/kg to about 40 mg/kg, or from about 0.1 mg/kg to about 30 mg/kg, or from about 0.1 mg/kg to about 20 mg/kg, or from about 0.1 mg/kg to about 15 mg/kg, or from about 0.1 mg/kg to about 10 mg/kg or from about 0.1 mg/kg to about 5 mg/kg, or from about 0.1 mg/kg to about 1 mg/kg to a patient in need thereof, wherein the abnormal cell growth is a cancer mediated by a ROS1 fusion protein, for example, SLC34A2-ROS1 fusion protein, CD74-ROS1 fusion protein, FIG-ROS1 fusion protein, FIG-ROS1(L) fusion protein, FIG-ROS1(S) fusion protein, FIG-ROS1(VL) fusion protein, or other ROS1 fusion protein; or a cancer harboring one or more mutations in a ROS1 fusion protein, for example, a mutation in a ROS1 kinase domain in a SLC34A2-ROS1 fusion protein, CD74-ROS1 fusion protein, FIG-ROS1 fusion protein, FIG-ROS1(L) fusion protein, FIG-ROS1(S) fusion protein, FIG-ROS1(VL) fusion protein, or other ROS1 fusion proteins, including in some embodiments, a cancer that has been previously treated with a therapeutic regimen of crizotinib, and/or carboplatin and is resistant to crizotinib, and/or carboplatin, for example, a crizotinib resistant cancer, for example, a crizotinib resistant lung adenocarcinoma, for example, a crizotinib resistant NSCLC.

### Administration

Administration of the compound of Formula I, Formula Ia, or compound 1, or a pharmaceutically acceptable salt thereof, in pure form or in an appropriate pharmaceutical composition, can be carried out via any of the accepted modes of administration or agents for serving similar utilities. Thus, administration can be, for example, orally, nasally, parenterally (intravenous, intramuscular, or subcutaneous), topically, transdermally, intravaginally, intravesically, intracistemally, or rectally, in the form of solid, semi-solid, lyophilized powder, or liquid dosage forms, such as for example, tablets, suppositories, pills, soft elastic and hard gelatin dosages (which can be in capsules or tablets), powders, solutions, suspensions, or aerosols, or the like, specifically in unit dosage forms suitable for simple administration of precise dosages.

The compositions will include a conventional pharmaceutical carrier or excipient and a compound of Formula I as the/an active agent, and, in addition, may include carriers and adjuvants, etc.

Adjuvants include preserving, wetting, suspending, sweetening, flavoring, perfuming, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

If desired, a pharmaceutical composition of the compound of Formula I may also contain minor amounts of auxiliary substances such as wetting or emulsifying agents, pH buffering agents, antioxidants, and the like, such as, for example, citric acid, sorbitan monolaurate, triethanolamine oleate, butylalted hydroxytoluene, etc.

The choice of composition depends on various factors such as the mode of drug administration (e.g., for oral administration, compositions in the form of tablets, pills or capsules) and the bioavailability of the drug substance. Recently, pharmaceutical compositions have been developed especially for drugs that show poor bioavailability based upon the principle that bioavailability can be increased by increasing the surface area i.e., decreasing particle size. For example, U.S. Pat No. 4,107,288 describes a pharmaceutical composition having particles in the size range from 10 to 1,000 nm in which the active material is supported on a crosslinked matrix of macromolecules. U.S. Pat. No. 5,145,684 describes the production of a pharmaceutical composition in which the drug substance is pulverized to nanoparticles (average particle size of 400 nm) in the presence of a surface modifier and then dispersed in a liquid medium to give a pharmaceutical composition that exhibits remarkably high bioavailability.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

One specific route of administration is oral, using a convenient daily dosage regimen that can be adjusted according to the degree of severity of the disease-state to be treated.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, cellulose derivatives, starch, alignates, gelatin, polyvinylpyrrolidone, sucrose, and gum acacia, (c) humectants, as for example, glycerol, (d) disintegrating agents, as for example, agar-agar, calcium carbonate, potato or tapioca starch, alginic acid, croscannellose sodium, complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, magnesium stearate and the like (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid dosage forms as described above can be prepared with coatings and shells, such as enteric coatings and others well known in the art. They may contain pacifying agents, and can also be of such composition that they release the active compound or compounds in a certain part of the intestinal tract in a delayed manner. Examples of embedded compositions that can be used are polymeric substances and waxes. The active compounds can also be in microencapsulated form, if appropriate, with one or more of the above-mentioned excipients.

Liquid dosage forms for oral administration include pharmaceutically acceptable emulsions, solutions, suspensions, syrups, and elixirs. Such dosage forms are prepared, for example, by dissolving, dispersing, etc., the compound of Formula I, or a pharmaceutically acceptable salt thereof, and optional pharmaceutical adjuvants in a carrier, such as, for example, water, saline, aqueous dextrose, glycerol, ethanol and the like; solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide; oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan; or mixtures of these substances, and the like, to thereby form a solution or suspension.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administration are, for example, suppositories that can be prepared by mixing the compound of Formula I, or a pharmaceutically acceptable salt thereof, with, for example, suitable non-irritating excipients or carriers such as cocoa butter, polyethyleneglycol or a suppository wax, which are solid at ordinary temperatures but liquid at body temperature and therefore, melt while in a suitable body cavity and release the active component therein.

Dosage forms for topical administration of the compound of Formula I, or a pharmaceutically acceptable salt thereof, include ointments, powders, sprays, and inhalants. The active component is admixed under sterile conditions with a physiologically acceptable carrier and any preservatives, buffers, or propellants as may be required. Ophthalmic compositions, eye ointments, powders, and solutions are also contemplated as being within the scope of this disclosure.

Compressed gases may be used to disperse the compound of Formula I, or a pharmaceutically acceptable salt thereof, in aerosol form. Inert gases suitable for this purpose are nitrogen, carbon dioxide, etc.

Generally, depending on the intended mode of administration, the pharmaceutically acceptable compositions will contain about 1% to about 99% by weight of a compound(s) of Formula I, or a pharmaceutically acceptable salt thereof, and 99% to 1% by weight of a suitable pharmaceutical excipient In one example, the composition will be between about 5% and about 75% by weight of a compound(s) of Formula I, Formula Ia, or compound 1, or a pharmaceutically acceptable salt thereof, with the rest being suitable pharmaceutical excipients.

Actual methods of preparing such dosage forms are known, or will be apparent, to those skilled in this art; for example, see Remington's Pharmaceutical Sciences, 18th Ed., (Mack Publishing Company, Easton, Pa., 1990). The composition to be administered will, in any event, contain a therapeutically effective amount of a compound of Formula I, or a pharmaceutically acceptable salt thereof, for treatment of a disease-state in accordance with the teachings of this disclosure.

The compounds of this disclosure, or their pharmaceutically acceptable salts or solvates, are administered in a therapeutically effective amount which will vary depending upon a variety of factors including the activity of the specific compound employed, the metabolic stability and length of action of the compound, the age, body weight, general health, sex, diet, mode and time of administration, rate of excretion, drug combination, the severity of the particular disease-states, and the host undergoing therapy. The compound of Formula I, Formula Ia, or compound 1, or a pharmaceutically acceptable salt thereof can be administered to a patient at dosage levels in the range of about 0.1 to about 1,000 mg per day. For a normal human adult having a body weight of about 70 kilograms, a dosage in the range of about 0.01 to about 100 mg per kilogram of body weight per day is an example. The specific dosage used, however, can vary. For example, the dosage can depend on a number of factors including the requirements of the patient, the severity of the condition being treated, and the pharmacological activity of the compound being used. The determination of optimum dosages for a particular patient is well known to one of ordinary skill in the art.

In other embodiments, the compound of Formula I, Formula Ia, or compound 1, or a pharmaceutically acceptable salt thereof, can be administered to the patient concurrently with other cancer treatments. Such treatments include other cancer chemotherapeutics, hormone replacement therapy, radiation therapy, or immunotherapy, among others. The choice of other therapy will depend on a number of factors including the metabolic stability and length of action of the compound, the age, body weight, general health, sex, diet, mode and time of administration, rate of excretion, drug combination, the severity of the particular disease-states, and the host undergoing therapy.

In another aspect, the invention provides a method for detecting, diagnosing and treating ROS1 fusions related disease such as SLC34A2-ROS1 fusion-positive NSCLC, CD74-ROS1 fusion-positive NSCLC, and FIG-ROS1 fusion-positive NSCLC, in addition to other ROS1 fusion positive NSCLC. We describe in more detail here methods for the detection and diagnosing of such disorders. Treating these disorders can be better accomplished with the administering of a therapeutically effective amount of a pharmaceutical composition comprising a compound of Formula I or the malate salt of a compound of Formula I or another pharmaceutically acceptable salt of a compound of Formula I, including in a specific embodiment, the compound of Formula I is compound 1 or the malate salt of compound 1 to a patient who has been identified or diagnosed as having a ROS1 fusion related disease such as SLC34A2-ROS1 fusion-positive NSCLC, CD74-ROS1 fusion-positive NSCLC, and FIG-ROS1 fusion-positive NSCLC.

In another aspect, a compound of Formula I, or a pharmaceutically acceptable salt thereof, is administered to a patient for preventing or treating a lung cancer. In some of these embodiments, the method comprises administering to the patient in need thereof, a composition comprising at least one inhibitor against an ROS1 fusion protein, at least one inhibitor against the ROS1 fusion gene encoding the fusion protein, at least one inhibitor against a ROS1 coding gene, or a combination thereof as an active ingredient

### ROS1 and Fusion Partners SLC34A2, CD74 and FIG

The ROS1 protein, SLC34A2 protein, CD74 protein and FIG protein, the SLC34A2-ROS1 fusion, or the CD74-ROS1 fusion; or the FIG-ROS1 fusion or sometimes simply called the "ROS1 fusion protein and nucleic acids," including methods of detection, diagnosing, kits for detecting and diagnosing, methods of screening, methods of treating and preventing and various therapy for lung cancer patients, methods to measure the effectiveness of treatments and other pharmaceutical ingredients can be used in combination with the various treatments are described below.

The ROS1 protein is an orphan transmembrane receptor tyrosine kinase. Human ROS1 kinase protein (encoded by the ROS1 gene) is a 2347 amino acid long receptor tyrosine kinase that is prone to aberrant expression leading to cancer. A description of full length human ROS1 kinase (with the amino acid sequence of the human ROS1 protein) can be found at UniProt Accession No. P08922 (E.C.- 2.7.10.1). As shown in Table 1, the signal peptide, extracellular, transmembrane, and kinase domains of ROS1 are found at the following amino acid residues in SEQ ID NO: 1

Table 1. Amino acid residues and domains of ROS1 protein in SEQ ID NO: 1 Signal peptide 1-27 Extracellular domain 28-1859 Transmembrane domain 1860-1882 Kinase domain 1945-2222

**Protein**

| **Domain** | **Amino Acid residues in SEQ ID NO: 1** |
|---|---|
| Signal Peptide | 1-27 |
| Extracellular domain | 28-1859 |
| Transmembrane domain | 1860-1882 |
| Kinase domain | 1945-2222 |

The human ROS1 protein may be encoded by a human ROS1 gene located on human chromosome 6. The C-terminal domain of ROS1 protein may include an amino acid sequence encoded by a polynucleotide from 31^{st} or 32^{nd} or 34^{th} exon to the last exon (for example, 32^{nd} or 34^{th} exon) or a fragment thereof of ROS1 gene. The C-terminal domain of ROS1 protein may include consecutively at least about 100 amino acids from the start position of 31^{st} or 32^{nd} exon. For example, the C-terminal domain of ROS1 protein may include consecutively about 100 to about 700 amino acids, consecutively about 200 to about 600 amino acids, or consecutively about 250 to about 500 amino acids, or consecutively about 270 to about 425 amino acids, or consecutively about 270 to about 450 amino acids, or consecutively about 475 to about 625 amino acids from the start position of the 32^{nd} exon (long form) or the 34^{th} exon (short form) toward C-terminus of the ROS1 protein. The inferred breakpoint for the ROS1 protein is 1750, or 1852-1853 aa for SLC34A2-ROS1 fusion proteins and CD74-ROS1 fusion proteins and 1880-1881 aa for FIG-ROS1 fusion proteins.

In one exemplary embodiment, the human SLC34A2 gene encoding the human SLC34A2 protein (mRNA NM_001177998) is localized to human chromosome 4 (4p15.2) and contains 13 exons and an amino acid length of about 690 amino acids. The protein encoded by this gene is a pH-sensitive sodium-dependent phosphate transporter. Defects in this gene are a cause of pulmonary alveolar microlithiasis. Three transcript variants encoding two different isoforms have been found for this gene. The SLC34A2 protein may be derived from a mammal, such as a human. The N-terminal domain of a SLC34A2-ROS1 fusion protein may include an amino acid sequence encoded by a polynucleotide from the first exon to the 12th exon, or from the first exon to the fourth exon, or from the first exon to the second exon of SLC34A2 gene. These last observed exons in the structure of SLC34A2 gene have inferred breakpoints of 429 and 2076. The N-terminal domain of a SLC34A2-ROS1 fusion protein may include consecutively at least about 30-250 amino acids from 1^{st} position (that is, at least amino acid sequence from 1^{st} to 250^{th} positions, or fragments thereof) of the SLC34A2 protein. The N-terminal domain of SLC34A2-ROS1 fusion protein may include consecutively about 30 to 250 amino acids, consecutively about 30 to 225 amino acids, consecutively about 40 to 200 amino acids, or consecutively about 40 to 175 amino acids from the 1^{st} amino acid position of the SLC34A2 protein.

In one exemplary embodiment, the SLC34A2-ROS1 fusion is a chromosomal translocation between human chromosomes 6 and 4 that fuses the 3' region of ROS1 to the 5' region of SLC34A2. The fusion protein is detected and validated using various methods known to one skilled in the art and or as described herein. In some embodiments, a compound of Formula I, or a pharmaceutically acceptable salt thereof, is then administered to a patient in need thereof for preventing or treating a lung cancer. In some embodiments, a composition is administered to a patient in need thereof comprising at least one inhibitor against the SLC34A2-ROS1 fusion protein, at least one inhibitor against the SLC34A2-ROS1 fusion gene encoding the fusion protein, at least one inhibitor against a ROS1 coding gene, or a combination thereof, as an active ingredient.

In the SLC34A2-ROS1 fusion protein, the fusion, or fusion region may occur between various exons of the SLC34A2 gene and the ROS1 gene. Many fusions are known to one skilled in the art. Examples of such fusions include the 2^{nd}, and/or 4^{th} exon of SLC34A2 gene and 32^{nd} (Long, L), and/or 34^{th} exon (Short, S) of ROS1 gene, which is called as a fusion point or breakpoint. Other ROS1 and SLC34A2 fusion or breakpoints are known, and can be found in the COSMIC-Catalogue of somatic mutations in cancer database v.68 at cancer.sanger.ac.uk/cancergenome/projects/cosmic/. The term "a fusion region" may refer to a polynucleotide fragment (about 10-30 nucleotides) or polypeptide (about 5-30 amino acids) fragment around the fusion point.

The CD74-ROS1 fusion is a ROS1-involved a chromosomal translocation between human chromosomes 5 and 6 that fuses the 3' region of ROS1 to the 5' region of CD74. The fusion protein is detected and validated using various methods known to one skilled in the art and or as described herein. The N-terminal domain of a CD74-ROS1 fusion protein may include an amino acid sequence encoded by a polynucleotide from the first exon to the 12th exon, or from the first exon to the fourth exon, or from the first exon to the second exon of CD74 gene. The N-terminal domain of a CD74-ROS1 fusion protein may include consecutively at least about 30-250 amino acids from 1^{st} position (that is, at least amino acid sequence from 1^{st} to 250^{th} positions, or fragments thereof) of the CD74 protein. The N-terminal domain of CD74-ROS1 fusion protein may include consecutively about 30 to 250 amino acids, consecutively about 30 to 225 amino acids, consecutively about 40 to 200 amino acids, or consecutively about 40 to 210 amino acids or fragments thereof from the 1^{st} amino acid position of the CD74 protein.

In the fusion protein, the fusion, or fusion region may occur between various exons of the CD74 gene and the ROS1 gene. Many fusions are known to one skilled in the art. Examples of such CD74-ROS1 fusions include the 6th exon of CD74 gene fused to the 32nd, or 34th exon of ROS1 gene, which is called as a fusion point or breakpoint Other ROS1 and CD74 fusion or breakpoints are known, and can be found in the COSMIC-Catalogue of somatic mutations in cancer database v.68 at cancer.sanger.ac.uk/cancergenome/projects/cosmic/. The term "a fusion region" may refer to a polynucleotide fragment (about 10-30 nucleotides) or polypeptide (about 5-30 amino acids) fragment around the fusion point.

The fusion protein FIG-ROS1 results from an intra-chromosomal deletion on human chromosome 6 fusing the 5' region of FIG (aka GOPC) to the 3' region of ROS1. FIG-ROS1 fusions have been identified in samples from cholangiocarcinoma and ovarian cancer patients at a frequency of 8.7% and 0.5%, respectively. The FIG-ROS 1 fusion protein can be detected and validated using various methods known to one skilled in the art and or as described herein. The N-terminal domain of a FIG-ROS1 fusion protein may include consecutively at least about 150-500 amino acids from 1^{st} position (that is, at least amino acid sequence from 1^{st} to 500^{th} positions, or fragments thereof) of the FIG protein. The N-terminal domain of FIG-ROS1 fusion protein may include consecutively about 150 to about 500 amino acids, consecutively about 200 to 450 amino acids, consecutively about 220 to 425 amino acids, or consecutively about 220 to about 420 amino acids or fragments thereof from the 1^{st} amino acid position of the FIG protein.

In the FIG-ROS 1 fusion protein, the fusion, or fusion region may occur between various exons of the FIG gene and the ROS1 gene. Several FIG-ROS1 fusions are known to one skilled in the art. Examples of such fusions include the 4^{th} or 8^{th} exon of FIG gene fused to the 35th, or 36th exon of ROS1 gene, which is called as a fusion point or breakpoint. Other ROS1 and FIG fusions or breakpoints are known, and can be found in the COSMIC-Catalogue of somatic mutations in cancer database v.68 at cancer.sanger.ac.uk/cancergenome/projects/cosmic/. The term "a fusion region" may refer to a polynucleotide fragment (about 10-30 nucleotides) or polypeptide (about 5-30 amino acids) fragment around the fusion point.

Other ROS1 fusion partners can include TPM3, SDC4, EZR, and LRIG3, in addition to the SLC34A2, CD74, and FIG fusions described in detail herein. (See Fig. 1 herein, and for example, Takeuchi K, Soda M, Togashi Y, Suzuki R, Sakata S, Hatano S, et al. RET, ROS1 and ALK fusions in lung cancer. Nat Med. 2012, and Kurtis D. Davies, Anh T. Le, Mariana F. Theodoro, Margaret C. Skokan, Dara L. Aisner, Eamon M. Berge, Luigi M. Terracciano, Matteo Incarbone, Massimo Roncalli, Federico Cappuzzo, D. Ross Camidge, Marileila Varella-Garcia, and Robert C. Doebelet, Identifying and Targeting ROS1 Gene Fusions in Non-Small Cell Lung Cancer, (2012), Clin Cancer Res. 2012 September 1; 18(17): 4570-4579, the contents of these disclosures relating to the ROS1 fusion proteins is incorporated herein in their entireties.

In one embodiment, the ROS1 fusion protein of the present invention comprises the C-terminal domain of human ROS1 protein (for example, human ROS1 as provided in SEQ ID NO. 1) providing a functional kinase domain, which consists essentially of consecutive about 50 to 300 amino acids starting from an amino acid corresponding to the start position of 32^{nd} exon of the ROS1 protein and then toward C-terminus of the ROS1 protein.

As used herein, the exon number is numbered according to the exon number allocated by the National Center for Biotechnology Information (NCBI) hosted by the National Institutes of Health, Bethesda, Maryland USA. In some exemplary embodiments, the fusion protein SLC34A2-ROS1 may have any of the amino acid sequence identified as such by the NCBI. The nucleotide sequences of DNA molecules and the amino acid sequences of proteins encoded by the DNA molecules may be determined by an automated DNA sequencer or an automated peptide sequencer. The (nucleotide or amino acid) sequences determined by such automated sequencing means may include partial error compared with actual sequences. Generally the sequences determined by automated sequencing may have sequence identity of at least about 90%, at least 20 about 95%, at least about 99%, or at least about 99.9% compared with actual sequences. Therefore, the fusion protein, the fusion gene or the fusion region may have an amino acid sequence or a nucleotide sequence having sequence identity of at least about 90%, at least about 95%, at least about 99%, or at least about 99.9% compared with the sequences of identified as such by the NCBI.

For the purpose of simplicity, the following disclosure refers to the ROS1 fusion protein SLC34A2-ROS1, but may also apply to other ROS1 fusion proteins described herein. The exemplified SLC34A2-ROS1 fusion protein, in some embodiments may consist of 80-200 N-terminal amino acid residues of SLC34A2 and at least 300 C-terminal residues of ROS1, preferably ranging from 300-500 C-terminal amino acid residues. The fusion gene has a protein tyrosine kinase domain together with helical domains. Without wishing to be bound by any theory, it is believed that the tertiary conformation of the SLC34A2-ROS1 fusion protein induces homo-dimerization which will activate the oncogenic protein tyrosine kinase domain by auto-phosphorylation. Taken together, the SLC34A2-ROS1 fusion gene may be highly expressed and then dimerized after translation owing to SLC34A2. Then, the dimerized ROS1 protein tyrosine kinase domain may be stimulated abnormally, thus facilitating the stimulation of an oncogenic pathway, for example, as seen in a ROS1 fusion related NSCLC lung cancer.

Once a fusion is detected it will be known as a fusion gene of SLC34A2-ROS1 encoding the fusion protein of SLC34A2-ROS1. A method of providing information for diagnosing a lung cancer, comprising the step of detecting, in a test sample obtained from a subject, a fusion as described herein. Diagnosis would compare a fusion gene encoding the fusion protein; and an overexpression of ROS 1 compared to a standard sample from an individual without a cancer, wherein when at least one element is selected and detected in the test sample, allowing the subject to be identified as any or all of the following: a cancer patient, a lung cancer patient, NSCLC lung cancer patient, a ROS1 fusion related NSCLC patient, and/or a SLC34A2-ROS1 fusion related NSCLC patient.

The deletion, inversion or translocation of chromosome 6 may be detected by using a polynucleotide (a probe) capable of hybridizing with (complementarily binding to) the deletion, inversion or translocation region in human chromosome 6 and/or a primer pair capable of detecting the deletion, inversion or translocation of human chromosome 6, for example, capable of producing a polynucleotide fragment having consecutive 100 to 200 nucleotides including the inversion region in human chromosome 6. The fusion protein, the fusion gene, and the fusion region are described herein. In an exemplary embodiment, the fusion protein may also be detected by detecting the presence of the fusion protein or the fusion gene or mRNA corresponding to the fusion gene, for example, with the use of ROS 1 specific or ROS1 fusion specific antibodies known in the art and described herein.

The presence of the fusion protein may be detected be a general assay that measures the interaction between the fusion protein and a material (e.g., an antibody or an aptamer) specifically binding to the fusion protein. The general assay may be immunochromatography, immunohistochemical staining, enzyme linked immunosorbent assay (ELISA), radioimmunoassay (RIA), enzyme immunoassay (EIA), florescence immunoassay (FIA), luminescence immunoassay (LIA), western blotting, FACS, and the like.

In addition, the presence of the fusion gene or the mRNA may be detected by a general assay such as PCR, FISH (fluorescent in situ hybridization), and the like, using a polynucleotide capable of hybridizing with (complementarily binding to) the fusion gene or the mRNA. FISH is described in more detail below. The fusion gene may be detected and/or validated by using the integration techniques of whole-transcriptome (RNA) and/or whole-genome DNA sequencing through massively parallel sequencing technologies. The polynucleotide capable of hybridizing with the fusion gene or the mRNA may be a siRNA, an oligonucleotide, DNA probe, or DNA primer, which can detect the fusion gene or the mRNA by a direct hybridization with the fused or truncated gene or transcript in the test sample.

In further embodiments, fluorescence in-situ hybridization (FISH) is employed in the methods of the invention (as described in Verma et al. Human Chromosomes: A Manual Of Basic Techniques, Pergamon Press, New York, N.Y. (1988)). A FISH assay can be performed using one or more probe sets, embodiments are provided such as: (1) A first probe set, which is a first probe set targeting a chromosomal site which contains the ROS1 gene (first chromosomal site); it consists of a probe 1A labeled with a first fluorescent substance and a probe 1B labeled with a second fluorescent substance; probe 1A is complementary to the first region, which is the 5' region in the aforementioned first chromosomal site, probe 1B is complementary to the second region, which is present at a distance from the aforementioned first region and is the 3' region in the aforementioned first chromosomal site, and the breakpoint in the ROS1 gene when the SLC34A2-ROS1 fusion gene is produced by a translocation between the SLC34A2 and ROS1 genes is located in the 3' tail of the aforementioned first region, between the aforementioned first and second regions, or in the 5' tail of the aforementioned second region; (2) A second probe set, which is a second probe set targeting a chromosomal site which contains the SLC34A2 gene (second chromosomal site); it consists of a probe 2A labeled with a first fluorescent substance and a probe 2B labeled with a second fluorescent substance; probe 2A is complementary to the first region, which is the 5' region in the aforementioned second chromosomal site, probe 2B is complementary to the second region, which is present at a distance from the aforementioned first region and is the 3' region in the aforementioned second chromosomal site, and the breakpoint in the SLC34A2 gene when the SLC34A2-ROS1 fusion gene is produced by a translocation between the SLC34A2 and ROS1 genes is located in the 3' tail of the aforementioned first region, between the aforementioned first and second regions, or in the 5' tail of the aforementioned second region; (3) a third probe set consisting of the aforementioned probe 2A and the aforementioned probe 1B; and (4) a fourth probe set consisting of a probe 4A which is complementary to the chromosomal site containing the ROS1 gene (the third chromosomal site), and a probe 4B which is complementary to the chromosomal site containing the SLC34A2 gene (the fourth chromosomal site).

The length of the aforementioned first chromosomal site can be 0.5-2.0 Mb. The length of the aforementioned second chromosomal site can be 0.5-2.0 Mb. The length of the aforementioned third chromosomal site can be 0.5-2.0 Mb. The length of the aforementioned fourth chromosomal site can be 0.5-2.0 Mb.

In some embodiments, the FISH assay may be correlated with other physical chromosome mapping techniques and genetic map data. The FISH technique is well known (see, e.g., U.S. Pat Nos. 5,756,696; 5,447,841; 5,776,688; and 5,663,319). Examples of genetic map data can be found in the 1994 Genome Issue of Science (265: 1981f). Correlation between the location of the gene encoding ROS1 protein and/or, in the case of ROS1 fusion polypeptides, the gene encoding the fusion partner of a ROS1 fusion protein (e.g., the FIG gene, the SLC34A2 gene, or the CD74 gene) on a physical chromosomal map and a specific disease, or predisposition to a specific disease, may help delimit the region of DNA associated with that genetic disease. The nucleotide sequences of the subject invention may be used to detect differences in gene sequences between normal, carrier, or affected individuals.

In situ hybridization of chromosomal preparations and physical mapping techniques such as linkage analysis using established chromosomal markers may be used for extending genetic maps. Often the placement of a gene on the chromosome of another mammalian species, such as mouse, may reveal associated markers even if the number or arm of a particular human chromosome is not known. New sequences can be assigned to chromosomal arms, or parts thereof, by physical mapping. This provides valuable information to investigators searching for disease genes using positional cloning or other gene discovery techniques. Once the disease or syndrome has been crudely localized by genetic linkage to a particular genomic region, for example, AT to 11q22-23 (Gatti et al., Nature 336: 577-580 (1988)), any sequences mapping to that area may represent associated or regulatory genes for further investigation. The nucleotide sequences encoding the ROS1 fusions of the subject invention, or portions thereof, may also be used to detect differences in the chromosomal location due to translocation, inversion, etc., among normal, carrier, or affected individuals.

It shall be understood that all of the methods (e.g., PCR and FISH) that detect polynucleotides encoding a polypeptide with ROS1 kinase activity (e.g., full-length ROS1 or an ROS1 fusion polynucleotides such as SLC34A2-ROS1, or CD74-ROS1, or FIG-ROS1(S)), may be combined with other methods that detect polypeptides with ROS1 kinase activity or polynucleotides encoding a polypeptide with ROS1 kinase activity. For example, detection of a FIG-ROS1 fusion polynucleotide in the genetic material of a biological sample (e.g., FIG-ROS1 in a circulating tumor cell) may be followed by Western blotting analysis or immuno-histochemistry (IHC) analysis of the proteins of the sample to determine if the FIG-ROS1 polynucleotide was actually expressed as a FIG-ROS1 fusion polypeptide in the biological sample. Such Western blotting or IHC analyses may be performed using an antibody that specifically binds to the polypeptide encoded by the detected FIG-ROS1 polynucleotide, or the analyses may be performed using antibodies that specifically bind either to full length FIG (e.g., bind to the N-terminus of the protein) or to full length ROS1 (e.g., bind an epitope in the kinase domain of ROS1). Such assays are known in the art (see, e.g., U.S. Pat. No. 7,468,252).

In another example, the CISH technology of Dako allows chromatogenic in situ hybridization with immuno-histochemistry on the same tissue section. See Elliot et al., Br J Biomed Sci 2008; 65(4): 167- 171, 2008 for a comparison of CISH and FISH.

Another aspect of the invention provides a method for diagnosing a patient as having a lung cancer or a suspected lung cancer driven by an ROS1 kinase. The method includes contacting a biological sample of said lung cancer or a suspected lung cancer (where the biological sample comprising at least one nucleic acid molecule) with a probe that hybridizes under stringent conditions to a nucleic acid molecule encoding a polypeptide with ROS1 kinase activity such as a full length ROS1 polynucleotide or a ROS1 fusion polynucleotide (e.g., a FIG-ROS1 fusion polynucleotide, an SLC34A2-ROS1 fusion polynucleotide, or a CD74-ROS1 fusion polynucleotide), and wherein hybridization of said probe to at least one nucleic acid molecule in said biological sample identifies said patient as having a lung cancer or a suspected lung cancer driven by a ROS1 kinase.

Yet another aspect of the invention provides a method for diagnosing a patient as having a lung cancer or suspected of having lung cancer driven by a ROS1 kinase. The method includes contacting a biological sample of said lung cancer or suspected lung cancer (where said biological sample comprises at least one polypeptide) with a reagent that specifically binds to a polypeptide with ROS1 kinase activity (e.g., a FIG-ROS1 fusion polypeptide, an SLC34A2-ROS1 or a CD74-ROS1 fusion polypeptide), wherein specific binding of said reagent to at least one fusion polypeptide in said biological sample identifies said patient as having a lung cancer or a suspected lung cancer driven by a ROS 1 kinase.

In various embodiments, the identification of a lung cancer or suspected lung cancer as being driven by a ROS1 kinase will identify that patient having that lung cancer or suspected lung cancer as being likely to respond to a ROS1-inhibiting therapeutic.

A kit for detecting translocations between SLC34A2 and ROS1 genes can include one or more probe sets. (1) A first probe set includes a probe 1A labeled with a first fluorescent substance and a probe 1B labeled with a second fluorescent substance; Probe 1A is complementary to the first region, which is the 5' region in the aforementioned first chromosomal site, probe 1B is complementary to the second region, which is present at a distance from the aforementioned first region and is the 3' region in the aforementioned first chromosomal site, and the breakpoint in the ROS1 gene when the SLC34A2-ROS 1 fusion gene is produced by a translocation between the SLC32A2 and ROS1 genes is located in the 3' tail of the aforementioned first region, between the aforementioned first and second regions, or in the 5' tale of the aforementioned second region; (2) A second probe set, which is a second probe set targeting a chromosomal site which contains the SLC32A2 gene (second chromosomal site); it consists of a probe 2A labeled with a first fluorescent substance and a probe 2B labeled with a second fluorescent substance; probe 2A is complementary to the first region, which is the 5' region in the aforementioned second chromosomal site, probe 2B is complementary to the second region, which is present at a distance from the aforementioned first region and is the 3' region in the aforementioned second chromosomal site, and the breakpoint in the SLC32A2 gene when the SLC34A2-ROS1 fusion gene is produced by a translocation between the SLC32A2 and ROS1 genes is located in the 3' tail of the aforementioned first region, between the aforementioned first and second regions, or in the 5' tale of the aforementioned second region; (3) A third probe set consisting of the aforementioned probe 2A and the aforementioned probe 1B; and a fourth probe set consisting of a probe 4A which is complementary to the chromosomal site containing the ROS1 gene (the third chromosomal site) and a probe 4B which is complementary to the chromosomal site containing the SLC32A2 gene (the fourth chromosomal site).

A kit useful for identifying patients susceptible to SLC34A2-ROS 1 translocation includes one or more elements selected from a group comprising an explanation of the use of the probes, a DNA contrast stain, a buffer for hybridization use, an encapsulant, and a control slide. The kit makes it possible to implement conveniently and efficiently the detection method of this invention. The kit can include as required elements (essential ingredients) the aforementioned first probe set, second probe set, third probe set or fourth probe set. Two or more types of probe sets can also be included in the kit For example, the kit can incorporate first probe set and third probe set. Since the details for each probe set have been described above, they will not be repeated here.

Detection of the presence or absence of a SLC34A2-ROS1 fusion polynucleotide can be performed directly using genome DNA that encodes the aforementioned fusion polypeptide or a transcript from that genome DNA, but it may also be performed indirectly using a translation product from that transcript (the aforementioned fusion polypeptide).

Because the genome DNA that encodes the fusion polypeptide is formed by inversion between the 117.61-117.75Mb region of chromosome 6, (6q22) the phenomenon of this invention may be detected in the "detection of the presence or absence of a SLC34A2-ROS1 fusion polynucleotide." In this detection of inversion, for example, a split between a 5'-side region upstream from the kinase domain coding region of the ROS1 gene and a 3'-side region downstream from that coding region of the ROS1 gene may be detected, or a split between the coding region of part or all of the helical domains and 5'-side region upstream from that coding region of the SLC32A2 gene and a 3'-side region downstream from the coding region of the helical domains of the SLC34A2 gene may be detected.

Techniques known and available to the skilled practitioner may be used in the "detection of the presence or absence of a SLC34A2-ROS1 fusion polynucleotide" in the present invention. If "genome DNA that encodes the aforementioned fusion polypeptide" is the object, for example, *in situ* hybridization (ISH) using fluorescence or the like, genome PCR, direct sequencing, southern blotting, or genome microarray analysis may be used. If a "transcript from the aforementioned genome DNA" is the object, for example, RT-PCR, direct sequencing, northern blotting, dot blotting, or cDNA microarray analysis may be used.

The kit of this invention can also include other elements. Examples of these other elements are the specification for use of the probes, a DNA counterstain such as DAPI, a hybridization buffer, a wash buffer, solvents, mounting media, control slides, reaction vessels, and other equipment. Specifications for diagnostic purposes can also be included. Furthermore, specifications, etc. can also be included that show how the detection (positive identification) of SLC34A2-ROS1 translocation in chromosome samples from cancer patients should be set up in these patients using an ROS1 kinase inhibitor. Moreover, a plan for determining the treatment course and an explanation of this plan can also be included.

Comparatively long probes (approximately 200 kb (probe 1A) to approximately 1,370 kb (probe 4B) are contemplated. Therefore, complementation between the probes and the target sequences does not need to be highly restrictive, to the extent that specific hybridization intended in this invention is achieved. An example of similarity between target sequences is at least 90%, preferably at least 95%, and more preferably at least 98%.

In the case where a fust probe set and second probe set are used, the two fluorescence signals are separated and detected individually in chromosomal samples in which the translocation between the SLC34A2 gene and the ROS1 gene occurred; in chromosomal samples in which the translocation did not occur, typically the two fluorescence signals are observed to be next to each other, or a signal (yellow) that is a combination of the two fluorescence signals is observed. Thus, the presence or absence of a translocation between the SLC34A2 gene and the ROS1 gene is reflected in the pattern of the fluorescence signal. Consequently, a translocation between the SLC34A2 gene and the ROS1 gene can be determined from the pattern of the fluorescence signal.

The judgment reached above is preferably and typically made based on a comparison of a result with a control (test sample). Here, the control is a chromosomal sample derived from a patient with non-small cell lung cancer or a chromosomal sample derived from a patient exhibiting precancerous lesions. In addition, chromosomal samples from a patient without precancerous lesions, chromosomal samples from patients who do not have cancer, or chromosomal samples taken from normal, healthy subjects can also be used as the control. Chromosomal samples derived from strains of cells can also be used as a control.

When the fusion gene is a fusion gene SLC34A2-ROS1 encoding the fusion protein of SLC34A2-ROS1, the fusion gene SLC34A2-ROS1 (or any other ROS1 fusion gene, e.g. CD74-ROS1, & FIG-ROS1) may be detected by using a polynucleotide (a probe) capable of hybridizing with (complementarity binding to) the fusion region and/or a primer pair capable of producing a polynucleotide fragment having consecutive 100 to 200 nucleotides including the fusion region. In addition, in one exemplary embodiment, the fusion protein SLC34A2-ROS1may be detected using an antibody or aptamer specifically binding to the fusion region of the fusion protein SLC34A2-ROS1.

In addition, the detection can be performed by a fusion assay which is a combination of chromogenic in situ hybridization (CISH) method and silver in situ hybridization (SISH) method. The "fusion point" in the present specification refers to a point where a portion derived from the respective genes of SLC34A2 is fused with a portion derived from the ROS1 gene.

The term "capable of hybridizing with the fusion region (or the inversion region)" may refer to having a complementary sequence or a sequence having sequence identity of at least 90% with that of the fusion region (or the inversion region). Another embodiment provides a composition for diagnosing a cancer, including one or more selected from the group consisting of a polynucleotide capable of hybridizing with the fusion region, a primer pair capable of producing a polynucleotide fragment having consecutive 100 to 200 nucleotides including the fusion region. Also a polynucleotide capable of hybridizing with the inversion region in human chromosome 6, a primer pair capable of producing a polynucleotide fragment having consecutive 100 to 200 nucleotides including the inversion region of human chromosome 6, and an antibody or aptamer binding to the fusion region. Another embodiment provides a use of the fusion protein and/or the fusion gene for diagnosing a cancer. The patient may be any mammal, for example, a primate such as a human or monkey, a rodent such as a mouse or a rat, in particular a human. The test sample, suitable for use in any assays mentioned, may be a cell (e.g., a lung cell); a tissue (e.g., a lung tissue); body fluid (e.g., blood); circulating tumor DNA, circulating tumor cells. The samples may be collected in any manner known to one skilled in the art, including collection from the surgical biopsy of tumor, a core biopsy of tumor, a fine needle aspirate of tumor, pleural effusion, and other known methods of separating cells and tissues from patients. For example, a FISH assay (described herein) could be performed on circulating tumor cells.

The patient may be receiving treatments or have plans to be treated with a kinase inhibitor. The test sample may include a cell derived from a human cancer cell or an extract thereof.

Another embodiment provides a fusion gene encoding the fusion protein, where a gene encoding the N-terminal domain of the fusion partner positions at 5' end and a gene encoding the C-terminal domain of the ROS1 protein positions at 3' end. In one exemplary embodiment, when the fusion protein is the SLC34A2-ROS1 protein, the fusion gene may be represented as SLC34A2-ROS1 gene, where a gene encoding the N-terminal domain of SLC34A2 positions at 5' end and a gene encoding the C-terminal domain of the ROS1 protein positions at 3' end.

Another embodiment provides an expression vector including the fusion gene and optionally transcription elements (e.g., a promoter and the like) operably linked to the fusion gene. Another embodiment provides a transformant cell transformed with the expression vector.

Biological specimens obtained in the course of treatment or diagnosis (biopsy samples, etc.) are often formalin fixed, but in this case, the use of *in situ* hybridization is advantageous because the DNA genome which is the detection object is stable under formalin fixing and because detection sensitivity is high.

In *in situ* hybridization, the genomic DNA that encodes a SLC34A2-ROS1 fusion polypeptide in the biological specimen can be detected by hybridizing the polynucleotide of (a) or (b) stated below having a chain length of at least 15 nucleotide bases:
(a) a polynucleotide that is at least one probe selected from the group made up of probes that hybridize to a polynucleotide that encodes the SLC34A2 protein and probes that hybridize to a polynucleotide that encodes the ROS1 protein
(b) a polynucleotide that is a probe that hybridizes to a fusion site of a polynucleotide that encodes the SLC34A2 protein and a polynucleotide that encodes the ROS1 protein.

In *in situ* hybridization, the genome DNA that encodes a CD74-ROS1 fusion polypeptide in the biological specimen can be detected by hybridizing the polynucleotide of (a) or (b) stated below having a chain length of at least 15 bases:
(a) a polynucleotide that is at least one probe selected from the group made up of probes that hybridize to a polynucleotide that encodes the CD74 protein and probes that hybridize to a polynucleotide that encodes the ROS1 protein
(b) a polynucleotide that is a probe that hybridizes to a fusion site of a polynucleotide that encodes the CD74 protein and a polynucleotide that encodes the ROS1 protein.

In in situ hybridization, the genome DNA that encodes a FIG-ROS1 fusion polypeptide in the biological specimen can be detected by hybridizing the polynucleotide of (a) or (b) stated below having a chain length of at least 15 bases:
(a) a polynucleotide that is at least one probe selected from the group made up of probes that hybridize to a polynucleotide that encodes the FIG protein and probes that hybridize to a polynucleotide that encodes the ROS1 protein
(b) a polynucleotide that is a probe that hybridizes to a fusion site of a polynucleotide that encodes the FIG protein and a polynucleotide that encodes the ROS1 protein.

However, the DNA sequence of a gene may mutate in the natural world (that is, non-artificially). Accordingly, such natural variants may also be the object of the present invention (similarly hereinafter).

The polynucleotide stated in (a) of the present invention may be any, provided that it can detect the presence of genome DNA that encodes the aforementioned ROS1 fusion protein in the biological specimen by hybridizing to a polynucleotide that encodes the ROS1 binding partner, i.e. SLC34A2, CD74 or FIG protein or a polynucleotide that encodes the ROS1 protein, which are the target base sequences of that polynucleotide. It is preferably a polynucleotide stated in (a1) through (a4) below.
(a1) A combination of a polynucleotide that hybridizes to the coding region of part or all of the helical domain of exons 1-2 and 5'-side region upstream from that coding region of the SLC34A2 gene (also referred to as "5' SLC34A2 probe 1" hereinafter) and a polynucleotide that hybridizes to the coding region of the kinase domain and 3'-side region downstream from that coding region of the ROS1 gene (also referred to as "3' ROS1 probe 1" hereinafter.)
(a2) A combination of a polynucleotide that hybridizes to the 5'-side region upstream from the coding region of the kinase domain of the ROS1 gene (also referred to as "5' ROS1 probe 1" hereinafter) and a polynucleotide that hybridizes to coding region of the kinase domain and 3'-side region downstream from that coding region of the ROS1 gene (also referred to as "3' ROS1 probe 1" hereinafter.)
(a3) A combination of a polynucleotide that hybridizes to the coding region of the Fibronectin type-III 9 and 5'-side region upstream from that region of the ROS1 gene (also referred to as "5' ROS1 probe 2" hereinafter) and a polynucleotide that hybridizes to the coding region of the transmembrane domain and 3'-side region downstream from that coding region of the ROS1 gene (also referred to as "3' ROS1 probe 2" hereinafter.)
(a4) A combination of a polynucleotide that hybridizes to the coding region of part or all of the helical domain and 5'-side region upstream from that coding region of the SLC34A2 gene (also referred to as "5' SLC34A2 probe 1" hereinafter) and a polynucleotide that hybridizes to the coding region of the coiled coil domain and 3'-side region downstream from that coding region of the SLC34A2 gene (also referred to as "3' SLC34A2 probe 1" hereinafter.)

The region (target base sequence) to which the polynucleotide of (a1) used in *in situ* hybridization hybridizes is preferably a region within 1,000,000 bases from the fusion site of the SLC34A2 gene and ROS1 gene, for reasons of specificity to the target base sequence and detection sensitivity, and the region to which the polynucleotides of (a2) through (a4) used in *in situ* hybridization hybridize is preferably a region within 1,000,000 bases from the breakpoint in the SLC34A2 gene or ROS1 gene, for the same reasons.

The polynucleotide stated in (a) or (b) above used in *in situ* hybridization is preferably a collection made up of a plurality of types of polypeptide that can cover all of the aforementioned target base sequences, for reasons of specificity to the target base sequence and detection sensitivity. In this case, the length of the polynucleotides that constitute the collection is at least 15 bases, and preferably 100 to 1000 bases.

The polynucleotide stated in (a) or (b) above used in *in situ* hybridization is preferably labeled by fluorescent dye or the like for detection. Examples of such a fluorescent dye include DEAC, FITC, R6G, TexRed and Cy5, but are not limited to these. Other than a fluorescent dye, the aforementioned polynucleotide may also be labeled with a dye (chromogen) such as DAB, or silver and the like based on enzymatic metal deposition.

In *in situ* hybridization, if 5' SLC34A2 probe 1 and 3'ROS1 probe 1 are used, or if 5' ROS1 probe 1 and 3'ROS1 probe 1 are used, or if 5' ROS1 probe 2 and 3' ROS1 probe 2 are used, or if 5' SLC34A2 probe 1 and 3' SLC34A2 probe 1 are used, it is preferred that these probes are labeled with mutually different dyes. When *in situ* hybridization is performed using a combination of probes labeled with different dyes in this manner, when the signal produced by the label of 5' SLC34A2 probe 1 (for example, fluorescence) and the signal produced by the label of 3' ROS1 probe 1 overlap, it can be determined that genome DNA that encodes a SLC34A2-ROS1 fusion polypeptide has been detected. On the other hand, when the signal produced by the label of 5' ROS1 probe 1 and the signal produced by the label of 3' ROS1 probe 1 are split, or the signal produced by the label of 5' ROS 1 probe 2 and the signal produced by the label of 3' ROS1 probe 2 are split, or the signal produced by the label of 5' SLC34A2 probe 1 and the signal produced by the label of 3' SLC34A2 probe 1 are split, it can be determined that genome DNA that encodes a SLC34A2-ROS1 fusion polypeptide has been detected.

Polynucleotide labeling may be performed by a known technique. For example, a substrate base labeled with fluorescent dye or the like by nick translation or random priming may be integrated in a polynucleotide, thereby labeling that polynucleotide. In *in situ* hybridization, the conditions used when hybridizing the polynucleotide stated in (a) or (b) above and the aforementioned biological specimen may be varied depending on various factors such as the length of the relevant polynucleotide, but an example of high-stringency hybridization conditions is 0.2xSSC, 65°C, and an example of low-stringency hybridization conditions is 2.0xSSC, 50°C. Note that hybridization conditions of the same stringency as the aforementioned conditions can be achieved by a person skilled in the art appropriately selecting the various conditions such as salt concentration (dilution ratio of SSC) and temperature, as well as concentration of surfactant (NP-40, etc.), concentration of formamide, and pH. For example, for general hybridization conditions for screening, polynucleotide compositions are provided that are capable of hybridizing under moderate to high stringency conditions to a polynucleotide sequence provided herein, or a fragment thereof, or a complementary sequence thereof. Hybridization techniques are well known in the art of molecular biology. For purposes of illustration, suitable moderately stringent conditions for testing the hybridization of a polynucleotide of this invention with other polynucleotides include prewashing in a solution of 5.times.SSC, 0.5% SDS, 1.0 mM EDTA (pH 8.0); hybridizing at 50°C-60°C. 5X SSC, overnight; followed by washing twice at 65°C for 20 minutes with each of 2X, 0.5X and 0.2X SSC containing 0.1% SDS. One skilled in the art will understand that the stringency of hybridization can be readily manipulated, such as by altering the salt content of the hybridization solution and/or the temperature at which the hybridization is performed. For example, in another embodiment, suitable highly stringent hybridization conditions include those described above, with the exception that the temperature of hybridization is increased, e.g., to 60-65°C or 65-70°C.

Examples of methods for detecting genome DNA that encodes a SLC34A2-ROS1 fusion polypeptide using the polynucleotide stated in (a) or (b) above other than the aforementioned *in situ* hybridization are southern blotting, northern blotting and dot blotting. In these methods, the aforementioned fusion gene is detected by hybridizing under moderate to high stringency conditions the polynucleotide stated in (a) or (b) above to a membrane on which a nucleic acid extract obtained from the aforementioned biological specimen has been transcribed. When using the polynucleotide of (a), it can be determined that genome DNA that encodes a SLC34A2-ROS1 fusion polypeptide has been detected when a polypeptide that hybridizes to a polynucleotide that encodes the SLC34A2 protein and a polypeptide that hybridizes to a polynucleotide that encodes the ROS1 protein recognize the same band developed on the membrane.

Genome microarray analysis and DNA microarray analysis are additional methods for detecting genome DNA that encodes a SLC34A2-ROS1 fusion polypeptide using the polynucleotide of (b) above. In these methods, an array of polynucleotides of (b) is fixed on a substrate, and the relevant genome DNA is detected by putting the biological specimen in contact with the polynucleotides on the array. In PCR or sequencing, the polynucleotide of (c) stated below may be used to specifically amplify part or all of a SLC34A2-ROS1 fusion polynucleotide, using DNA (genome DNA, cDNA) or RNA prepared from the biological specimen as a template. (c) A polynucleotide that is a pair of primers designed to sandwich a fusion site of a polynucleotide that encodes the SLC34A2 protein and a polynucleotide that encodes the ROS1 protein. The "polynucleotide that is a pair of primers" is a primer set of which one primer hybridizes to a polynucleotide that encodes the SLC34A2 protein and the other primer hybridizes to a polynucleotide that encodes the ROS1 protein in a base sequence such as the aforementioned fusion polynucleotide that serves as a target. The length of these polynucleotides is normally 15 to 100 bases, and preferably 17 to 30 bases.

From the viewpoints of precision and sensitivity of detection by PCR, the polynucleotide stated in (c) of the present invention is preferably a complementary sequence to the base sequence of the aforementioned fusion polynucleotide within 5000 bases from the fusion site of the polynucleotide that encodes the SLC34A2 protein and the polynucleotide that encodes the ROS1 protein.

The "polynucleotide that is a pair of primers" can be appropriately designed by known techniques based on the base sequence of the SLC34A2-ROS1 fusion polynucleotide that serves as a target. Advantageous examples of the "polynucleotide that is a pair of primers" are primer sets made up of one primer selected from the group made up of SLC34A2-ROS1-F1, SLC34A2-int15-F1, SLC34A2-int15-F2, SLC34A2-ex16-F1, SLC34A2-ex23-Fl, SLC34A2-ex24-Fl, SLC34A2-F-orf2438 and SLC34A2-int15-F3.5, and one primer selected from the group made up of SLC34A2-ROS1-R1, ROS1-int11-R3, ROS1-int7-R1, ROS1-int11R0.5, ROS1-int11-R1, ROS1-int7-R2 and ROS1-R-orf2364. More preferably, it is SLC34A2-ROS1-F1 and SLC34A2-ROS1-R1, SLC34A2-int15-F1 and SLC34A2-ROS1-R1, SLC34A2-intl5-F2 and ROS1-int11-R3, SLC34A2-ex16-F1 and SLC34A2-ROS1-R1, SLC34A2-ex23-Fl and SLC34A2-ROS1-R1, or SLC34A2-ex24-F1 primer and ROSl-int7-Rl primer.

In some embodiments, the invention provides, methods of: identifying, assessing or detecting a SLC34A2-ROS1 fusion; methods of identifying, assessing, evaluating, and/or treating a subject having a cancer, e.g., a cancer having a SLC34A2-ROS1, a CD74-ROS1, or a FIG-ROS1 fusion; isolated SLC34A2-ROS1, CD74-ROS1, or a FIG-ROS1 nucleic acid molecules, nucleic acid constructs, host cells containing the nucleic acid molecules; purified SLC34A2-ROS1, CD74-ROS1, or FIG-ROS1 polypeptides and binding agents; detection reagents (e.g., probes, primers, antibodies, kits, capable, e.g., of specific detection of SLC34A2-ROS1, CD74-ROS1, or FIG-ROS1 nucleic acids or proteins); screening assays for identifying molecules that interact with, e.g., inhibit, 5'SLC34A2-3'ROS1, 5'CD74-3'ROS1, or a 5'FIG-3'ROS1 fusions, e.g., novel kinase inhibitors; as well as assays and kits for evaluating, identifying, assessing and/or treating a subject having a cancer, e.g., a cancer having one or more of a SLC34A2-ROS1, CD74-ROS1, or FIG-ROS1 fusion. The compositions and methods identified herein can be used, for example, to identify new SLC34A2-ROS1, CD74-ROS1, or FIG-ROS1 inhibitors; to evaluate, identify or select subject, e.g., a patient, having a cancer; and to treat or prevent a cancer.

SLC34A2-ROS1, CD74-ROS1, and FIG-ROS1 Nucleic Acid Molecules. In one aspect, the invention features a nucleic acid molecule (e.g., an isolated or purified) nucleic acid molecule that includes a fragment of a SLC34A2, or a CD74, or a FIG gene and a fragment of a ROS1 proto-oncogene. In one embodiment, the nucleic acid molecule includes a fusion, e.g., an in-frame fusion, of at least one exon of SLC34A2, CD74, or FIG and an exon of ROS1 (e.g., one or more exons encoding a ROS1 tyrosine kinase domain or a fragment thereof).

In an illustrative embodiment, the 5'SLC34A2-3'ROS1 nucleic acid molecule comprises sufficient SLC34A2 and sufficient ROS1 sequence such that the encoded 5'SLC34A2-3'ROS1 fusion has constitutive kinase activity, e.g., has elevated activity, e.g., kinase activity, as compared with wild type ROS1, e.g., in a cell of a cancer referred to herein. In an embodiment the encoded 5'SLC34A2-3'ROS1 fusion comprises at least 1, 2, 3, 4, 5, 6, 7, 9, 10, or 11 exons from SLC34A2 and at least 1, 2, 3, 4, 5, 6, 7, 9,10, 11, 12, or 13 ROS1 exons. In one embodiment, the encoded 5'SLC34A2-3'ROS1 fusion polypeptide includes a helical domain, or a functional fragment thereof, and a ROS1 tyrosine kinase domain or a functional fragment thereof.

In an illustrative embodiment, the 5'CD74-3'ROS1 nucleic acid molecule comprises sufficient CD74 and sufficient ROS1 sequence such that the encoded 5'CD74-3'ROS1 fusion has constitutive kinase activity, e.g., has elevated activity, e.g., kinase activity, as compared with wild type ROS1, e.g., in a cell of a cancer referred to herein. In an embodiment, the encoded 5'CD74-3'ROS1 fusion comprises at least 1, 2, 3, 4, 5, 6, exons from CD74 and at least 1, 2, 3, 4, 5, 6, 7, 9,10, 11, 12, or 13 ROS1 exons. In one embodiment, the encoded 5'CD74-3'ROS1 fusion polypeptide includes a helical; signal-anchor for type II membrane protein domain, or a functional fragment thereof, and a ROS1 tyrosine kinase domain or a functional fragment thereof.

In an illustrative embodiment, the 5'FIG-3'ROS 1 nucleic acid molecule comprises sufficient FIG and sufficient ROS1 sequence such that the encoded 5'FIG-3'ROS1 fusion has constitutive kinase activity, e.g., has elevated activity, e.g., kinase activity, as compared with wild type ROS1, e.g., in a cell of a cancer referred to herein. In an embodiment the encoded 5'FIG-3'ROS1 fusion comprises at least 1, 2, 3, 4, 5, 6, 7, or 8 exons from FIG and at least 1, 2, 3, 4, 5, 6, 7, 9,10, 11, 12, or 13 ROS1 exons. In one embodiment, the encoded 5'FIG-3'ROS1 fusion polypeptide includes a coiled coil domain, or a functional fragment thereof, and a ROS1 tyrosine kinase domain or a functional fragment thereof.

In one embodiment, the SLC34A2-ROS1 fusion nucleic acid molecule includes a nucleotide sequence that has an in-frame fusion of exon 2 and/or 4 of SLC34A2 with exon 32, and/or 34, and/or 35 of ROS1. In another embodiment, the nucleic acid molecule includes a nucleotide sequence that includes a breakpoint. For example, the SLC34A2-ROS1 fusion can include an in-frame fusion of at least exon 4 of SLC34A2 or a fragment thereof (e.g., exons 1-4 of SLC34A2 or a fragment thereof) with at least exon 32 and/or 34 of ROS1 or a fragment thereof (e.g., exons 30-43 of ROS1 or a fragment thereof). In certain embodiments, the SLC34A2-ROS1 fusion is in a 5-SLC34A2 to 3'-ROS1 configuration. In one embodiment, the nucleic acid molecule includes the nucleotide sequence of exons 1-4 of the SLC34A2 gene, or a fragment thereof. In another embodiment, the nucleic acid molecule includes the nucleotide sequence of exons 30-43 e.g. 32, and/or 34, and/or 35 of the ROS1 gene, or a fragment thereof, or a sequence substantially identical there to.

In one embodiment, the CD74-ROS1 fusion nucleic acid molecule includes a nucleotide sequence that has an in-frame fusion of exons 1-6 of CD74 with exon 32, and/or 34 of ROS1. In another embodiment, the nucleic acid molecule includes a nucleotide sequence that includes a breakpoint. For example, the CD74-ROS1 fusion can include an in-frame fusion of at least exon 6 of CD74 or a fragment thereof (e.g., exons 1-6 of CD74 or a fragment thereof) with at least exon 32 and/or 34, and/or 35 of ROS1, or a fragment thereof (e.g., exons 30-43 of ROS1 or a fragment thereof). In certain embodiments, the CD74-ROS1 fusion is in a 5'-CD74 to 3'-ROS1 configuration. In one embodiment, the nucleic acid molecule includes the nucleotide sequence of exons 1-6 of the CD74 gene, or a fragment thereof. In another embodiment, the nucleic acid molecule includes the nucleotide sequence of exons 30-43 e.g. 32, and/or 34 and/or 35 of the ROS1 gene, or a fragment thereof, or a sequence substantially identical there to.

In one embodiment, the FIG-ROS1 fusion nucleic acid molecule includes a nucleotide sequence that has an in-frame fusion of exons 1-8, e.g. 1-4, or 1-8 of FIG with exon 32, and/or 34, and/or 35 of ROS1. In another embodiment, the nucleic acid molecule includes a nucleotide sequence that includes a breakpoint. For example, the FIG-ROS1 fusion can include an in-frame fusion of at least exon 4 and/or exon 8 of FIG or a fragment thereof (e.g., exons 1-4, or 1-8, of FIG or a fragment thereof) with at least exon 32 and/or 34, and/or 35 of ROS1 or a fragment thereof (for example, exons 30-43 of ROS1 or a fragment thereof). In certain embodiments, the FIG-ROS1 fusion is in a 5'-CD74 to 3'-ROS1 configuration. In one embodiment, the nucleic acid molecule includes the nucleotide sequence of exons 1-6 of the CD74 gene, or a fragment thereof. In another embodiment, the nucleic acid molecule includes the nucleotide sequence of exons 30-43 e.g. 32, and/or 34, and/or 35 of the ROS1 gene, or a fragment thereof, or a sequence substantially identical there to.

In other embodiments, the SLC34A2-ROS1 fusion nucleic acid molecule includes a nucleotide sequence encoding a SLC34A2-ROS1 fusion polypeptide that includes a fragment of a SLC34A2 gene and a fragment of a ROS 1 proto-oncogene. In one embodiment, the nucleotide sequence encodes a SLC34A2-ROS1 fusion polypeptide that includes a helical domain or a functional fragment thereof, and a ROS1 tyrosine kinase domain or a functional fragment thereof.

In another embodiment, the CD74-ROS1 fusion nucleic acid molecule includes a CD74-ROS1 fusion that includes a fusion junction between the ROS1 transcript and the CD74 transcript In another embodiment, the nucleic acid molecule includes a fusion, e.g., an in-frame fusion, of at least exon 32, or 34, or 35 of ROS1 or a fragment thereof (e.g., exons 30-43 of ROS1 or a fragment thereof), and at least exon 1 or a fragment thereof (e.g., exons 1-6 of CD74 or a fragment thereof). In certain embodiments, the CD74-ROS1 fusion is in a 5'-CD74 to 3'-ROS1 configuration. In one embodiment, the nucleic acid molecule includes the nucleotides corresponding to exons 30-43 of a ROS1 gene, e.g. exons 32, 34, or 35 or a fragment thereof, or a sequence substantially identical there to.

In another embodiment, the FIG-ROS1 fusion nucleic acid molecule includes a FIG-ROS1 fusion that includes a fusion junction between the ROS1 transcript and the FIG transcript In another embodiment, the nucleic acid molecule includes a fusion, e.g., an in-frame fusion, of at least exon 32, or 34, or 35 of ROS1 or a fragment thereof (e.g., exons 30-43 of ROS1 or a fragment thereof), and at least exon 4 or 8 a fragment thereof (e.g., exons 1-8 of FIG or a fragment thereof). In certain embodiments, the FIG-ROS1 fusion is in a 5'- FIG to 3'- ROS1 configuration. In one embodiment, the nucleic acid molecule includes the nucleotides corresponding to exons 30-43 of a ROS1 gene, e.g. exons 32, 34, or 35 or a fragment thereof, or a sequence substantially identical there to.

In a related aspect, the invention features nucleic acid constructs that include the SLC34A2-ROS1, CD74-ROS1, and FIG-ROS1fusion nucleic acid molecules described herein. In certain embodiments, the nucleic acid molecules are operatively linked to a native or a heterologous regulatory sequence. Also included are vectors and host cells that include the SLC34A2-ROS1 nucleic acid molecules described herein, e.g., vectors and host cells suitable for producing the nucleic acid molecules and polypeptides described herein.

In another aspect, the invention features nucleic acid molecules that reduces or inhibits the expression of a nucleic acid molecule that encodes a SLC34A2-ROS1 fusion described herein. Examples of such nucleic acid molecules include, for example, antisense molecules, ribozymes, RNAi, triple helix molecules that hybridize to a nucleic acid encoding SLC34A2-ROS1, CD74-ROS1, and FIG-ROS1 or a transcription regulatory region of SLC34A2-ROS1, CD74-ROS1, and FIG-ROS1 and blocks or reduces mRNA expression of SLC34A2-ROS1, CD74-ROS1, or FIG-ROS1.

The invention also features a nucleic acid molecule, e.g., nucleic acid fragment, suitable as probe, primer, bait or library member that includes, flanks, hybridizes to, which are useful for identifying, or are otherwise based on, the ROS1 fusions described herein. In certain embodiments, the probe, primer or bait molecule is an oligonucleotide that allows capture, detection or isolation of a ROS1 fusion nucleic acid molecule described herein. The oligonucleotide can comprise a nucleotide sequence substantially complementary to a fragment of the ROS1 fusion nucleic acid molecules described herein. The sequence identity between the nucleic acid fragment, e.g., the oligonucleotide, and the target ROS1 fusion sequence need not be exact, so long as the sequences are sufficiently complementary to allow the capture, detection or isolation of the target sequence. In one embodiment, the nucleic acid fragment is a probe or primer that includes an oligonucleotide between about 5 and 25, e.g., between 10 and 20, or 10 and 15 nucleotides in length. In other embodiments, the nucleic acid fragment is a bait that includes an oligonucleotide between about 100 to 300 nucleotides, 130 to 230 nucleotides, 150 to 200 nucleotides, 200 to 350, 350 to 950, 300 to 600, 500- 1000, 750- 2000, nucleotides 00 nucleotides, in length and do not necessarily include the ROS1 fusion nucleic acids.

In one embodiment, the nucleic acid fragment can be used to identify or capture, e.g., by hybridization, a SLC34A2-ROS1 fusion, or a CD74-ROS1 fusion, or a FIG-ROS1 fusion. In an illustrative example, the nucleic acid fragment can be a probe, a primer, for use in identifying or capturing, e.g., by hybridization under moderate to high stringency conditions, a SLC34A2-ROS1 fusion or a CD74-ROS1 fusion, or a FIG-ROS1 fusion described herein. In one embodiment, the nucleic acid fragment can be useful for identifying or capturing a SLC34A2-ROS1, or a CD74-ROS1, or a FIG-ROS1 breakpoint. In one exemplary embodiment, the nucleic acid fragment hybridizes to a nucleotide sequence within a chromosomal rearrangement that creates an in-frame fusion of exon 4 or 13 of SLC34A2 with exon 32, 34, or 35 of ROS1 (e.g., a sequence within a translocation on chromosome 6).

The probes or primers described herein can be used, for example, for FISH detection or PCR amplification, or for RT-PCT confirmation of ROS1 rearrangements and identification of binding partners. In one exemplary embodiment where detection is based on PCR, amplification of the SLC34A2-ROS1, or CD74-ROS1, or FIG-ROS1 fusion junctions can be performed using a primer or a primer pair, e.g., for amplifying a sequence flanking the SLC34A2-ROS1, or CD74-ROS1, or FIG-ROS1 fusion junctions described herein, e.g., the mutations or the junction of a chromosomal rearrangement described herein. In one embodiment, a pair of isolated oligonucleotide primers can amplify a region containing or adjacent to a position in the SLC34A2-ROS1 fusion. For example, forward primers can be designed to hybridize to a nucleotide sequence within SLC34A2, CD74, or FIG genomic or mRNA sequence. In various embodiments, primers to ROS1, SLC34A2, and FIG (various species including human) are commercially available from Qiagen (Cat. Nos. QF0018545, QF00449078, and QF00278992 Qiagen, Gaithersburg, MD, USA). In other embodiments, confirmation of the presence of ROS1 fusion polynucleotides can be performed on patient's tumor samples using a panel of PCR primers including SLC34A2 exon 4 and CD74 exon 6 forward primers paired each with a ROS1 exon 32 and exon 34 reverse primer. RNA from the tumor sample can be extracted from FFPE tissue using the Agencourt Formapure method (Agencourt Biosciences, Beverly, MA, USA) and reverse transcribed using the Superscript III cDNA synthesis kit commercially available from Invitrogen, Carlsbad, CA, USA. The following primers can be used to determine SLC34A2-ROS1 or CD74-ROS1 breakpoints: SLC34A2 exon 4 forward, TCGGATTTCTCTACTTTTTCGTG (SEQ ID NO:3); CD74 exon 6 forward, CTCCTGTTTGAAATGAGCAGG (SEQ ID NO:4); ROS1 exon 32 reverse, GGAATGCCTGGTTTATTTGG (SEQ ID NO:5); and ROS1 exon 34 reverse, TGAAACTTGTTTCTGGTATCCAA (SEQ ID NO:6) . PCR amplifications can be performed in any PCR thermal cycler, for example, a Eppendorf Mastercycler Gradient (Eppendorf, Hamburg, Germany), using Platinum Taq polymerase (Invitrogen) under standard conditions with 40 ng of cDNA. cDNA was sequenced using the BigDye 3.0 kit (Life Technologies, Carlsbad, CA).

The nucleic acid fragment can be detectably labeled with, e.g., a radiolabel, a fluorescent label, a bioluminescent label, a chemiluminescent label, an enzyme label, a binding pair label, or can include an affinity tag; a tag, or identifier (e.g., an adaptor, barcode or other sequence identifier).

In another exemplary embodiment, a method of determining the presence of a SLC34A2-ROS1, or CD74-ROS1, or FIG-ROS1 fusion comprising: directly acquiring knowledge that a SLC34A2-ROS1, or CD74-ROS1, or FIG-ROS1 fusion nucleic acid molecule or polypeptide is present in a sample from a subject. The sample can be a sample comprised of fluid, cells, tissue, e.g., a tumor tissue, it can include a nucleic acid sample, a protein sample, a tumor biopsy or a circulating tumor cell or nucleic acid, it can be chosen from a lung cancer, including a NSCLC, a SCLC, a SCC, or a combination thereof and an adenocarcinoma or a melanoma.

The method of detecting the SLC34A2-ROS1, or CD74-ROS1, or FIG-ROS1 fusion in a nucleic acid molecule, and by the use of any of the following methods: nucleic acid hybridization assay, amplification-based assays, PCR-RFLP assay, real-time PCR, sequencing, screening analysis, FISH, spectral karyotyping or MFISH, comparative genomic hybridization), in situ hybridization, SSP, HPLC or mass-spectrometric genotyping.

The method detecting a SLC34A2-ROS1, or CD74-ROS1, or FIG-ROS1 fusion polypeptide or protein is described including the method of contacting a protein sample with a reagent which specifically binds to a SLC34A2-ROS1, or CD74-ROS1, or FIG-ROS 1 fusion polypeptide; and detecting the formation of a complex of the SLC34A2-ROS1, or CD74-ROS1, or FIG-ROS 1 fusion polypeptide and the reagent. Methods of polypeptide detection include using a reagent labeled with a detectable group to facilitate detection of the bound and unbound reagent, wherein the reagent is an antibody molecule, wherein the level or activity the SLC34A2-ROS1, or CD74-ROS1, or FIG-ROS1 fusion is evaluated, wherein the SLC34A2-ROS1, or CD74-ROS1, or FIG-ROS1 fusion is detected prior to initiating, during, or after, a treatment in a subject, wherein the SLC34A2-ROS1, or CD74-ROS1, or FIG-ROS1 fusion is detected at the time of diagnosis with a cancer, wherein the SLC34A2-ROS1, or CD74-ROS1, or FIG-ROS1 fusion is detected at a predetermined interval, e.g., a first point in time and at least at a subsequent point in time.

Responses to treatment are also included, specifically where, responsive to a determination of the presence of the SLC34A2-ROS1, or CD74-ROS1, or FIG-ROS1 fusion, one or more of the following are used: (1) stratifying a patient population; (2) identifying or selecting the subject as likely or unlikely to respond to a treatment; (3) selecting a treatment option; and/or (4) prognosticating the time course of the disease in the subject

An isolated or purified nucleic acid molecule that encodes a SLC34A2-ROS1, or CD74-ROS1, or FIG-ROS 1 fusion or a breakpoint comprising fragment thereof can be used for confirmatory purpose and direct specific treatment of NSCLC and lung adenocarcinoma ROS 1 fusion positive cancer using the compounds of the present invention. Other constructs can be used to direct specific treatment of ROS1 fusion positive cancers, for example, ROS1-rearranged NSCLC, including: an isolated or purified nucleic SLC34A2-ROS1, or CD74-ROS1, or FIG-ROS1 fusion nucleic acid molecule operatively linked to a native or a heterologous regulatory sequence. An isolated or purified vector comprising a nucleic acid molecule that encodes a SLC34A2-ROS1, or CD74-ROS1, or FIG-ROS1 fusion or a breakpoint comprising fragment thereof. A host cell comprising a vector. A nucleic acid molecule that specifically reduces or inhibits the expression of a nucleic acid molecule that encodes a SLC34A2-ROS1, or CD74-ROS1, or FIG-ROS1 fusion, which can be selected from an antisense molecule, ribozyme, siRNA, or triple helix molecule. An isolated or purified SLC34A2-ROS1, or CD74-ROS1, or FIG-ROS1 fusion polypeptide or breakpoint containing fragment thereof. The isolated or purified SLC34A2-ROS1, or CD74-ROS1, or FIG-ROS1 fusion polypeptide having a ROS1 kinase activity, and/or a dimerizing or multimerizing activity. An isolated or purified antibody molecule that specifically binds a SLC34A2-ROS1, or CD74-ROS1, or FIG-ROS1 fusion polypeptide. The antibody molecule, wherein said antibody molecule is a monospecific antibody molecule to the SLC34A2-ROS1, or CD74-ROS1, or FIG-ROS1 fusion polypeptide.

Examples of the method for detecting the translation product of the SLC34A2-ROS1, or CD74-ROS1, or FIG-ROS 1 fusion polynucleotide in the present invention are immunostaining, western blotting, ELISA, flow cytometry, immunoprecipitation and antibody array analysis. In these methods, an antibody that binds to a SLC34A2-ROS1, or CD74-ROS1, or FIG-ROS 1 fusion polypeptide is used. Examples of such an antibody are an antibody specific to a polypeptide containing a fusion site of the SLC34A2, or CD74, or FIG protein and ROS1 protein (also referred to as "fusion site-specific antibody" hereinafter), an antibody that binds to a polypeptide made up of a region on the C terminal side from the aforementioned fusion site of the ROS1 protein (also referred to as "ROS1-C terminal antibody" hereinafter), and an antibody that binds to a polypeptide made up of a region on the N terminal side from the aforementioned fusion site of the SLC34A2, or CD74, or FIG protein (also referred to as " SLC34A2, or CD74, or FIG -N terminal antibody" hereinafter). Here, "fusion site-specific antibody" means an antibody that specifically binds to a polypeptide containing the aforementioned fusion site but does not bind to either wild-type (normal-type) SLC34A2, or CD74, or FIG protein or wild-type (normal-type) ROS1 protein.

A SLC34A2-ROS1, or CD74-ROS1, or FIG-ROS1 fusion polypeptide can be detected by the aforementioned fusion site-specific antibody or a combination of the aforementioned ROS1-C terminal antibody and SLC34A2, or CD74, or FIG -N terminal antibody. However, since almost no expression of the ROS1 protein, for example, is detected in normal lung cells, the presence of a SLC34A2-ROS1, or CD74-ROS1, or FIG-ROS1 fusion polypeptide in lung adenocarcinoma tissue can be detected even if the ROS1-C terminal antibody alone is used in immunostaining.

In various embodiments, immunohistochemical staining (IHC) and western blotting applications for the identification of ROS1 fusion polypeptides of the present invention can be performed using phospho-ROS1, ROS1 antibodies that are commercially available from Cell Signaling Technology (Danvers, MA, USA). In one embodiment, identification of ROS1 fusion positive cancer cells, for example NSCLC ROS1 rearranged lung cancer cells can be made with rabbit anti-ROS1 monoclonal antibody D4D6, Cat. No. 3287 (Cell Signaling Technology, Danvers, MA, USA).

In some embodiments, the "antibody that binds to a SLC34A2-ROS1, or CD74-ROS1, or FIG-ROS1 fusion polypeptide" can be prepared by a person skilled in the art selecting an appropriate known method. An example of such known methods is a method in which an immune animal is inoculated with the aforementioned polypeptide made up of a C terminal portion of the ROS1 protein, a SLC34A2-ROS1, or CD74-ROS1, or FIG-ROS1 fusion polypeptide, the aforementioned polypeptide made up of an N terminal portion of the SLC34A2, or CD74, or FIG protein, etc. thereby activating the immune system of the animal, and then the blood serum of the animal is recovered (polyclonal antibody), and methods for producing monoclonal antibodies such as the hybridoma method, recombinant DNA method and phage display method. If an antibody to which a labeled substance is bound is used, the target protein can be directly detected by detecting the label. The labeled substance is not particularly limited provided that it can bind to the antibody and can be detected. Examples include peroxidase, β-D-galactosidase, microperoxidase, horseradish peroxidase (HRP), fluorescein isothiocyanate (FITC), rhodamine isothiocyanate (RITC), alkali phosphatase, biotin, and radioactive substances. Furthermore, in addition to methods that directly detect a target protein using an antibody to which a labeled substance is bound, methods that indirectly detect the target protein using protein G, protein A or a secondary antibody to which a labeled substance is bound may also be used.

If the presence of a SLC34A2-ROS1, or CD74-ROS1, or FIG-ROS1 fusion polynucleotide or polypeptide is detected in a specimen isolated from a subject by the aforementioned methods, it is judged that efficacy of cancer treatment by a ROS1 tyrosine kinase inhibitor such as a compound of Formula I, Ia or compound 1, or a pharmaceutically acceptable salt thereof, will be high in that patient, whereas if the presence of a SLC34A2-ROS1, or CD74-ROS1, or FIG-ROS1 fusion polynucleotide is not detected, it is judged that efficacy of cancer treatment by a ROS1 tyrosine kinase inhibitor will be low in that patient

As described above, any of the polynucleotides stated in (a) through (c) below having a chain length of at least 15 bases may be advantageously used in detecting the presence or absence of a SLC34A2-ROS1, or CD74-ROS1, or FIG-ROS1 fusion polynucleotide;
(a) a polynucleotide that is at least one probe selected from the group made up of probes that hybridize to a polynucleotide that encodes the SLC34A2, or CD74, or FIG protein and probes that hybridize to a polynucleotide that encodes the ROS1 protein.;
(b) a polynucleotide that is a probe that hybridizes to a fusion site of a polynucleotide that encodes the SLC34A2, or CD74, or FIG protein and a polynucleotide that encodes the ROS1 protein;
(c) a polynucleotide that is a pair of primers designed to sandwich a fusion site of a polynucleotide that encodes the SLC34A2, or CD74, or FIG protein and a polynucleotide that encodes the ROS1 protein.

These polynucleotides have a base sequence complementary to the specific base sequence of the target gene. Here, "complementary" may mean not completely complementary, provided that it hybridizes, for example, under mopderate to high stringency conditions. For example, these polypeptides have 80% or higher, preferably 90% or higher, more preferably 95% or higher, and most preferably 100% homology with the specified base sequence.

In the polynucleotides of (a) through (c), in part or all of DNA or RNA, nucleotides may be substituted with artificial nucleic acids such as PNA (polyamide nucleic acid, peptide nucleic acid), LNA (trademark, locked nucleic acid, bridged nucleic acid), ENA (trademark, 2'-O,4'-C-ethylene-bridged nucleic acids), GNA (glycerol nucleic acid) and TNA (threose nucleic acid).

Further, as described above, an antibody that binds to a SLC34A2-ROS1, or CD74-ROS1, or FIG-ROS1 fusion polypeptide is advantageously used in detecting the translation product of a SLC34A2-ROS1, or CD74-ROS1, or FIG-ROS1 fusion polynucleotide. Accordingly, the present invention provides a drug for determining the effectiveness of cancer treatment by a ROS1 tyrosine kinase inhibitor, comprising this antibody.

The detection method of a fusion gene of the present invention includes a step of detecting the presence of polynucleotides of the present specification in a sample obtained from a test subject. As the sample obtained from a test subject, substances collected from a test subject (samples isolated from a living body), specifically, any types of body fluid (preferably blood) collected, alveolar and bronchial washings, samples having undergone biopsy, and phlegm samples are used. Preferably, a biopsy sample or a phlegm sample from an affected area in the lung of a test subject is used. From the sample, genome DNA can be extracted and used. In addition, transcripts thereof (products produced as a result of transcription and translation of a genome; for example, mRNA, cDNA and proteins) can be used. Particularly, it is preferable to prepare and use mRNA or cDNA.

Genome DNA can be extracted by known methods, and the extraction can be easily performed using a commercially available DNA extraction kit.

The step of detection can be performed according to known gene analysis methods (for example, known methods that are commonly used as gene detection methods such as PCR, LCR (Ligase chain reaction), SDA (Strand displacement amplification), NASBA (Nucleic acid sequence-based amplification), ICAN (Isothermal and chimeric primer-initiated amplification of nucleic acids), LAMP (Loop-mediated isothermal amplification) method, TMA (Gen-Probe's TMA system) method, in situ hybridization method, and microar-rays). For example, a hybridization technology in which a nucleic acid hybridized with a polynucleotide to be detected is used as a probe, a gene amplification technology in which DNA hybridized with a polynucleotide to be detected is used as a primer, or the like is used.

Specifically, the detection is performed using nucleic acids derived from a sample obtained from a test subject, for example, mRNA and the like. The amount of mRNA is measured by a method of gene amplification reaction by using primers that are designed so as to be able to specifically amplify the sequence of a polynucleotide to be detected. The primers used in the detection method of the present invention, or the primers included in the detection kit are not particularly limited as long as the primers can specifically amplify the sequence of a polynucleotide to be detected, and designed based on the base sequence of a polynucleotide to be detected. Primers used in the PCR amplification monitor method can be designed using primer design software (for example, Primer Express manufactured by PE Biosystems) and the like. In addition, since the larger the size of a PCR product is, the more the amplification efficiency worsens, it is appropriate for a sense primer and an antisense primer to be designed such that the size of amplification products obtained when mRNA or cDNA is amplified becomes 1 kb or less.

More specifically, a sense primer (5'-primer) is designed from a portion encoding SLC34A2, and an antisense primer (3'-primer) is designed from a portion encoding ROS1. It is preferable to use the primer included in the detection kit of the present invention, and it is more preferable to use the primer that is most suitably included in the detection kit. In the PCR amplification monitor method, it is also possible to design multiplex PCR for detecting all fusion polynucleotides in a single reaction liquid, by mixing the above sense primers corresponding to respective genes. By the method suitable for each amplification technology, it is possible to confirm whether or not a target gene (whole gene or a specific portion thereof) has been amplified. For example, in the PCR method, PCR products are analyzed by agarose gel electrophoresis and subjected to ethidium bromide staining and the like, whereby it is possible to confirm whether or not amplified fragments having a target size have been obtained. When the amplified fragments having a target size have been obtained, this indicates that a polynucleotide to be detected is present in the sample obtained from a test subject The presence of a polynucleotide to be detected can be detected in this manner.

The detection method of a fusion gene of the present invention preferably includes a step of detecting the presence of a specific polynucleotide in a sample obtained from a test subject by a gene amplification reaction and a step of detecting whether or not amplified fragments having a target size have been obtained.

The detection using a hybridization technology is performed using, for example, northern hybridization, dot blotting method, DNA microarray method, and RNA protection method. As probes used for hybridization, it is possible to use a probe which comprises sequences consisting of 16 bases respectively upstream and downstream of the fusion point as a center of a nucleic acid molecule consisting of at least 32 consecutive bases hybridizing with a polynucleotide to be detected or with a complementary strand thereof under a moderate to high stringency conditions (preferably under a high stringency condition), or comprises complementary strands thereof.

It is also possible to use a gene amplification technology such as RT-PCR. In the RT-PCR method, the PCR amplification monitor (real time PCR) method is performed during the process of gene amplification, whereby the presence of a polynucleotide to be detected can be more quantitatively analyzed. PCR amplification monitor methods can be used. Real time PCR is a known method, and can be simply performed using commercially available instruments and kits for this method.

The detection method of a fusion protein of some of the embodiments of the present invention includes a step of detecting the presence of a specific polypeptide in a sample obtained from a test subject, that is, a polypeptide encoded by a polynucleotide to be detected (hereinafter, called a polypeptide to be detected). Such a detection step can be performed by immunoassay method or enzyme activity assay method that is conducted by preparing a solubilized liquid derived from a sample obtained from a test subject (for example, a cancer tissue or cells obtained from a test subject) and combining a polypeptide to be detected contained in the liquid with an anti-SLC34A2, or an anti-CD74, or an anti-FIG antibody and an anti-ROS1 antibody. Preferably, it is possible to use techniques using a monoclonal antibody or a polyclonal antibody specific to a polypeptide to be detected, such as enzymatic immunoassay method, double antibodies sandwich ELISA method, fluorescence immunoassay method, radioimmunoassay method, and western blotting method.

When the polynucleotide to be detected or polypeptide to be detected in the detection method of the present invention is detected from a sample obtained from a test subject, the test subject is a subject (patient) who has cancer with the polynucleotide positive and is to be provided with treatment using ROS1 inhibitors.

The detection kit of the present invention comprises at least sense and antisense primers that are designed so as to be able to specifically amplify a polynucleotide to be detected in the detection method of the present invention. The sense and antisense primer set is a set of polynucleotides functioning as primers for amplifying a polynucleotide to be detected.

In one embodiment, the primer set of the present invention for the detection of a fusion gene comprises (1) a primer set which comprises a sense primer designed from a portion encoding SLC34A2, or CD74, or FIG and an antisense primer designed from a portion encoding ROS1 and is for detecting a fusion gene of SLC34A2, or CD74, or FIG and ROS 1 gene, wherein the anti-sense primer consists of a nucleic acid molecule (preferably a nucleic acid molecule consisting of at least 16 bases) hybridizing with a "polynucleotide to be detected" under moderate to high stringencyconditions (preferably under high stringency conditions), and the sense primer consists of a nucleic acid molecule (preferably a nucleic acid molecule consisting of at least 16 bases) hybridizing with a complementary strand of the "polynucleotide to be detected" under a stringent condition (preferably under high stringency conditions).

The following primer sets (2) and (3) are included in the primer set as more specific variants of primer set (1).

(2) A primer set of a sense primer that consists of an oligonucleotide consisting of at least any 16 consecutive bases.

(3) A primer set of a sense primer that consists of an oligonucleotide consisting of at least any 16 consecutive bases.

In these primer sets (1) to (3), an interval between the positions where the sense primer and the anti-sense primer are selected is preferably 1 kb or less, or the size of an amplification product amplified by the sense primer and the antisense primer is preferably 1 kb or less.

In addition, the primer has a strand length consisting of 15 to 40 bases in general, preferably consisting of 16 to 24 bases, more preferably consisting of 18 to 24 bases, and particularly preferably consisting of 20 to 24 bases.

The primer set can be used for amplifying and detecting a polynucleotide to be detected. Moreover, though not particularly limited, the respective primers included in the primer set of the present invention can be prepared by, for example, chemical synthesis.

Exemplary ROS1, SLC34A2, or CD74 primers operable to detect ROS1 fusion polynucleotides are provided in Table 2.

**Table 2. Exemplary primer set for identifying various ROS1 fusion proteins in accordance with the present invention.**

| **Primer name** | **Primer Sequence (5'-3')** |
|---|---|
| *ROS1* InvPCR F1 | GTCTGGCATAGAAGATTAAAG (SEQ ID NO: 7) |
| *ROS1* InvPCR F2 | AAACGAAGACAAAGAGTTGG (SEQ ID NO: 8) |
| *ROS1* InvPCR R1 | GTCAGTGGGATTGTAACAAC (SEQ ID NO: 9) |
| *ROS1* InvPCR R2 | AAACTTGTTTCTGGTATCC (SEQ ID NO: 10) |
| *ROS1* Break Rev1 | CAGCTCAGCCAACTCTTT (SEQ ID NO: 11) |
| *ROS1* E43R1 | TCTATTTCCCAAACAACGCTATTAATCAGACCC (SEQ ID NO: 12) |
| *ROS1* E34R | TGAAACTTGTTTCTGGTATCCAA (SEQ ID NO: 13) |
| *CD74* E5F | CCTGAGACACCTTAAGAACACCA (SEQ ID NO: 14) |
| *SLC34A2* E4F | TCGGATTTGTGTACTTTTTCGTG (SEQ ID NO: 15) |

A method of screening an anticancer drug includes: contacting a sample compound to a cell expressing the fusion protein; and measuring the fusion protein expression level in the cell, wherein the fusion protein expression level in the cell treated with the sample compound is decreased compared with that before the treatment with the sample compound or that in a non-treated cell, the sample compound is determined as a candidate compound for the anticancer drug.

The method of screening an anticancer drug may further include the step of measuring the fusion protein expression level in the cell before the treatment of the sample compound. In this case the sample compound may be determined as a candidate compound for the anticancer drug when the fusion protein expression level after treatment of the sample compound is decreased compared with that before the treatment with the sample compound in the same cell. Alternatively, the method of screening an anticancer drug may include providing cells expressing the fusion protein, and contacting a sample compound to a part of the provided cells. In this case the sample compound may be determined as a candidate compound for the anticancer drug when the fusion protein expression level in the cell contacted with the sample compound is decreased compared with that in the cells which are not contacted with the sample compound.

The cell used in the screening method may be a cell derived from a cancer cell where the fusion gene or the fusion protein is expressed and/or activated, an extract of the cell, or a culture of the cell. The cancer cell may be a solid cancer cell, in particular a lung cancer, for example a non-small cell lung cancer such as a lung adenocarcinoma, as described above.

Still another embodiment provides a method of screening an anticancer drug against lung cancer including: treating a cell expressing the fusion protein with a sample compound; measuring the fusion protein expression level in the cell, wherein the fusion protein expression level in the cell treated with the sample compound is decreased compared with that before the treatment with the sample compound or that in a non-treated cell, the sample compound is determined as a candidate compound for the anticancer drug against lung cancer.

The ROS1 fusion proteins (i.e. SLC34A2-ROS1, CD74-ROS1, and FIG-ROS1) can be used as a marker for diagnosing a lung cancer or for treating or preventing or treating a lung cancer. The treatment or prevention of lung cancer comprises the step of administering a therapeutically effective amount of at least one inhibitor against the fusion protein, at least one inhibitor against the fusion gene encoding the fusion protein, at least one inhibitor against a ROS1 coding gene, or a combination thereof to a patient in need thereof. The inhibitor can be such as Formula I, Ia or compound 1, or a pharmaceutically acceptable salt thereof.

Another embodiment provides a method of preventing and/or treating a cancer, comprising administering a pharmaceutically (therapeutically) effective amount of at least one inhibitor against the fusion protein, at least one inhibitor against the fusion gene encoding the fusion protein, at least one inhibitor against a ROS1 coding gene, or a combination thereof, to a patient in need thereof, any of which may be the compounds of Formula 1 and the other specific compounds disclosed herein. The method may further comprise the step of identifying the patient who needs the prevention and/or treatment of a cancer, prior to the step of administering such treatment. Another embodiment provides a composition for preventing and/or treating a cancer, comprising administering at least one inhibitor against the fusion protein, including Formula 1, either alone or with at least one inhibitor against the fusion gene encoding the fusion protein, at least one inhibitor against a ROS1 coding gene, or a combination thereof. Another embodiment provides a use of an inhibitor against the fusion protein, an inhibitor against the fusion gene encoding the fusion protein, an inhibitor against a ROS1 coding gene, or a combination thereof, for preventing and/or treating a cancer. The inhibitor can be such as Formula I, Ia or compound 1, or a pharmaceutically acceptable salt thereof.

In the present invention, the cancer may be a lung cancer, in particular a small cell lung cancer (SCLC) or a non-small cell lung cancer (NSCLC) such as a lung adenocarcinoma, a squamous cell lung carcinoma, or a large cell lung carcinoma.

The composition wherein the inhibitor against the ROS1 fusion protein is at least one selected from the group consisting of an aptamer specifically binding to the fusion protein, an antibody specifically binding to the fusion protein and the inhibitor against the fusion gene or the ROS1 coding gene is at least one selected from the group consisting of siRNA, shRNA, miRNA, and an aptamer, which are capable of specifically binding to the fusion gene or the ROS1 coding gene.

Any ROS1 kinase inhibitor that inhibits the expression of ROS1 and/or ROS1 kinase activity (e.g., transcription, translation, or stability) may be used alone or in combination with the compound of Formula I, Ia, or compound 1, or a pharmaceutically acceptable salt thereof.

The inhibiting agent may be ROS1-specific, or it may be non-specific (e.g., non-specific kinase inhibitors, multi-target inhibitors). Several ROS1 kinase inhibitors have been developed, and their clinical applications are being investigated. Downstream of the ROS 1 kinase activity, there are kinases such as phosphatidylinositol 3 kinases (PI3K) and extracellular signal kinases 1/2 (ERK) (Wixted JH et al. J Biol Chem 2011), and STAT3 (Hwang JH et al. Mol Endocrinol 2003; 17: 1155-1166). A drug that inhibits such downstream signal transmission routes can also be used as an alternative to an ROS1 kinase inhibitor or as an adjuvant, alone or in combination with the compounds of Formula 1.

In one mode, subjects judged to have a SLC34A2-ROS1, or CD74-FIG translocation or a FIG-ROS1 deletion, are also judged as having an ROS1 mutation at a site beyond the translocation site. An example of an ROS1 mutation is an activating mutation, which enables constitutive kinase activity. The ROS1 activating mutation is an arbitrary mutation that causes an increase in activation in comparison with the wild type mutation. For example, an ROS1 activating mutation may bring about permanent ROS1 activation. There may even be a secondary ROS1 activating mutation variant that is associated with or is due to the use of an ROS1 inhibitor. Mutations that give rise to an increase in ROS1 signal activity occur because of, for example, a kinase domain point mutation, deletion, insertion, duplication, or inversion, or combination of two or more of those, which give rise to an increase in the ROS1 signal. For subjects who are judged to have both a ROS1 fusion translocation or deletion as provided herein, and an ROS1 mutation, the decision can also be made to treat them with an ROS1 kinase inhibitor. In addition, treatment/therapy may be carried out on them based on that decision.

As described above, the effectiveness of cancer treatment by a ROS1 tyrosine kinase inhibitor is considered to be high in a patient in whom a SLC34A2-ROS1, or a CD74-ROS1, or a FIG-ROS1 fusion polynucleotide is detected by the method of the present invention. For this reason, cancer treatment can be efficiently performed by administering a ROS1 tyrosine kinase inhibitor selectively to those cancer patients who possess the SLC34A2-ROS1, or a CD74-ROS1, or a FIG-ROS1 fusion gene and the ROS1 gene. Accordingly, the present invention provides a method for treating cancer, comprising a step of administering a ROS1 tyrosine kinase inhibitor which is Formula I, Ia or compound 1, or a pharmaceutically acceptable salt thereof, to a patient in whom the effectiveness of cancer treatment by that ROS1 tyrosine kinase inhibitor was determined to be high by the aforementioned diagnostic method described herein.

In the present invention, a "specimen" is not only a biological specimen (for example, cells, tissue, organ, fluid (blood, lymph fluid, etc.), digestive fluid, sputum, alveolar/bronchial lavage fluid, urine, stools), but also includes nucleic acid extracts (genome DNA extract, mRNA extract, or cDNA preparation or cRNA preparation prepared from mRNA extract) or protein extracts obtained from these biological specimens. This specimen may also be one that has undergone formalin fixing treatment, alcohol fixing treatment, freezing treatment or paraffin embedding treatment.

Furthermore, the genome DNA, mRNA, cDNA or protein may be prepared by a person skilled in the art after selected a suitable known technique considering the type, state and so forth of the specimen.

In addition to the aforementioned substances (polynucleotides, antibodies) as active ingredients, the drug of the present invention may contain other pharmaceutically acceptable ingredients. Examples of such ingredients include buffering agents, emulsifiers, suspending agents, stabilizers, preservatives, physiological saline and so forth. As buffering agents, phosphates, citrates, acetates and so forth may be used. As emulsifiers, gum arabic, sodium alginate, tragacanth and so forth may be used. As suspending agents, glycerol monostearate, aluminum monostearate, methylcellulose, carboxymethylcellulose, hydroxymethylcellulose, sodium lauryl sulfate and so forth may be used. As stabilizers, propylene glycol, diethyl sulfite, ascorbic acid and so forth may be used. As preservatives, sodium azide, benzalkonium chloride, paraoxybenzoic acid, chlorobutanol and so forth may be used.

Furthermore, in addition to the preparation that contains polynucleotides and antibodies, preparations such as a substrate, a positive control (for example, SLC34A2-ROS1, CD74-ROS1, or FIG-ROS1 fusion polynucleotides, SLC34A2-ROS1, CD74-ROS1, or FIG-ROS1 fusion polypeptides, or cells that possess them, etc.) and a negative control necessary for detection of the label appended to the polynucleotides and antibodies, a counterstaining reagent (DAPI, etc.) used in *in situ* hybridization or the like, a molecule required in detecting the antibody (for example, a secondary antibody, protein G, protein A), and buffer solution used in dilution or washing of the antibody may be combined as a kit for use in the method of the present invention. This kit may include instructions for use of the kit. The present invention also provides the aforementioned kit for use in the method of the present invention.

The methods of detection described herein are particularly useful when a fusion protein is detected which consists essentially of N-terminal domain of a fusion partner and C-terminal domain of ROS1 protein encoding a kinase domain. The fusion protein may be SLC34A2-ROS1 fusion protein consisting essentially of N-terminal domain of SLC34A2 protein or fragment thereof and C-terminal domain of ROS1 protein encoding a kinase domain. The fusion protein may be CD74-ROS1 fusion protein consisting essentially of N-terminal domain of CD74 protein or a fragment thereof and C-terminal domain of ROS1 protein encoding a kinase domain. The fusion protein may be FIG-ROS1 fusion protein consisting essentially of N-terminal domain of FIG protein or a fragment thereof and C-terminal domain of ROS1 protein encoding a kinase domain. The method can be used for diagnosing a lung cancer, particularly non small cell lung cancer and includes: detecting at least one of a ROS1-involved chromosomal rearrangement including inversion, translocation, or deletion in chromosome 6; a fusion protein wherein protein is fused with other protein; a fusion gene encoding the fusion protein; and the overexpression of ROS1 compared to a standard sample from an individual without a cancer. When one of the chromosomal rearrangements described above selected from the above group is detected in the test sample, the individual for treatment of a ROS1 inhibitor, such as a Formula I, Ia or compound 1, or a pharmaceutically acceptable salt thereof.

### Preparation of Compound 1

Preparation of N-(4-{[6,7-bis(methyloxy)quinolin-4-yl]oxy}phenyl)-N-(4-fluorophenyl)cyclopropane-1,1-dicarboxamide and the (L)-malate salt thereof.

The synthetic route used for the preparation of N-(4-[6,7-bis(methyloxy)quinolin-4-yl]oxy}phenyl)-N'-(4-fluorophenyl)cyclopropane-1,1-dicarboxamide and the (L)-malate salt thereof is depicted in Scheme 1:

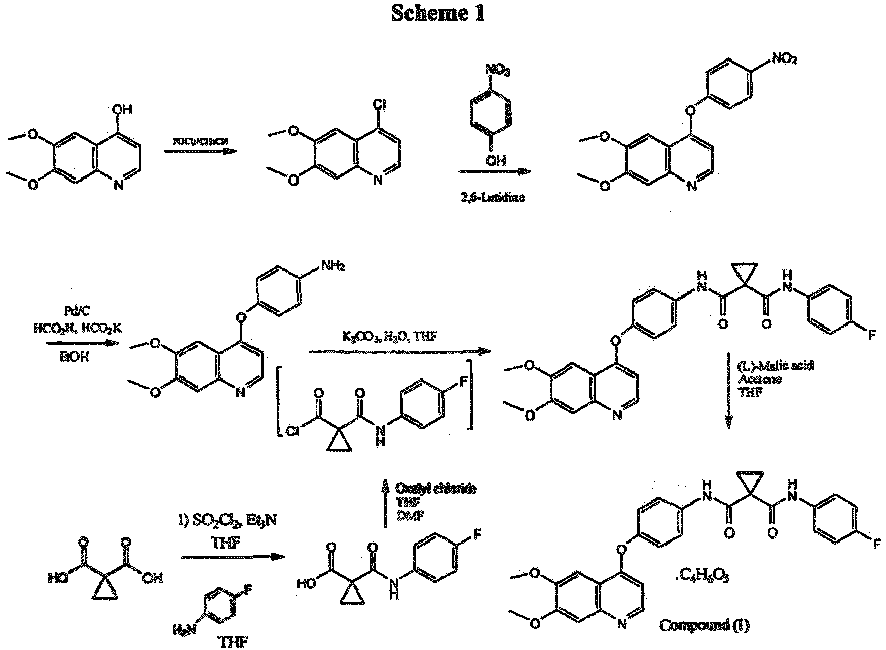

### Preparation of 4-Chloro-6,7-dimethoxy-quinoline

A reactor was charged sequentially with 6,7-dimethoxy-quinoline-4-ol (10.0 kg) and acetonitrile (64.0 L). The resulting mixture was heated to approximately 65 °C and phosphorus oxychloride (POCl₃, 50.0 kg) was added. After the addition of POCl₃, the temperature of the reaction mixture was raised to approximately 80 °C. The reaction was deemed complete (approximately 9.0 hours) when less than 2 percent of the starting material remained (in process high-performance liquid chromotography [HPLC] analysis). The reaction mixture was cooled to approximately 10 °C and then quenched into a chilled solution of dichloromethane (DCM, 238.0 kg), 30% NH₄OH (135.0 kg), and ice (440.0 kg). The resulting mixture was warmed to approximately 14 °C, and phases were separated. The organic phase was washed with water (40.0 kg) and concentrated by vacuum distillation to remove the solvent (approximately 190.0 kg). Methyl-t-butyl ether (MTBE, 50.0 kg) was added to the batch, and the mixture was cooled to approximately 10 °C, during which time the product crystallized out. The solids were recovered by centrifugation, washed with n heptane (20.0 kg), and dried at approximately 40 °C to afford the title compound (8.0 kg).

### Preparation of 6,7-Dimethyl-4-(4-nitro-phenoxy)-quinoline

A reactor was sequentially charged with 4-chloro-6,7-dimethoxy-quinoline (8.0 kg), 4 nitrophenol (7.0 kg), 4 dimethylaminopyridine (0.9 kg), and 2,6 lutidine (40.0 kg). The reactor contents were heated to approximately 147°C. When the reaction was complete (less than 5 percent starting material remaining as determined by in process HPLC analysis, approximately 20 hours), the reactor contents were allowed to cool to approximately 25 °C. Methanol (26.0 kg) was added, followed by potassium carbonate (3.0 kg) dissolved in water (50.0 kg). The reactor contents were stirred for approximately 2 hours. The resulting solid precipitate was filtered, washed with water (67.0 kg), and dried at 25 °C for approximately 12 hours to afford the title compound (4.0 kg).

### Preparation of 4-(6,7-Dimethoxy-quinoline-4-yloxy)-phenylamine

A solution containing potassium formate (5.0 kg), formic acid (3.0 kg), and water (16.0 kg) was added to a mixture of 6,7-dimethoxy4-(4-nitro-phenoxy)-quinoline (4.0 kg), 10 percent palladium on carbon (50 percent water wet, 0.4 kg) in tetrahydrofuran (THF, 40.0 kg) that had been heated to approximately 60 °C. The addition was carried out such that the temperature of the reaction mixture remained approximately 60 °C. When the reaction was deemed complete as determined using in-process HPLC analysis (less than 2 percent starting material remaining, typically 15 hours), the reactor contents were filtered. The filtrate was concentrated by vacuum distillation at approximately 35 °C to half of its original volume, which resulted in the precipitation of the product. The product was recovered by filtration, washed with water (12.0 kg), and dried under vacuum at approximately 50 °C to afford the title compound (3.0 kg; 97 percent area under curve (AUC)).

### Preparation of 1-(4-Fluoro-phenylcarbamoyl)-cyclopropanecarboxylic acid

Triethylamine (8.0 kg) was added to a cooled (approximately 4 °C) solution of commercially available cyclopropane-1,1-dicarboxylic acid (21, 10.0 kg) in THF (63.0 kg) at a rate such that the batch temperature did not exceed 10 °C. The solution was stirred for approximately 30 minutes, and then thionyl chloride (9.0 kg) was added, keeping the batch temperature below 10 °C. When the addition was complete, a solution of 4-fluoroaniline (9.0 kg) in THF (25.0 kg) was added at a rate such that the batch temperature did not exceed 10 °C. The mixture was stirred for approximately 4 hours and then diluted with isopropyl acetate (87.0 kg). This solution was washed sequentially with aqueous sodium hydroxide (2.0 kg dissolved in 50.0 L of water), water (40.0 L), and aqueous sodium chloride (10.0 kg dissolved in 40.0 L of water). The organic solution was concentrated by vacuum distillation followed by the addition of heptane, which resulted in the precipitation of solid. The solid was recovered by centrifugation and then dried at approximately 35 °C under vacuum to afford the title compound. (10.0 kg).

### Preparation of 1-(4-Fluoro-phenylcarbamoyl)-cyclopropanecarbonyl chloride

Oxalyl chloride (1.0 kg) was added to a solution of 1-(4-fluoro-phenylearbainoyl)-cyclopropanecarboxylic acid (2.0 kg) in a mixture of THF (11 kg) and N,N-dimethylformamide (DMF; 0.02 kg) at a rate such that the batch temperature did not exceed 30 °C. This solution was used in the next step without further processing.

### Preparation of N-(4-{[6,7-bis(methyloxy)quinolin-4-yl]oxy}phenyl)-N'-(4-fluorophenyl)cyclopropane-1,1-dicarboxamide

The solution from the previous step containing 1-(4-fluoro-phenylcarbamoyl)-cyclopropanecarbonyl chloride was added to a mixture of 4-(6,7-dimethoxy-quinoline-4-yloxy)-phenylamine (3.0 kg) and potassium carbonate (4.0 kg) in THF (27.0 kg) and water (13.0 kg) at a rate such that the batch temperature did not exceed 30 °C. When the reaction was complete (in typically 10 minutes), water (74.0 kg) was added. The mixture was stirred at 15-30 °C for approximately 10 hours, which resulted in the precipitation of the product. The product was recovered by filtration, washed with a pre-made solution of THF (11.0 kg) and water (24.0 kg), and dried at approximately 65 °C under vacuum for approximately 12 hours to afford the title compound (free base, 5.0 kg). ¹H NMR (400 MHz, d₆-DMSO): δ 10.2 (s, 1H), 10.05 (s, 1H), 8.4 (s, 1H), 7.8 (m, 2H), 7.65 (m, 2H), 7.5 (s, 1H), 7.35 (s, 1H), 7.25 (m, 2H), 7.15(m, 2H), 6.4 (s, 1H), 4.0 (d, 6H), 1.5 (s, 4H). LC/MS: M+H= 502.

### Preparation of N-(4-{[6,7-bis(methyloxy)quinolin-4-yl]oxy}phenyl)-N'-(4-fluorophenyl)cyclopropane-1,1-dicarboxamide, (L) malate salt

A solution of L-malic acid (2.0 kg) in water (2.0 kg) was added to a solution of Cyclopropane-1,1-dicarboxylic acid [4-(6,7-dimethoxy-quinoline-4-yloxy)-phenyl]-amide (4-fluoro-phenyl)-amide free base (15,5.0 kg) in ethanol, maintaining a batch temperature of approximately 25 °C. Carbon (0.5 kg) and thiol silica (0.1 kg) were then added, and the resulting mixture was heated to approximately 78 °C, at which point water (6.0 kg) was added. The reaction mixture was then filtered, followed by the addition of isopropanol (38.0 kg), and was allowed to cool to approximately 25 °C. The product was recovered by filtration and washed with isopropanol (20.0 kg), and dried at approximately 65 °C to afford the title compound (5.0 kg).

### Alternative Preparation of N-(4-{[6,7-Bis(methyloxy)quinolin-4-yl]oxy}phenyl)-N'-(4-fluorophenyl)cyclopropane-1,1-dicarboxamide and the (L)-malate salt thereof.

An alternative synthetic route that can be used for the preparation of N-(4-{[6,7-bis(methyloxy)quinolin-4-yl]oxy}phenyl)-N'-(4-fluorophenyl)cyclopropane-1,1-dicarboxamide and the (L)-malate salt thereof is depicted in Scheme 2, as described in PCT/US2012/024591, the entire contents of which is incorporated by reference.

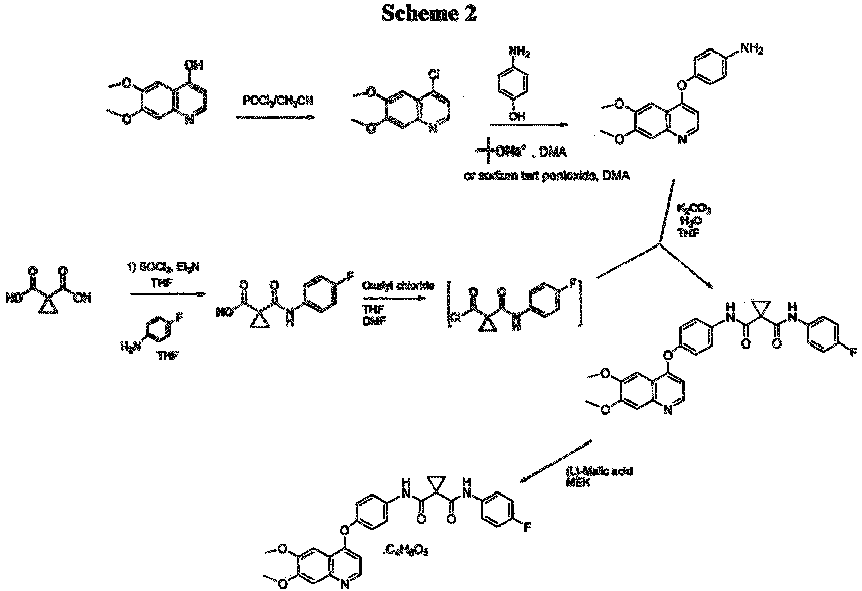

### Preparation of 4-Chloro-6,7-dimethoxy-quinoline

A reactor was charged sequentially with 6,7-dimethoxy-quinoline-4-ol (47.0 kg) and acetonitrile (318.8 kg). The resulting mixture was heated to approximately 60 °C and phosphorus oxychloride (POCl₃, 130.6 kg) was added. After the addition of POCl₃, the temperature of the reaction mixture was raised to approximately 77 °C. The reaction was deemed complete (approximately 13 hours) when less than 3% of the starting material remained (in-process high-performance liquid chromatography [HPLC] analysis). The reaction mixture was cooled to approximately 2-7 °C and then quenched into a chilled solution of dichloromethane (DCM, 482.8 kg), 26 percent NH₄OH (251.3 kg), and water (900 L). The resulting mixture was warmed to approximately 20-25 °C, and phases were separated. The organic phase was filtered through a bed of AW hyflo super-cel NF (Celite; 5.4 kg) and the filter bed was washed with DCM (118.9 kg). The combined organic phase was washed with brine (282.9 kg) and mixed with water (120 L). The phases were separated and the organic phase was concentrated by vacuum distillation with the removal of solvent (approximately 95 L residual volume). DCM (686.5 kg) was charged to the reactor containing organic phase and concentrated by vacuum distillation with the removal of solvent (approximately 90 L residual volume). Methyl t-butyl ether (MTBE, 226.0 kg) was then charged and the temperature of the mixture was adjusted to -20 to -25 °C and held for 2.5 hours resulting in solid precipitate which was then filtered and washed with n-heptane (92.0 kg), and dried on a filter at approximately 25 °C under nitrogen to afford the title compound. (35.6 kg).

### Preparation of 4-(6,7-Dimethoxy-quinoline-4-yloxy)-phenylamine

4-Aminophenol (24.4 kg) dissolved in N,N-dimethylacetamide (DMA, 184.3 kg) was charged to a reactor containing 4-chloro-6,7-dimethoxyquinoline (35.3 kg), sodium t-butoxide (21.4 kg) and DMA (167.2 kg) at 20-25 °C. This mixture was then heated to 100-105 °C for approximately 13 hours. After the reaction was deemed complete as determined using in-process HPLC analysis (less than 2 percent starting material remaining), the reactor contents were cooled at 15-20 °C and water (pre-cooled, 2-7 °C, 587 L) charged at a rate to maintain 15-30 °C temperature. The resulting solid precipitate was filtered, washed with a mixture of water (47 L) and DMA (89.1 kg) and finally with water (214 L). The filter cake was then dried at approximately 25 °C on filter to yield crude 4-(6, 7-dimethoxy-quinoline-4-yloxy)-phenylamine (59.4 kg wet, 41.6 kg dry calculated based on LOD). Crude 4-(6, 7-dimethoxy-quinoline-4-yloxy)-phenylamine was refluxed (approximately 75 °C) in a mixture of tetrahydrofuran (THF, 211.4 kg) and DMA (108.8 kg) for approximately 1hour and then cooled to 0-5 °C and aged for approximately 1 hour after which time the solid was filtered, washed with THF (147.6 kg) and dried on a filter under vacuum at approximately 25 °C to yield 4-(6,7-dimethoxy-quinoline-4-yloxy)-phenylamine (34.0 kg).

### Alternative Preparation of 4-(6,7-Dimethoxy-quinoline-4-yloxy)-phenylamine

4-chloro-6,7-dimethoxyquinoline (34.8 kg) and 4-aminophenol (30.8 kg) and sodium tert pentoxide (1.8 equivalents) 88.7 kg, 35 weight percent in THF) were charged to a reactor, followed by N,N-dimethylacetamide (DMA, 293.3 kg). This mixture was then heated to 105-115 °C for approximately 9 hours. After the reaction was deemed complete as determined using in-process HPLC analysis (less than 2 percent starting material remaining), the reactor contents were cooled at 15-25 °C and water (315 kg) was added over a two hour period while maintaining the temperature between 20-30 °C. The reaction mixture was then agitated for an additional hour at 20-25 °C. The crude product was collected by filtration and washed with a mixture of 88kg water and 82.1 kg DMA, followed by 175 kg water. The product was dried on a filter drier for 53 hours. The LOD showed less than 1 percent w/w.

In an alternative procedure, 1.6 equivalents of sodium tert-pentoxide were used and the reaction temperature was increased from 110-120 °C. In addition, the cool down temperature was increased to 35-40 °C and the starting temperature of the water addition was adjusted to 35-40 °C, with an allowed exotherm to 45 °C.

### Preparation of 1-(4-Fluoro-phenylcarbamoyl)-cyclopropanecarboxylic acid

Triethylamine (19.5 kg) was added to a cooled (approximately 5 °C) solution of cyclopropane-1,1-dicarboxylic acid (24.7 kg) in THF (89.6 kg) at a rate such that the batch temperature did not exceed 5 °C. The solution was stirred for approximately 1.3 hours, and then thionyl chloride (23.1 kg) was added, keeping the batch temperature below 10 °C. When the addition was complete, the solution was stirred for approximately 4 hours keeping temperature below 10 °C. A solution of 4-fluoroaniline (18.0 kg) in THF (33.1 kg) was then added at a rate such that the batch temperature did not exceed 10 °C. The mixture was stirred for approximately 10 hours after which the reaction was deemed complete. The reaction mixture was then diluted with isopropyl acetate (218.1 kg). This solution was washed sequentially with aqueous sodium hydroxide (10.4 kg, 50 percent dissolved in 119 L of water) further diluted with water (415 L), then with water (100 L) and finally with aqueous sodium chloride (20.0 kg dissolved in 100 L of water). The organic solution was concentrated by vacuum distillation (100 L residual volume) below 40 °C followed by the addition of n-heptane (171.4 kg), which resulted in the precipitation of solid. The solid was recovered by filtration and washed with n-heptane (102.4 kg), resulting in wet, crude 1-(4-fluoro-phenylearbamoyl)-cyclopropanecarboxylic acid (29.0 kg). The crude, 1-(4-fluoro-phenylcarbamoyl)-cyclopropanecarboxylic acid was dissolved in methanol (139.7 kg) at approximately 25 °C followed by the addition of water (320 L) resulting in slurry which was recovered by filtration, washed sequentially with water (20 L) and n-heptane (103.1 kg) and then dried on the filter at approximately 25 °C under nitrogen to afford the title compound (25.4 kg).

### Preparation of 1-(4-Fluoro-phenylcarbamoyl)-cyclopropanecarbonyl chloride

Oxalyl chloride (12.6 kg) was added to a solution of 1-(4-fluoro-phenylcarbamoyl)-cyclopropanecarboxylic acid (22.8 kg) in a mixture of THF (96.1 kg) and N, N-dimethylformamide (DMF; 0.23 kg) at a rate such that the batch temperature did not exceed 25 °C. This solution was used in the next step without further processing.

### Alternative Preparation of 1-(4-Fluoro-phenylcarbamoyl)-cyclopropanecarbonyl chloride

A reactor was charged with 1-(4-fluoro-phenylcarbamoyl)-cyclopropanecarboxylic acid (35 kg), 344 g DMF, and 175kg THF. The reaction mixture was adjusted to 12-17 °C and then to the reaction mixture was charged 19.9 kg of oxalyl chloride over a period of 1 hour. The reaction mixture was left stirring at 12-17 °C for 3 to 8 hours. This solution was used in the next step without further processing.

### Preparation of cyclopropane-1,1-dicarboxylic acid [4-(6,7-dimethoxy-quinoline-4-yloxy)-phenyl]-amide(4-fluoro-phenyl)-amide

The solution from the previous step containing 1-(4-fluoro-phenylcarbamoyl)-cyclopropanecarbonyl chloride was added to a mixture of compound 4-(6,7-dimethoxy-quinoline-4-yloxy)-phenylamine (23.5 kg) and potassium carbonate (31.9 kg) in THF (245.7 kg) and water (116 L) at a rate such that the batch temperature did not exceed 30 °C. When the reaction was complete (in approximately 20 minutes), water (653 L) was added. The mixture was stirred at 20-25 °C for approximately 10 hours, which resulted in the precipitation of the product. The product was recovered by filtration, washed with a pre-made solution of THF (68.6 kg) and water (256 L), and dried first on a filter under nitrogen at approximately 25 °C and then at approximately 45 °C under vacuum to afford the title compound (41.0 kg, 38.1 kg, calculated based on LOD).

### Alternative Preparation of cyclopropane-1,1-dicarboxylic acid [4-(6,7-dimethoxy-quinoline-4-yloxy)-phenyl]-amide (4-fluoro-phenyl)-amide

A reactor was charged with 4-(6,7-dimethoxy-quinoline-4-yloxy)-phenylamine (35.7 kg, 1 equivalent), followed by 412.9 kg THF. To the reaction mixture was charged a solution of 48.3 K₄CO₃ in 169 kg water. The acid chloride solution of described in the Alternative Preparation of 1-(4-Fluoro-phenylcarbamoyl)-cyclopropanecarbonyl chloride above was transferred to the reactor containing 4-(6,7-dimethoxy-quinoline-4-yloxy)-phenylamine while maintaining the temperature between 20-30 °C over a minimum of two hours. The reaction mixture was stirred at 20-25 °C for a minimum of three hours. The reaction temperature was then adjusted to 30-25 °C and the mixture was agitated. The agitation was stopped and the phases of the mixture were allowed to separate. The lower aqueous phase was removed and discarded. To the remaining upper organic phase was added 804 kg water. The reaction was left stirring at 15-25 °C for a minimum of 16 hours.

The product precipitated. The product was filtered and washed with a mixture of 179 kg water and 157.9 kg THF in two portions. The crude product was dried under a vacuum for at least two hours. The dried product was then taken up in 285.1 kg THF. The resulting suspension was transferred to reaction vessel and agitated until the suspension became a clear (dissolved) solution, which required heating to 30-35 °C for approximately 30 minutes. 456 kg water was then added to the solution, as well as 20 kg SDAG-1 ethanol (ethanol denatured with methanol over two hours. The mixture was agitated at 15-25 °C fir at least 16 hours. The product was filtered and washed with a mixture of 143 kg water and 126.7 THF in two portions. The product was dried at a maximum temperature set point of 40 °C.

In an alternative procedure, the reaction temperature during acid chloride formation was adjusted to 10-15 °C. The recrystallization temperature was changed from 15-25 °C to 45-50 °C for 1 hour and then cooled to 15-25 °C over 2 hours.

### Preparation of cyclopropane-1,1-dicarboxylic acid [4-(6,7-dimethoxy-quinoline-4-yloxy)-phenyl]-amide (4-fluoro-phenyl)-amide, malate salt

Cyclopropane-1,1-dicarboxylic acid [4-(6,7-dimethoxy-quinoline-4-yloxy)-phenyl]-amide (4-fluoro-phenyl)-amide (1-5; 13.3 kg), L-malic acid (4.96 kg), methyl ethyl ketone (MEK; 188.6 kg) and water (37.3 kg) were charged to a reactor and the mixture was heated to reflux (approximately 74 °C) for approximately 2 hours. The reactor temperature was reduced to 50 to 55 °C and the reactor contents were filtered. These sequential steps described above were repeated two more times starting with similar amounts of starting material (13.3 kg), L-Malic acid (4.96 kg), MEK (198.6 kg) and water (37.2 kg). The combined filtrate was azeotropically dried at atmospheric pressure using MEK (1133.2 kg) (approximate residual volume 711 L; KF ≤ 0.5 % w/w) at approximately 74 °C. The temperature of the reactor contents was reduced to 20 to 25 °C and held for approximately 4 hours resulting in solid precipitate which was filtered, washed with MEK (448 kg) and dried under vacuum at 50 °C to afford the title compound (45.5 kg).

### Alternative Preparation of cyclopropane-1,1-dicarboxylic acid [4-(6,7-dimethoxy-quinoline-4-yloxy)-phenyl]-amide (4-fluoro-phenyl)-amide, (L) malate salt

Cyclopropane-1,1-dicarboxylic acid [4-(6,7-dimethoxy-quinoline-4-yloxy)-phenyl]-amide (4-fluoro-phenyl)-amide (47.9 kg), L-malic acid (17.2), 658.2 kg methyl ethyl ketone, and 129.1 kg water (37.3 kg) were charged to a reactor and the mixture was heated 50-55 °C for approximately 1-3 hours, and then at 55-60 °C for an addition al 4-5 hours. The mixture was clarified by filtration through a 1 µm cartridge. The reactor temperature was adjusted to 20-25 °C and vacuum distilled with a vacuum at 150-200 mm Hg with a maximum jacket temperature of 55 °C to the volume range of 558-731 L.

The vacuum distillation was performed two more times with the charge of 380 kg and 380.2 kg methyl ethyl ketone, respectively. After the third distillation, the volume of the batch was adjusted to 18 v/w of cyclopropane-1,1-dicarboxylic acid [4-(6,7-dimethoxy-quinoline-4-yloxy)-phenyl]-amide (4-fluoro-phenyl)-amide by charging 159.9 kg methyl ethyl ketone to give a total volume of 880L. An addition al vacuum distillation was carried out by adjusting 245.7 methyl ethyl ketone. The reaction mixture was left with moderate agitation at 20-25 °C for at least 24 hours. The product was filtered and washed with 415.1 kg methyl ethyl ketone in three portions. The product was dried under a vacuum with the jacket temperature set point at 45 °C.

In an alternative procedure, the order of addition was changed so that a solution of 17.7 kg L-malic acid dissolved in 129.9 kg water was added to cyclopropane-1,1-dicarboxylic acid [4-(6,7-dimethoxy-quinoline-4-yloxy)-phenyl]-amide (4-fluoro-phenyl)-amide (48.7 kg) in methyl ethyl ketone (673.3 kg).

### Biological Example

### Growth of HCC-78 and NCI-H2228 cells

Both HCC-78 and NCI-H2228 cells were received from DSMZ and ATCC respectively as a frozen biological sample thawed into a T-75 flask (Nunc) and grown until 80 to 90% confluent for adherent cells or until cells were at a density of 1 - 2 x 10⁶ cells/mL for suspension cells.

NCI-H2228, a human lung NSCLC adenocarcinoma cell line (ATCC, CRL 5935) harboring the activating EML4-ALK fusions, were seeded at a density of 3.5 x 10⁴ cells/well and 2.5 x 10⁴ cells/well for 48 hours or 72 hours respectively, in 0.1 ml in RPMI 1640 (ATCC 30-2001), 10 % FBS, 1 % penicillin-streptomycin onto black 96-well plates (Costar, 3904).

HCC-78, a human lung NSCLC adenocarcinoma cell line (DSMZ, ACC 563) harboring the activating SLC34A2-ROS1 fusions and phosphor-Met, were seeded at a density of 2.5 x 10⁴ cells/well and 2 x 10⁴ cells/well for 48 hours or 72 hours respectively, in 0.1 ml in RPMI 1640 (ATCC 30-2001), 10 % FBS (heat-inactivated, Cellgro), 1 % penicillin-streptomycin onto black 96-well plates (Costar, 3904).

Cells were grown at 37°C in a humidified, 5% CO₂ atmosphere for 16 hours. DMSO or serial dilutions of compound 1 in fresh serum free medium or serum containing medium were added to the cells and incubated for 48 hours or 72 hours at 37°C in a humidified, 5% CO₂ atmosphere prior to the addition of 5'-bromo-2'-deoxy-uridine (BrdU) labelling solution (Roche) for 2 to 4 hours. Suspension cells were transferred to filter plates (Corning, 3504) before performing the assay. Cells were assayed for proliferation by monitoring BrdU incorporation. Reagents from the Cell Proliferation ELISA, BrdU (chemiluminescence) kit (Roche Biosciences, 11 669 915 001) were used according to the manufacturer's instructions. After BrdU labelling, cells were fixed with FixDenat solution. anti-BrdU peroxidase (POD) conjugate was added and plates were washed with 1x PBS. Substrate solution was added, and luminescence measurements were taken on a Victor Wallac V plate reader (Perkin Elmer). The percentage inhibition of proliferation was calculated by comparing cell proliferation with compound 1 treatment to DMSO treated samples. Percentage inhibition values at 5 to 8 compound 1 concentrations were used to calculate IC₅₀ values.

### Biological Example

### Inhibition of ROS1 phosphorylation

HCC-78 cells, a human lung NSCLC adenocarcinoma cell line (DSMZ, ACC 563) harboring the activating SLC34A2-ROS1 fusion and phosphor-Met, were seeded at 3 x 10⁵ cells/well onto 6-well plates (Nunclon, 152795), RPMI 1640 (ATCC, 30-2001) containing 10% FBS (heat-inactivated, Cellgro) and 1% penicillin-streptomycin (Cellgro). DMSO or serial dilutions of compound 1 in fresh medium with 10% FBS in a final concentration of 0.3% DMSO (vehicle) were added to the cells and incubated for 3 hour. After treatment, cells were collected and washed with cold PBS and immediately lysed with 0.15 mL of cold lysis buffer (50 mM Tris HCl, pH 8.0,150 mM NaCl, 1% NP 40, 0.1% SDS, 0.5% sodium deoxycholate, 1 mM EDTA, 50 mM NaF, 1 mM sodium pyrophosphate, 1 mM 2Na3VO₄, 2 mM PMSF, 10 µg/mL aprotinin, 5 µg/mL leupeptin and 5 µg/mL pepstatin A). Lysates were collected, and protein concentrations were measured by the BCA method (Pierce). Lysates were collected and protein concentrations were measured by the BCA method (Pierce). Lysates were mixed with NuPage LDS sample buffer (Invitrogen NP0007) and Reducing Agent (Invitrogen #NP0004), then heated at 70°C for 10 min. 20 µg protein was loaded onto NuPage 10% Bis Tris gels (Invitrogen, NP0302). Proteins were transferred to nitrocellulose membranes (Invitrogen, LC2001), blocked for 1 hr. in Odyssey Blocking Buffer (Li-Cor,927 40000), and incubated at 4°C overnight with the anti-ROS1 mouse monoclonal antibody (1:1000) (Cell Signaling Technology, 3266) and Anti-phospho ROS1 (Y2274) rabbit antibody (1:1000) (Cell Signaling Technology, 3077) diluted in Odyssey Blocking Buffer containing 0.1% Tween 20. Membranes were washed four times for 10 min each with TBS T buffer (50 mM Tris HCl, pH7.2; 150 mM NaCl; 0.1% Tween 20) and blotted with Goat anti Mouse IRDye680 (Li-Cor, 926 32220) and Goat anti Rabbit IRDye800 (Li-Cor, 926 32211) secondary antibodies in Odyssey Blocking buffer containing 0.1% Tween 20 and 0.01% SDS for 60 min at room temperature. Membranes were washed four times for 10 min each with TBS-T buffer and rinsed with PBS twice. The membranes were scanned using the Odyssey Scanner (Li-Cor) and the signal intensity of each band was quantified using ImageQuant (Molecular Devices). IC₅₀ values were calculated based on the signal with compound treatment compared to the vehicle (DMSO) control.

Inhibition of SLC34A2-ROS1mediated kinase activity in HCC-78 NSCLC cells administered with single escalating doses of compound 1, or 3-[(1*R*)-1-(2,6-dichloro-3-fluorophenyl)ethoxy]-5-(1-piperidin-4-ylpyrazol-4-yl)pyridin-2-amine (Crizotinib; XALKORI®) is shown in Fig. 1. Fig. 1 clearly illustrates the potent inhibitory activity of compound 1 against ROS1 fusion kinase, and is more potent than the comparator control, 3-[(1*R*)-1-(2,6-dichloro-3-fluorophenyl)ethoxy]-5-(1-piperidin4-ylpyrazol-4-yl)pyridin-2-amine (Crizotinib) at the concentrations tested in vitro.

### Biological Example

### ROS1-Dependent Proliferation of HCC-78 NSCLC Cells

IC₅₀ values were calculated for compound 1 and comparator control, 3-[(1*R*)-1-(2,6-dichloro-3-fluorophenyl)ethoxy]-5-(1-piperidin-4-ylpyrazol-4-yl)pyridin-2-amine (Crizotinib) after incubation of active agents with HCC-78 NSCLC cells for 48 or 72 hours at various concentrations. Quantitation of proliferation of HCC-78 NSCLC cells was performed using BrdU ELISA (chemiluminescence, Roche Biosciences, Cat. No. 11 669 915 001) in accordance with manufacturer's recommendations. Determination of IC₅₀ values (A-E) (concentration at 50% inhibition) were determined as provided above, and computationally determined using GraphPad Prism software.

**Table 3. ROS1-Dependent Proliferation of HCC-78 NSCLC Cells in the presence of compound 1 of the present invention and 3-[(1R)-1-(2,6-dichloro-3-fluorophenyl)ethoxy]-5-(1-piperidin-4-ylpyrazol-4-yl)pyridin-2-amine (Crizotinib).**

| | NCI-H2228 | | HCC-78 | |
|---|---|---|---|---|
| Tissue Type | Lung | | | |
| Pathology | NSCLC Adenocarcinoma | | | |
| Genotype Information | EMIA-ALK fusion TP53 Q331* | | SLS34A2-ROS1 fusion | |
| | CDKN2A M1 *157 del, ?fs: RB1 E204fs*10 | | | |
| Compound Name | 48 hr Prolif. IC₅₀ (nM) | 72 hr Prolif. IC₅₀ (nM) | 48 hr Prolif. IC₅₀ (nM) | 72 hr Prolif. IC₅₀ (nM) |
| Compound 1 | D | E | A | A |
| 3-[(1*R*)-1-(2,6-dichloro-3-fluorophenyl)ethoxy]-5-(1-piperidin-4-ylpyrazol-4-yl)pyridin-2-amine (Crizotinib) | C | C | B | A |

| | | | | |
|---|---|---|---|---|
| IC₅₀ data provided: E- 5,000 - 10,000 nM; D- 1,000-5,000 nM; C- 500-1000 nM; B- 100-500 nM; A- 10-100 nM. | | | | |

### Other Embodiments

The foregoing disclosure has been described in some detail by way of illustration and example, for purposes of clarity and understanding. The invention has been described with reference to various specific and preferred embodiments and techniques. However, it should be understood that many variations and modifications can be made while remaining within the spirit and scope of the invention. It will be obvious to one of skill in the art that changes and modifications can be practiced within the scope of the appended claims. Therefore, it is to be understood that the above description is intended to be illustrative and not restrictive.

The scope of the invention should, therefore, be determined not with reference to the above description, but should instead be determined with reference to the following appended claims, along with the full scope of equivalents to which such claims are entitled.

Preferred embodiments of the present invention are described below and are referred to as Embodiments 1-42:
Embodiment 1. A method for treating lung adenocarcinoma, comprising administering to a patient in need of such treatment a compound of Formula I: 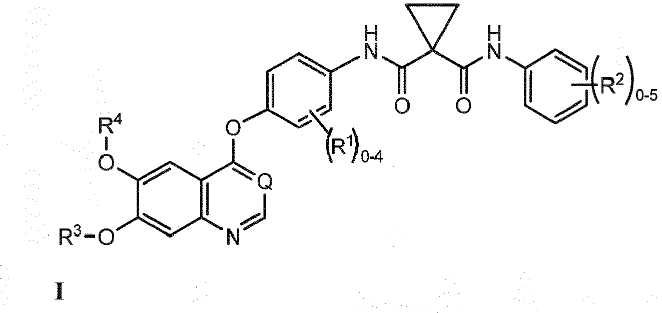 or a pharmaceutically acceptable salt thereof, wherein:
   R¹ is halo;
   R² is halo;
   R³ is (C₁-C₆)alkyl;
   R⁴ is (C₁-C₆)alkyl; and
   Q is CH or N.
Embodiment 2. The method of embodiment 1, wherein the lung adenocarcinoma is non-small cell lung cancer.
Embodiment 3. The method of embodiment 1, wherein the lung adenocarcinoma is SLC34A2-ROS1, CD74-ROS1, or FIG-ROS1 fusion-positive non-small cell lung cancer.
Embodiment 4. The method of any one of embodiments 1-3, wherein the compound of Formula I is a compound of Formula Ia 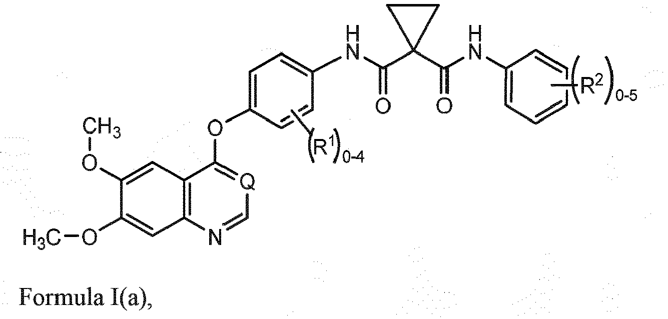 or a pharmaceutically acceptable salt thereof, wherein:
   R¹ is halo;
   R² is halo; and
   Q is CH or N.
Embodiment 5. The method of any one of embodiments 1-4, wherein the compound of Formula I is compound 1: 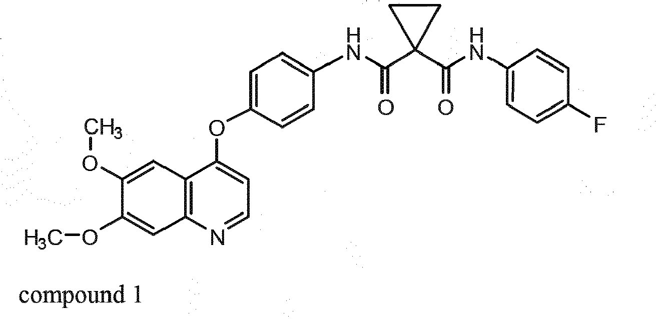 or a pharmaceutically acceptable salt thereof.
Embodiment 6. The method of embodiment 5, wherein compound 1 is N-(4-{[6,7-bis(methyloxy)quinolin-4-yl]oxy}phenyl)-N'-(4-fluorophenyl)cyclopropane-1,1-dicarboxamide, or a pharmaceutically acceptable salt thereof.
Embodiment 7. The method of any one of embodiments 1-6, wherein the compound of Formula (I), Formula I(a) and compound 1 is the (L)- or (D)-malate salt.
Embodiment 8. The method of any one of embodiments 1-7, wherein the compound of Formula (I) is in the crystalline N-1 form or N-2 form of the (L) malate salt and/or the (D) malate salt.
Embodiment 9. The method of any one of embodiments 1-8 wherein the compound of Formula I, I(a), or compound 1, or a pharmaceutically acceptable salt thereof, is administered as a pharmaceutical composition additionally comprising a pharmaceutically acceptable carrier, excipient, or diluent.
Embodiment 10. The method of any one of embodiments 1-9 wherein the compound of Formula I, or a pharmaceutically acceptable salt thereof, is administered subsequent to another form of treatment.
Embodiment 11. The method of any one of embodiments 1-9 wherein the compound of Formula I, or a pharmaceutically acceptable salt thereof, is administered post-cisplatin and/or gemcitabine treatment.
Embodiment 12. The method of any one of embodiments 1-9 wherein the compound of Formula I, or a pharmaceutically acceptable salt thereof, is administered post-carboplatin treatment.
Embodiment 13. The method of any one of embodiments 1-9 wherein the compound of Formula I, or a pharmaceutically acceptable salt thereof, is administered post-carboplatin and/or gemcitabine treatment.
Embodiment 14. The method of any one of embodiments 1-9 wherein the compound of Formula I is administered post-cisplatin and/or carboplatin treatment.
Embodiment 15. The method of any one of embodiments 1-9 wherein the compound of Formula I, or a pharmaceutically acceptable salt thereof, is administered post-docetaxel treatment.
Embodiment 16. The method of any one of embodiments 1-9 wherein the compound of Formula I, or a pharmaceutically acceptable salt thereof, is administered post-crizotinib treatment.
Embodiment 17. The method of any one of embodiments 1-9 wherein the compound of Formula I, or a pharmaceutically acceptable salt thereof, is administered post-crizotinib and/or gemcitabine treatment.
Embodiment 18. The method of any one of embodiments 1-9 wherein the compound of Formula I, or a pharmaceutically acceptable salt thereof, is administered post-crizotinib and/or gemcitabine and/or docetaxel treatment.
Embodiment 19. The method of any one of embodiments 1-9 wherein the compound of Formula I, or a pharmaceutically acceptable salt thereof, is administered post-cisplatin and/or gemcitabine and/or docetaxel treatment.
Embodiment 20. A method for treating a ROS1 fusion-positive non-small cell lung cancer in a patient in need of such treatment comprising administering a therapeutically effective amount of compound 1 or a pharmaceutically acceptable salt thereof.
Embodiment 21. A method for inhibiting or reversing the progress of abnormal cell growth in a mammal, comprising administering compound 1 or a pharmaceutically acceptable salt thereof, wherein the abnormal cell growth is cancer mediated by ROS1 kinase.
Embodiment 22. The method of embodiment 21, wherein the cancer is lung adenocarcinoma.
Embodiment 23. The method of embodiment 21, wherein the lung adenocarcinoma is non-small cell lung cancer.
Embodiment 24. The method of embodiment 21, wherein the lung adenocarcinoma is SLC34A2-ROS1, CD74-ROS1, or FIG-ROS1 fusion-positive non-small cell lung cancer.
Embodiment 25. The method of embodiment 24, wherein compound 1 or a pharmaceutically acceptable salt thereof is administered as a pharmaceutical composition comprising compound 1 or a pharmaceutically acceptable salt thereof and at least one pharmaceutically acceptable carrier.
Embodiment 26. The method of embodiment 24, wherein compound 1 or a pharmaceutically acceptable salt thereof is administered as a pharmaceutical composition comprising compound 1 or a pharmaceutically acceptable salt thereof and at least one pharmaceutically acceptable carrier; wherein the pharmaceutical composition is administered daily for more than 3 months.
Embodiment 27. The method of embodiment 24, wherein compound 1 or a pharmaceutically acceptable salt thereof is administered as a pharmaceutical composition comprising compound 1 or a pharmaceutically acceptable salt thereof and at least one pharmaceutically acceptable carrier; wherein the pharmaceutical composition is administered at a dosage of 5, 10, 15, 20, 25, 30, 35, 40, 45, 50, 55, 65, 70, 75, 80, 85, 90, or 95 mg/day.
Embodiment 28. The method of embodiment 24, wherein the detection of the SLC34A2-ROS1, CD74-ROS1, or FIG-ROS1 fusion-positive non-small cell lung cancer is made using a FISH, CISH or SISH assay.
Embodiment 29. The method of embodiment 24, wherein the detection of the SLC34A2-ROS1, CD74-ROS1, or FIG-ROS1 fusion-positive non-small cell lung cancer is made using any form of genome PCR, direct sequencing, PCR sequencing, RT-PCR or similar assay.
Embodiment 30. The method of embodiment 24, wherein the detection of the SLC34A2-ROS1, CD74-ROS1, or FIG-ROS1 fusion-positive non-small cell lung cancer is made using an antibody which specifically binds to SLC34A2-ROS1, CD74-ROS1, or FIG-ROS1 fusion polypeptide or a fragment thereof.
Embodiment 31. A method of diagnosing and treating a patient wherein the patient has a NSCLC tumor and the tumor is identified as SLC34A2-ROS1, CD74-ROS1, or FIG-ROS1 fusion-positive NSCLC, and the treatment comprises the administration of a therapeutically effective amount of a compound of Formula I, or a pharmaceutically acceptable salt thereof, and at least one pharmaceutically acceptable carrier.
Embodiment 32. A method for treating a lung adenocarcinoma which is SLC34A2-ROS1, CD74-ROS1, or FIG-ROS1 fusion positive non-small cell lung cancer in a patient in need of such treatment, comprising administering to the patient a therapeutically effective amount of compound 1: 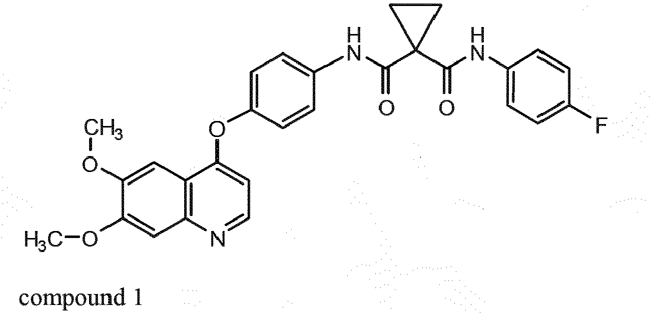 or a pharmaceutically acceptable salt thereof.
Embodiment 33. The method of any one of embodiments 1-32, wherein the effective amount of a compound of Formula I, la, or compound 1 produces at least one therapeutic effect selected from the group consisting of reduction in size of a tumor, reduction in metastasis, complete remission, partial remission, stable disease, increase in overall response rate, or a pathologic complete response.
Embodiment 34. A method of inhibiting ROS1 fusion kinase activity in a cancerous cell, the method comprising contacting said cell with an effective amount of a compound of Formula I: 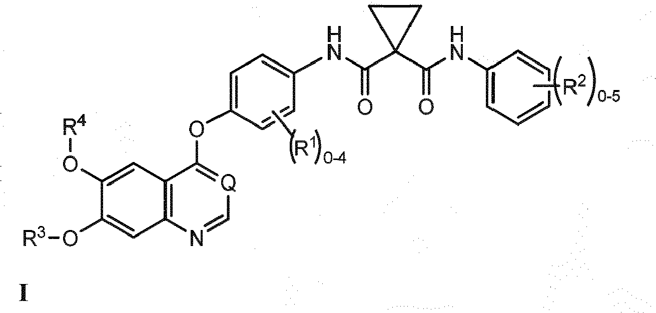 or a pharmaceutically acceptable salt thereof, wherein:
   R¹ is halo;
   R² is halo;
   R³ is (C₁-C₆)alkyl;
   R⁴ is (C₁-C₆)alkyl; and
   Q is CH or N.
Embodiment 35. The method of embodiment 34, wherein the cancerous cell is a non-small cell lung cancer adenocarcinoma cell.
Embodiment 36. The method of embodiment 34, wherein the cancerous cell is a SLC34A2-ROS1, CD74-ROS1, or FIG-ROS1 fusion-positive non-small cell lung cancer adenocarcinoma cell.
Embodiment 37. The method of any one of embodiments 34-36, wherein the compound of Formula I is a compound of Formula Ia 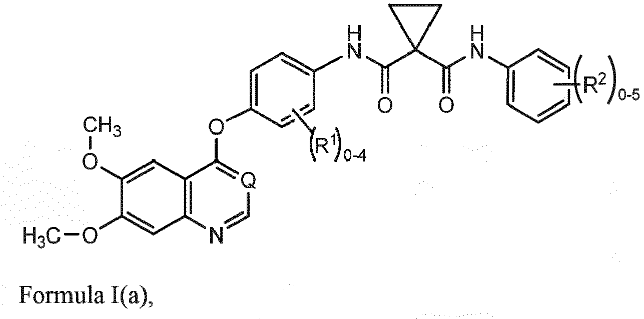 or a pharmaceutically acceptable salt thereof, wherein:
   R¹ is halo;
   R² is halo; and
   Q is CH or N.
Embodiment 38. The method of any one of embodiments 34-37, wherein the compound of Formula I is compound 1: 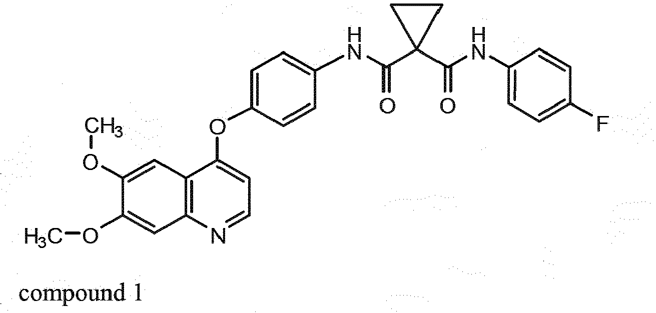 or a pharmaceutically acceptable salt thereof.
Embodiment 39. The method of embodiment 38, wherein compound 1 is N-(4-{[6,7-bis(methyloxy)quinolin-4-yl]oxy}phenyl)-N'-(4-fluorophenyl)cyclopropane-1,1-dicarboxamide or a pharmaceutically acceptable salt thereof.
Embodiment 40. The method of any one of embodiments 34-39, wherein the compound of Formula (I), Formula I(a) and compound I is the (L)- or (D)-malate salt.
Embodiment 41. The method of any one of embodiments 34-40, wherein the compound of Formula (I) is in the crystalline N-1 form or N-2 form of the (L) malate salt and/or the (D) malate salt.
Embodiment 42. The method of any one of embodiments 34-41 wherein the compound of Formula I, I(a), or compound 1, or a pharmaceutically acceptable salt thereof, is administered to the cancerous cell as a pharmaceutical composition additionally comprising a pharmaceutically acceptable carrier, excipient, or diluent.

## Claims

1. A compound for use in a method of treating lung adenocarcinoma in a patient, wherein the compound is a compound of Formula I: 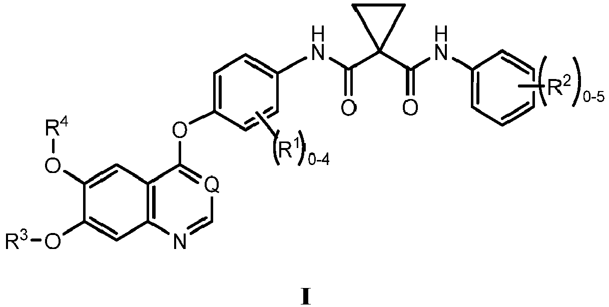 or a pharmaceutically acceptable salt thereof, wherein:
R¹ is halo;
R² is halo;
R³ is (C₁-C₆)alkyl;
R⁴ is (C₁-C₆)alkyl; and
Q is CH or N.

2. The compound for use of claim 1, wherein the lung adenocarcinoma is non-small cell lung cancer.

3. The compound for use of claim 1, wherein the lung adenocarcinoma is SLC34A2-ROS1, CD74-ROS1, or FIG-ROS1 fusion-positive non-small cell lung cancer.

4. The compound for use of any one of claims 1-3, wherein the compound of Formula I is a compound of Formula Ia 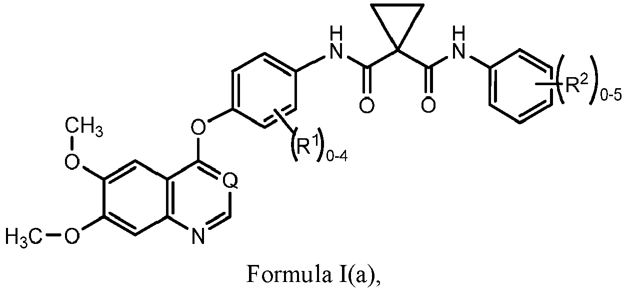 or a pharmaceutically acceptable salt thereof, wherein:
R¹ is halo;
R² is halo; and
Q is CH or N.

5. The compound for use of any one of claims 1-4, wherein the compound of Formula I is compound 1: 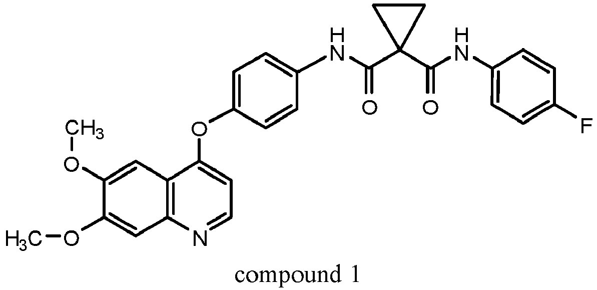 or a pharmaceutically acceptable salt thereof.

6. The compound for use of Claim 1, wherein the compound of Formula I is the (L)- or (D)-malate salt of compound 1.

7. The compound for use of any of claims 1-6 wherein the compound is administered as a pharmaceutical composition additionally comprising a pharmaceutically acceptable carrier, excipient, or diluent.

8. The compound for use of any preceding claim wherein the compound is administered post-cisplatin and/or gemcitabine treatment; or wherein the compound is administered post-carboplatin treatment; or wherein the compound is administered post-carboplatin and/or gemcitabine treatment; or wherein the compound is administered post-cisplatin and/or carboplatin treatment; or wherein the compound is administered post-docetaxel treatment; or wherein the compound is administered post-crizotinib treatment; or wherein the compound is administered post-crizotinib and/or gemcitabine treatment; or wherein the compound is administered post-crizotinib and/or gemcitabine and/or docetaxel treatment; or wherein the compound is administered post-cisplatin and/or gemcitabine and/or docetaxel treatment.

9. A compound for use in a method of treating a ROS1 fusion-positive non-small cell lung cancer in a patient, wherein the compound is compound 1: 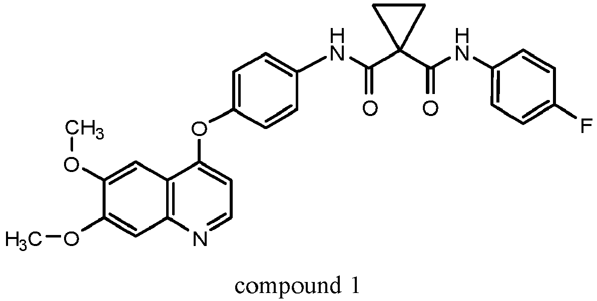 or a pharmaceutically acceptable salt thereof.

10. A compound for use in a method of inhibiting or reversing the progress of cancer mediated by ROS1 kinase in a mammal, wherein the compound is compound 1: 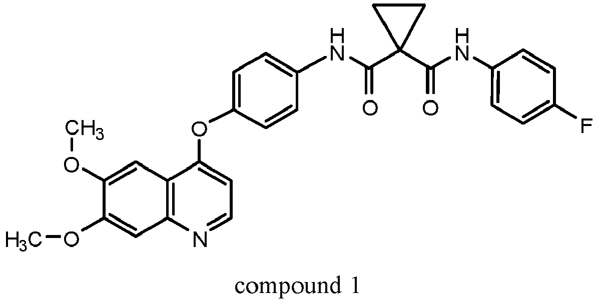 or a pharmaceutically acceptable salt thereof.

11. The compound for use of claim 10, wherein the compound is administered as a pharmaceutical composition comprising compound 1 or a pharmaceutically acceptable salt thereof and at least one pharmaceutically acceptable carrier.

12. The compound for use of claim 11, wherein the pharmaceutical composition is administered daily for more than 3 months.

13. The compound for use of claim 11 or claim 12, wherein the pharmaceutical composition is administered at a dosage of 5, 10, 15, 20, 25, 30, 35, 40, 45, 50, 55, 65, 70, 75, 80, 85, 90, or 95 mg/day.

14. A compound for use in a method of diagnosing and treating a patient wherein the patient has a NSCLC tumor and the tumor is identified as SLC34A2-ROS1, CD74-ROS1, or FIG-ROS1 fusion-positive NSCLC, and the treatment comprises the administration of a therapeutically effective amount of a compound of Formula I, or a pharmaceutically acceptable salt thereof, and wherein the compound is administered with at least one pharmaceutically acceptable carrier.

15. The compound for use of Claim 14, wherein identification of the SLC34A2-ROS1, CD74-ROS1, or FIG-ROS1 fusion-positive non-small cell lung cancer is made using a FISH, CISH or SISH assay; or wherein identification of the SLC34A2-ROS1, CD74-ROS1, or FIG-ROS1 fusion-positive non-small cell lung cancer is made using any form of genome PCR, direct sequencing, PCR sequencing, RT-PCR or similar assay; or wherein identification of the SLC34A2-ROS1, CD74-ROS1, or FIG-ROS1 fusion-positive non-small cell lung cancer is made using an antibody which specifically binds to SLC34A2-ROS1, CD74-ROS1, or FIG-ROS1 fusion polypeptide or a fragment thereof.


SEQUENCE LISTING

<110>  Exelixis Inc.

<120>  Method of Treating Lung Adenocarcinoma

<130>  P48041EP-D1-PCT

<140>  TBA
<141>  2015-04-27

<150>  US 61/984,599
<151>  2014-04-25

<160>  15

<170>  PatentIn version 3.5

<210>  1
<211>  2347
<212>  PRT
<213>  Homo sapiens

<220>
<221>  MISC_FEATURE
<222>  (1)..(2347)
<223>  Human ROS1 Protein UniProt Accession No. P08922

<400>  1

Met Lys Asn Ile Tyr Cys Leu Ile Pro Lys Leu Val Asn Phe Ala Thr
1               5                   10                  15

Leu Gly Cys Leu Trp Ile Ser Val Val Gln Cys Thr Val Leu Asn Ser
            20                  25                  30

Cys Leu Lys Ser Cys Val Thr Asn Leu Gly Gln Gln Leu Asp Leu Gly
        35                  40                  45

Thr Pro His Asn Leu Ser Glu Pro Cys Ile Gln Gly Cys His Phe Trp
    50                  55                  60

Asn Ser Val Asp Gln Lys Asn Cys Ala Leu Lys Cys Arg Glu Ser Cys
65                  70                  75                  80

Glu Val Gly Cys Ser Ser Ala Glu Gly Ala Tyr Glu Glu Glu Val Leu
                85                  90                  95

Glu Asn Ala Asp Leu Pro Thr Ala Pro Phe Ala Ser Ser Ile Gly Ser
            100                 105                 110

His Asn Met Thr Leu Arg Trp Lys Ser Ala Asn Phe Ser Gly Val Lys
        115                 120                 125

Tyr Ile Ile Gln Trp Lys Tyr Ala Gln Leu Leu Gly Ser Trp Thr Tyr
    130                 135                 140

Thr Lys Thr Val Ser Arg Pro Ser Tyr Val Val Lys Pro Leu His Pro
145                 150                 155                 160

Phe Thr Glu Tyr Ile Phe Arg Val Val Trp Ile Phe Thr Ala Gln Leu
                165                 170                 175

Gln Leu Tyr Ser Pro Pro Ser Pro Ser Tyr Arg Thr His Pro His Gly
            180                 185                 190

Val Pro Glu Thr Ala Pro Leu Ile Arg Asn Ile Glu Ser Ser Ser Pro
        195                 200                 205

Asp Thr Val Glu Val Ser Trp Asp Pro Pro Gln Phe Pro Gly Gly Pro
    210                 215                 220

Ile Leu Gly Tyr Asn Leu Arg Leu Ile Ser Lys Asn Gln Lys Leu Asp
225                 230                 235                 240

Ala Gly Thr Gln Arg Thr Ser Phe Gln Phe Tyr Ser Thr Leu Pro Asn
                245                 250                 255

Thr Ile Tyr Arg Phe Ser Ile Ala Ala Val Asn Glu Val Gly Glu Gly
            260                 265                 270

Pro Glu Ala Glu Ser Ser Ile Thr Thr Ser Ser Ser Ala Val Gln Gln
        275                 280                 285

Glu Glu Gln Trp Leu Phe Leu Ser Arg Lys Thr Ser Leu Arg Lys Arg
    290                 295                 300

Ser Leu Lys His Leu Val Asp Glu Ala His Cys Leu Arg Leu Asp Ala
305                 310                 315                 320

Ile Tyr His Asn Ile Thr Gly Ile Ser Val Asp Val His Gln Gln Ile
                325                 330                 335

Val Tyr Phe Ser Glu Gly Thr Leu Ile Trp Ala Lys Lys Ala Ala Asn
            340                 345                 350

Met Ser Asp Val Ser Asp Leu Arg Ile Phe Tyr Arg Gly Ser Gly Leu
        355                 360                 365

Ile Ser Ser Ile Ser Ile Asp Trp Leu Tyr Gln Arg Met Tyr Phe Ile
    370                 375                 380

Met Asp Glu Leu Val Cys Val Cys Asp Leu Glu Asn Cys Ser Asn Ile
385                 390                 395                 400

Glu Glu Ile Thr Pro Pro Ser Ile Ser Ala Pro Gln Lys Ile Val Ala
                405                 410                 415

Asp Ser Tyr Asn Gly Tyr Val Phe Tyr Leu Leu Arg Asp Gly Ile Tyr
            420                 425                 430

Arg Ala Asp Leu Pro Val Pro Ser Gly Arg Cys Ala Glu Ala Val Arg
        435                 440                 445

Ile Val Glu Ser Cys Thr Leu Lys Asp Phe Ala Ile Lys Pro Gln Ala
    450                 455                 460

Lys Arg Ile Ile Tyr Phe Asn Asp Thr Ala Gln Val Phe Met Ser Thr
465                 470                 475                 480

Phe Leu Asp Gly Ser Ala Ser His Leu Ile Leu Pro Arg Ile Pro Phe
                485                 490                 495

Ala Asp Val Lys Ser Phe Ala Cys Glu Asn Asn Asp Phe Leu Val Thr
            500                 505                 510

Asp Gly Lys Val Ile Phe Gln Gln Asp Ala Leu Ser Phe Asn Glu Phe
        515                 520                 525

Ile Val Gly Cys Asp Leu Ser His Ile Glu Glu Phe Gly Phe Gly Asn
    530                 535                 540

Leu Val Ile Phe Gly Ser Ser Ser Gln Leu His Pro Leu Pro Gly Arg
545                 550                 555                 560

Pro Gln Glu Leu Ser Val Leu Phe Gly Ser His Gln Ala Leu Val Gln
                565                 570                 575

Trp Lys Pro Pro Ala Leu Ala Ile Gly Ala Asn Val Ile Leu Ile Ser
            580                 585                 590

Asp Ile Ile Glu Leu Phe Glu Leu Gly Pro Ser Ala Trp Gln Asn Trp
        595                 600                 605

Thr Tyr Glu Val Lys Val Ser Thr Gln Asp Pro Pro Glu Val Thr His
    610                 615                 620

Ile Phe Leu Asn Ile Ser Gly Thr Met Leu Asn Val Pro Glu Leu Gln
625                 630                 635                 640

Ser Ala Met Lys Tyr Lys Val Ser Val Arg Ala Ser Ser Pro Lys Arg
                645                 650                 655

Pro Gly Pro Trp Ser Glu Pro Ser Val Gly Thr Thr Leu Val Pro Ala
            660                 665                 670

Ser Glu Pro Pro Phe Ile Met Ala Val Lys Glu Asp Gly Leu Trp Ser
        675                 680                 685

Lys Pro Leu Asn Ser Phe Gly Pro Gly Glu Phe Leu Ser Ser Asp Ile
    690                 695                 700

Gly Asn Val Ser Asp Met Asp Trp Tyr Asn Asn Ser Leu Tyr Tyr Ser
705                 710                 715                 720

Asp Thr Lys Gly Asp Val Phe Val Trp Leu Leu Asn Gly Thr Asp Ile
                725                 730                 735

Ser Glu Asn Tyr His Leu Pro Ser Ile Ala Gly Ala Gly Ala Leu Ala
            740                 745                 750

Phe Glu Trp Leu Gly His Phe Leu Tyr Trp Ala Gly Lys Thr Tyr Val
        755                 760                 765

Ile Gln Arg Gln Ser Val Leu Thr Gly His Thr Asp Ile Val Thr His
    770                 775                 780

Val Lys Leu Leu Val Asn Asp Met Val Val Asp Ser Val Gly Gly Tyr
785                 790                 795                 800

Leu Tyr Trp Thr Thr Leu Tyr Ser Val Glu Ser Thr Arg Leu Asn Gly
                805                 810                 815

Glu Ser Ser Leu Val Leu Gln Thr Gln Pro Trp Phe Ser Gly Lys Lys
            820                 825                 830

Val Ile Ala Leu Thr Leu Asp Leu Ser Asp Gly Leu Leu Tyr Trp Leu
        835                 840                 845

Val Gln Asp Ser Gln Cys Ile His Leu Tyr Thr Ala Val Leu Arg Gly
    850                 855                 860

Gln Ser Thr Gly Asp Thr Thr Ile Thr Glu Phe Ala Ala Trp Ser Thr
865                 870                 875                 880

Ser Glu Ile Ser Gln Asn Ala Leu Met Tyr Tyr Ser Gly Arg Leu Phe

                885                 890                 895

Trp Ile Asn Gly Phe Arg Ile Ile Thr Thr Gln Glu Ile Gly Gln Lys
            900                 905                 910

Thr Ser Val Ser Val Leu Glu Pro Ala Arg Phe Asn Gln Phe Thr Ile
        915                 920                 925

Ile Gln Thr Ser Leu Lys Pro Leu Pro Gly Asn Phe Ser Phe Thr Pro
    930                 935                 940

Lys Val Ile Pro Asp Ser Val Gln Glu Ser Ser Phe Arg Ile Glu Gly
945                 950                 955                 960

Asn Ala Ser Ser Phe Gln Ile Leu Trp Asn Gly Pro Pro Ala Val Asp
                965                 970                 975

Trp Gly Val Val Phe Tyr Ser Val Glu Phe Ser Ala His Ser Lys Phe
            980                 985                 990

Leu Ala Ser Glu Gln His Ser Leu Pro Val Phe Thr Val Glu Gly Leu
        995                 1000                1005

Glu Pro Tyr Ala Leu Phe Asn Leu Ser Val Thr Pro Tyr Thr Tyr
    1010                1015                1020

Trp Gly Lys Gly Pro Lys Thr Ser Leu Ser Leu Arg Ala Pro Glu
    1025                1030                1035

Thr Val Pro Ser Ala Pro Glu Asn Pro Arg Ile Phe Ile Leu Pro
    1040                1045                1050

Ser Gly Lys Cys Cys Asn Lys Asn Glu Val Val Val Glu Phe Arg
    1055                1060                1065

Trp Asn Lys Pro Lys His Glu Asn Gly Val Leu Thr Lys Phe Glu
    1070                1075                1080

Ile Phe Tyr Asn Ile Ser Asn Gln Ser Ile Thr Asn Lys Thr Cys
    1085                1090                1095

Glu Asp Trp Ile Ala Val Asn Val Thr Pro Ser Val Met Ser Phe
    1100                1105                1110

Gln Leu Glu Gly Met Ser Pro Arg Cys Phe Ile Ala Phe Gln Val
    1115                1120                1125

Arg Ala Phe Thr Ser Lys Gly Pro Gly Pro Tyr Ala Asp Val Val
    1130                1135                1140

Lys Ser Thr Thr Ser Glu Ile Asn Pro Phe Pro His Leu Ile Thr
    1145                1150                1155

Leu Leu Gly Asn Lys Ile Val Phe Leu Asp Met Asp Gln Asn Gln
    1160                1165                1170

Val Val Trp Thr Phe Ser Ala Glu Arg Val Ile Ser Ala Val Cys
    1175                1180                1185

Tyr Thr Ala Asp Asn Glu Met Gly Tyr Tyr Ala Glu Gly Asp Ser
    1190                1195                1200

Leu Phe Leu Leu His Leu His Asn Arg Ser Ser Ser Glu Leu Phe
    1205                1210                1215

Gln Asp Ser Leu Val Phe Asp Ile Thr Val Ile Thr Ile Asp Trp
    1220                1225                1230

Ile Ser Arg His Leu Tyr Phe Ala Leu Lys Glu Ser Gln Asn Gly
    1235                1240                1245

Met Gln Val Phe Asp Val Asp Leu Glu His Lys Val Lys Tyr Pro
    1250                1255                1260

Arg Glu Val Lys Ile His Asn Arg Asn Ser Thr Ile Ile Ser Phe
    1265                1270                1275

Ser Val Tyr Pro Leu Leu Ser Arg Leu Tyr Trp Thr Glu Val Ser
    1280                1285                1290

Asn Phe Gly Tyr Gln Met Phe Tyr Tyr Ser Ile Ile Ser His Thr
    1295                1300                1305

Leu His Arg Ile Leu Gln Pro Thr Ala Thr Asn Gln Gln Asn Lys
    1310                1315                1320

Arg Asn Gln Cys Ser Cys Asn Val Thr Glu Phe Glu Leu Ser Gly
    1325                1330                1335

Ala Met Ala Ile Asp Thr Ser Asn Leu Glu Lys Pro Leu Ile Tyr
    1340                1345                1350

Phe Ala Lys Ala Gln Glu Ile Trp Ala Met Asp Leu Glu Gly Cys
    1355                1360                1365

Gln Cys Trp Arg Val Ile Thr Val Pro Ala Met Leu Ala Gly Lys
    1370                1375                1380

Thr Leu Val Ser Leu Thr Val Asp Gly Asp Leu Ile Tyr Trp Ile
    1385                1390                1395

Ile Thr Ala Lys Asp Ser Thr Gln Ile Tyr Gln Ala Lys Lys Gly
    1400                1405                1410

Asn Gly Ala Ile Val Ser Gln Val Lys Ala Leu Arg Ser Arg His
    1415                1420                1425

Ile Leu Ala Tyr Ser Ser Val Met Gln Pro Phe Pro Asp Lys Ala
    1430                1435                1440

Phe Leu Ser Leu Ala Ser Asp Thr Val Glu Pro Thr Ile Leu Asn
    1445                1450                1455

Ala Thr Asn Thr Ser Leu Thr Ile Arg Leu Pro Leu Ala Lys Thr
    1460                1465                1470

Asn Leu Thr Trp Tyr Gly Ile Thr Ser Pro Thr Pro Thr Tyr Leu
    1475                1480                1485

Val Tyr Tyr Ala Glu Val Asn Asp Arg Lys Asn Ser Ser Asp Leu
    1490                1495                1500

Lys Tyr Arg Ile Leu Glu Phe Gln Asp Ser Ile Ala Leu Ile Glu
    1505                1510                1515

Asp Leu Gln Pro Phe Ser Thr Tyr Met Ile Gln Ile Ala Val Lys
    1520                1525                1530

Asn Tyr Tyr Ser Asp Pro Leu Glu His Leu Pro Pro Gly Lys Glu
    1535                1540                1545

Ile Trp Gly Lys Thr Lys Asn Gly Val Pro Glu Ala Val Gln Leu
    1550                1555                1560

Ile Asn Thr Thr Val Arg Ser Asp Thr Ser Leu Ile Ile Ser Trp
    1565                1570                1575

Arg Glu Ser His Lys Pro Asn Gly Pro Lys Glu Ser Val Arg Tyr
    1580                1585                1590

Gln Leu Ala Ile Ser His Leu Ala Leu Ile Pro Glu Thr Pro Leu
    1595                1600                1605

Arg Gln Ser Glu Phe Pro Asn Gly Arg Leu Thr Leu Leu Val Thr
    1610                1615                1620

Arg Leu Ser Gly Gly Asn Ile Tyr Val Leu Lys Val Leu Ala Cys
    1625                1630                1635

His Ser Glu Glu Met Trp Cys Thr Glu Ser His Pro Val Thr Val
    1640                1645                1650

Glu Met Phe Asn Thr Pro Glu Lys Pro Tyr Ser Leu Val Pro Glu
    1655                1660                1665

Asn Thr Ser Leu Gln Phe Asn Trp Lys Ala Pro Leu Asn Val Asn
    1670                1675                1680

Leu Ile Arg Phe Trp Val Glu Leu Gln Lys Trp Lys Tyr Asn Glu
    1685                1690                1695

Phe Tyr His Val Lys Thr Ser Cys Ser Gln Gly Pro Ala Tyr Val
    1700                1705                1710

Cys Asn Ile Thr Asn Leu Gln Pro Tyr Thr Ser Tyr Asn Val Arg
    1715                1720                1725

Val Val Val Val Tyr Lys Thr Gly Glu Asn Ser Thr Ser Leu Pro
    1730                1735                1740

Glu Ser Phe Lys Thr Lys Ala Gly Val Pro Asn Lys Pro Gly Ile
    1745                1750                1755

Pro Lys Leu Leu Glu Gly Ser Lys Asn Ser Ile Gln Trp Glu Lys
    1760                1765                1770

Ala Glu Asp Asn Gly Cys Arg Ile Thr Tyr Tyr Ile Leu Glu Ile
    1775                1780                1785

Arg Lys Ser Thr Ser Asn Asn Leu Gln Asn Gln Asn Leu Arg Trp
    1790                1795                1800

Lys Met Thr Phe Asn Gly Ser Cys Ser Ser Val Cys Thr Trp Lys
    1805                1810                1815

Ser Lys Asn Leu Lys Gly Ile Phe Gln Phe Arg Val Val Ala Ala
    1820                1825                1830

Asn Asn Leu Gly Phe Gly Glu Tyr Ser Gly Ile Ser Glu Asn Ile

1835                1840                1845

Ile Leu Val Gly Asp Asp Phe Trp Ile Pro Glu Thr Ser Phe Ile
    1850                1855                1860

Leu Thr Ile Ile Val Gly Ile Phe Leu Val Val Thr Ile Pro Leu
    1865                1870                1875

Thr Phe Val Trp His Arg Arg Leu Lys Asn Gln Lys Ser Ala Lys
    1880                1885                1890

Glu Gly Val Thr Val Leu Ile Asn Glu Asp Lys Glu Leu Ala Glu
    1895                1900                1905

Leu Arg Gly Leu Ala Ala Gly Val Gly Leu Ala Asn Ala Cys Tyr
    1910                1915                1920

Ala Ile His Thr Leu Pro Thr Gln Glu Glu Ile Glu Asn Leu Pro
    1925                1930                1935

Ala Phe Pro Arg Glu Lys Leu Thr Leu Arg Leu Leu Leu Gly Ser
    1940                1945                1950

Gly Ala Phe Gly Glu Val Tyr Glu Gly Thr Ala Val Asp Ile Leu
    1955                1960                1965

Gly Val Gly Ser Gly Glu Ile Lys Val Ala Val Lys Thr Leu Lys
    1970                1975                1980

Lys Gly Ser Thr Asp Gln Glu Lys Ile Glu Phe Leu Lys Glu Ala
    1985                1990                1995

His Leu Met Ser Lys Phe Asn His Pro Asn Ile Leu Lys Gln Leu
    2000                2005                2010

Gly Val Cys Leu Leu Asn Glu Pro Gln Tyr Ile Ile Leu Glu Leu
    2015                2020                2025

Met Glu Gly Gly Asp Leu Leu Thr Tyr Leu Arg Lys Ala Arg Met
    2030                2035                2040

Ala Thr Phe Tyr Gly Pro Leu Leu Thr Leu Val Asp Leu Val Asp
    2045                2050                2055

Leu Cys Val Asp Ile Ser Lys Gly Cys Val Tyr Leu Glu Arg Met
    2060                2065                2070

His Phe Ile His Arg Asp Leu Ala Ala Arg Asn Cys Leu Val Ser
    2075                2080                2085

Val Lys Asp Tyr Thr Ser Pro Arg Ile Val Lys Ile Gly Asp Phe
    2090                2095                2100

Gly Leu Ala Arg Asp Ile Tyr Lys Asn Asp Tyr Tyr Arg Lys Arg
    2105                2110                2115

Gly Glu Gly Leu Leu Pro Val Arg Trp Met Ala Pro Glu Ser Leu
    2120                2125                2130

Met Asp Gly Ile Phe Thr Thr Gln Ser Asp Val Trp Ser Phe Gly
    2135                2140                2145

Ile Leu Ile Trp Glu Ile Leu Thr Leu Gly His Gln Pro Tyr Pro
    2150                2155                2160

Ala His Ser Asn Leu Asp Val Leu Asn Tyr Val Gln Thr Gly Gly
    2165                2170                2175

Arg Leu Glu Pro Pro Arg Asn Cys Pro Asp Asp Leu Trp Asn Leu
    2180                2185                2190

Met Thr Gln Cys Trp Ala Gln Glu Pro Asp Gln Arg Pro Thr Phe
    2195                2200                2205

His Arg Ile Gln Asp Gln Leu Gln Leu Phe Arg Asn Phe Phe Leu
    2210                2215                2220

Asn Ser Ile Tyr Lys Ser Arg Asp Glu Ala Asn Asn Ser Gly Val
    2225                2230                2235

Ile Asn Glu Ser Phe Glu Gly Glu Asp Gly Asp Val Ile Cys Leu
    2240                2245                2250

Asn Ser Asp Asp Ile Met Pro Val Ala Leu Met Glu Thr Lys Asn
    2255                2260                2265

Arg Glu Gly Leu Asn Tyr Met Val Leu Ala Thr Glu Cys Gly Gln
    2270                2275                2280

Gly Glu Glu Lys Ser Glu Gly Pro Leu Gly Ser Gln Glu Ser Glu
    2285                2290                2295

Ser Cys Gly Leu Arg Lys Glu Glu Lys Glu Pro His Ala Asp Lys
    2300                2305                2310

Asp Phe Cys Gln Glu Lys Gln Val Ala Tyr Cys Pro Ser Gly Lys
    2315                2320                2325

Pro Glu Gly Leu Asn Tyr Ala Cys Leu Thr His Ser Gly Tyr Gly
    2330                2335                2340

Asp Gly Ser Asp
    2345

<210> 2
<211> 278
<212> PRT
<213> Homo sapiens

<220>
<221> MISC_FEATURE
<222> (1)..(278)
<223> Human ROS1 Kinase Domain

<400> 2

Leu Thr Leu Arg Leu Leu Leu Gly Ser Gly Ala Phe Gly Glu Val Tyr
1               5                   10                  15

Glu Gly Thr Ala Val Asp Ile Leu Gly Val Gly Ser Gly Glu Ile Lys
            20                  25                  30

Val Ala Val Lys Thr Leu Lys Lys Gly Ser Thr Asp Gln Glu Lys Ile
        35                  40                  45

Glu Phe Leu Lys Glu Ala His Leu Met Ser Lys Phe Asn His Pro Asn
    50                  55                  60

Ile Leu Lys Gln Leu Gly Val Cys Leu Leu Asn Glu Pro Gln Tyr Ile
65                  70                  75                  80

Ile Leu Glu Leu Met Glu Gly Gly Asp Leu Leu Thr Tyr Leu Arg Lys
                85                  90                  95

Ala Arg Met Ala Thr Phe Tyr Gly Pro Leu Leu Thr Leu Val Asp Leu
            100                 105                 110

Val Asp Leu Cys Val Asp Ile Ser Lys Gly Cys Val Tyr Leu Glu Arg
        115                 120                 125

Met His Phe Ile His Arg Asp Leu Ala Ala Arg Asn Cys Leu Val Ser
    130                 135                 140

Val Lys Asp Tyr Thr Ser Pro Arg Ile Val Lys Ile Gly Asp Phe Gly

145                 150                 155                 160

Leu Ala Arg Asp Ile Tyr Lys Asn Asp Tyr Tyr Arg Lys Arg Gly Glu
                165                 170                 175

Gly Leu Leu Pro Val Arg Trp Met Ala Pro Glu Ser Leu Met Asp Gly
            180                 185                 190

Ile Phe Thr Thr Gln Ser Asp Val Trp Ser Phe Gly Ile Leu Ile Trp
        195                 200                 205

Glu Ile Leu Thr Leu Gly His Gln Pro Tyr Pro Ala His Ser Asn Leu
    210                 215                 220

Asp Val Leu Asn Tyr Val Gln Thr Gly Gly Arg Leu Glu Pro Pro Arg
225                 230                 235                 240

Asn Cys Pro Asp Asp Leu Trp Asn Leu Met Thr Gln Cys Trp Ala Gln
                245                 250                 255

Glu Pro Asp Gln Arg Pro Thr Phe His Arg Ile Gln Asp Gln Leu Gln
            260                 265                 270

Leu Phe Arg Asn Phe Phe
        275

<210>  3
<211>  23
<212>  DNA
<213>  Artificial Sequence

<220>
<223>  SLC34A2 exon 4 forward

<400>  3
   tcggatttct ctactttttc gtg                                                23

<210>  4
<211>  21
<212>  DNA
<213>  Artificial Sequence

<220>
<223>  CD74 exon 6 forward

<400>  4
ctcctgtttg aaatgagcag g                                                 21

<210>  5
<211>  20
<212>  DNA
<213>  Artificial Sequence

<220>
<223>  ROS1 exon 32 reverse

<400>  5
ggaatgcctg gtttatttgg                                                   20


<210>  6
<211>  23
<212>  DNA
<213>  Artificial Sequence

<220>
<223>  ROS1 exon 34 reverse

<400>  6
tgaaacttgt ttctggtatc caa                                               23


<210>  7
<211>  21
<212>  DNA
<213>  Artificial Sequence

<220>
<223>  ROS1 InvPCR F1

<400>  7
gtctggcata gaagattaaa g                                                 21


<210>  8
<211>  20
<212>  DNA
<213>  Artificial Sequence

<220>
<223>  ROS1 InvPCR F2

<400>  8
aaacgaagac aaagagttgg                                                   20


<210>  9
<211>  20
<212>  DNA
<213>  Artificial Sequence

<220>
<223>  ROS1 InvPCR R1

<400>  9
gtcagtggga ttgtaacaac                                                   20


<210>  10
<211>  19
<212>  DNA
<213>  Artificial Sequence

<220>
<223>  ROS1 InvPCR R2

<400>  10
aaacttgttt ctggtatcc                                                    19

<210>  11
<211>  18
<212>  DNA
<213>  Artificial Sequence

<220>
<223>  ROS1 Break Rev1

<400>  11
cagctcagcc aactcttt                                                     18

<210>  12
<211>  33
<212>  DNA
<213>  Artificial Sequence

<220>
<223>  ROS1 E43R1

<400>  12
tctatttccc aaacaacgct attaatcaga ccc                                    33

<210>  13
<211>  23
<212>  DNA
<213>  Artificial Sequence

<220>
<223>  ROS1 E34R

<400>  13
tgaaacttgt ttctggtatc caa                                               23

<210>  14
<211>  23
<212>  DNA
<213>  Artificial Sequence

<220>
<223>  CD74 E5F

<400>  14
cctgagacac cttaagaaca cca                                               23

<210>  15
<211>  23
<212>  DNA
<213>  Artificial Sequence

<220>
<223>  SLC34A2 E4F

<400>  15
tcggatttct ctactttttc gtg                                               23